# EUROPÄISCHE PATENTSCHRIFT

(11) **EP 0 572 429 B1**
(45) Veröffentlichungstag und Bekanntmachung des Hinweises auf die Patenterteilung: **18.04.2001**
(21) Anmeldenummer: 92903131.8
(22) Anmeldetag: 29.01.1992
(51) Int. Cl.: C07D 239/52, C07D 239/47, C07D 251/46, A01N 47/36

(54) **SULFONYLHARNSTOFFDERIVATE, VERFAHREN ZU IHRER HERSTELLUNG UND IHRE VERWENDUNG**
SULFONYL UREA DERIVATES, METHOD FOR PREPARING THEM AND USE THEREOF
DERIVES DE SULFONYLUREE, LEUR PROCEDE DE FABRICATION ET LEUR UTILISATION

(30) Priorität: 22.02.1991 DE 4105518
(43) Veröffentlichungstag der Anmeldung: 08.12.1993
(73) Patentinhaber: BASF Aktiengesellschaft, 67063 Ludwigshafen (DE)
(72) Erfinder: MAYER, Horst, D-6700 Ludwigshafen (DE); HAMPRECHT, Gerhard, D-6940 Weinheim (DE); WESTPHALEN, Karl-Otto, D-6720 Speyer (DE); GERBER, Matthias, D-6704 Mutterstadt (DE); WALTER, Helmut, D-6719 Obrigheim (DE)
(86) Internationale Anmeldenummer: EP9200182
(87) Internationale Veröffentlichungsnummer: WO9214715

(56) Entgegenhaltungen:
- EP-A- 0 044 212
- EP-A- 0 085 028
- EP-A- 0 125 205
- US-A- 4 515 624

## Beschreibung

Die vorliegende Erfindung betrifft substituierte Sulfonylharnstoffderivate der allgemeinen Formel I, 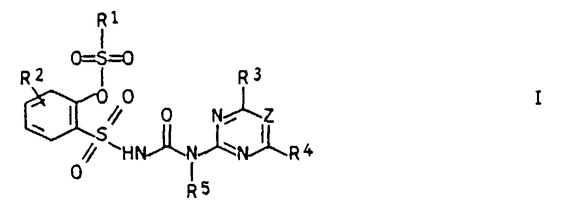 in der
- R¹: eine C₁-C₄-Alkylgruppe, die bis zu drei der folgenden Reste tragen kann: Halogen oder eine C₁-C₂-Alkoxygruppe; eine C₂-C₃-Alkenylgruppe; die Propargylgruppe; eine C₁-C₃-Alkylaminogruppe oder eine Di(C₁-C₄-alkyl)aminogruppe; einen Phenylrest, der bis zu drei der folgenden Reste tragen darf: Halogen, eine C₁-C₄-Alkylgruppe oder eine C₁-C₂-Alkoxygruppe,
- R²: Wasserstoff, Halogen, die Methyl-, Methoxy- oder Ethoxygruppe, die jeweils 1 bis 3 Halogenatome tragen können, eine C₁-C₂-Alkylsulfonylgruppe, die Nitrogruppe oder die Cyanogruppe;
- R³: die Trifluormethoxygruppe, die Bromdifluormethoxygruppe, die Chlordifluormethoxygruppe oder . Fluor;
- R⁴: Halogen, die Methyl- oder Ethylgruppe, eine C₁-C₂-Haloalkylgruppe, eine C₁-C₂-Haloalkoxygruppe, die Methoxy- oder Ethoxygruppe oder die Methyl- oder Dimethylaminogruppe;
- R⁵: Wasserstoff, eine C₁-C₃-Alkylgruppe, eine C₂-C₃-Alkenylgruppe oder eine C₃-C₄-Alkinylgruppe;
- Z: CH oder N
bedeuten, mit der Maßgabe, daß
wenn R³ ein Fluoratom bedeutet und Z für N steht, R⁴ keine Alkylaminogruppe bedeutet.
sowie deren landwirtschaftlich brauchbare Salze.

Desweiteren betrifft die Erfindung Verfahren zur Herstellung der genannten Verbindungen der allgemeinen Formel I sowie ihre Verwendung als Herbizide.

In den europäischen Patentschriften EP-B 30 433, 44 212, 125 205, 135 332, 136 061, 158 600 und den US-Schriften 4,534,789 und 4,127,405 werden gegebenenfalls substituierte Alkyl- bzw. Arylsulfonester, in der EP-A 125 205 und den US-Patentschriften 4,576,633 oder 4,515,624 werden gegebenenfalls substituierte Aminosulfonate auf Sulfonylharnstoffbasis als Herbizide beschrieben. Sie befriedigen jedoch nicht alle Anforderungen hinsichtlich Wirkung und Selektivität.

Im Hinbick auf die biologische Wirksamkeit bevorzugte Endprodukte der Formel I sind solche, in deren Formel I
- R¹: C₁-C₄-Alkylreste wie Methyl, Ethyl, n-Propyl oder i-Propyl, n-Butyl, i-Butyl, s-Butyl oder t-Butyl vorzugsweise Methyl oder Ethyl,
C₁-C₃-Halogenalkylreste wie Fluormethyl, Chlormethyl, Brommethyl, 2-Fluorethyl, 2-Chlorethyl, Difluormethyl, Dichlormethyl, Trifluormethyl, Trichlormethyl, 2,2,2-Trifluorethyl oder 2,2,2-Trichlorethyl, vorzugsweise 2,2,2-Trifluorethyl,
C₂-C₅-Alkoxyalkylreste wie Methoxymethyl, Ethoxymethyl, 2-Methoxyethyl, 2-Ethoxyethyl, 2-Methoxypropyl, 3-Methoxypropyl, 2-Ethoxypropyl oder 3-Ethoxypropyl vorzugsweise Methoxymethyl, Ethoxymethyl oder 2-Methoxyethyl,
Propargyl,
C₂-C₃-Alkenylreste wie Vinyl, 1-Propen-3-yl, 1-E-Propen-1-yl, vorzugsweise Vinyl oder 1-Propen-3-yl,
C₁-C₃-Alkylaminoreste wie Methyl-, Ethyl-, n-Propyl- oder i-Propylamino, vorzugsweise Methylamino; Di(C₁-C₄-alkyl)reste wie Dimethyl-,
Diethyl-, Di(n-propyl)-, Di(i-propyl)-, Di(tert.-butyl)-, Methylethyl-oder Methyl(i-propyl)amino, vorzugsweise Dimethylamino,
Arylreste wie Phenyl, 2-Fluorphenyl, 3-Fluorphenyl, 4-Fluorphenyl, 2-Chlorphenyl, 3-Chlorphenyl, 4-Chlorphenyl, 2-Tolyl, 3-Tolyl, 4-Tolyl, 2-(t-Butyl)phenyl, 3-(t-Butyl)phenyl, 4-(t-Butyl)phenyl, 2-Anisyl, 3-Anisyl, 4-Anisyl, 2-Ethoxyphenyl, 3-Ethoxyphenyl oder 4-Ethoxyphenyl, vorzugsweise Phenyl, 4-Tolyl oder 4-Anisyl,
- R2: Wasserstoff, Fluor, Chlor, Brom, Iod, Methyl, Methoxy,
Ethoxy, Nitro, Cyano, Trichlormethyl, Trifluormethyl,
Methylsulfonyl, Ethylsulfonyl, vorzugsweise Wasserstoff, Fluor, Chlor, Methyl oder Methoxy,
- R³: Trifluormethoxy, Chlordifluormethoxy und Fluor,
- R⁴: Fluor, Chlor, Brom, Iod, Methoxy, Ethoxy, Methyl, Ethyl, Trifluormethyl, 1,1,1-Trifluorethyl, Difluormethoxy, Trifluormethoxy, Bromdifluormethoxy, Chlordifluormethoxy, 2,2,2-Trifluorethoxy, 1,1,2,2-Tetrafluorethoxy, 2-Chlor-1,1,2-trifluorethoxy, Methylamino oder Dimethylamino, vorzugsweise Methoxy, Chlor, Fluor, Methyl, Trifluormethoxy, Chlordifluormethoxy oder Trifluormethyl,
- R⁵: Wasserstoff, Methyl, Ethyl, n-Propyl, i-Propyl, Vinyl, 1-Propen-3-yl, Propargyl, 2-Butin-1-yl, vorzugsweise Wasserstoff, Methyl und 1-Propen-3-yl
bedeuten.

Ebenso bevorzugte Endprodukte der Formel I sind solche, in deren Formel I
- R⁵: Wasserstoff oder Methyl
bedeuten.

Ebenso bevorzugte Endprodukte der Formel I sind solche, in deren Formel I
- R¹: C₁-C₄-Alkyl, das ein- bis dreifach durch Halogen oder durch eine C₁-C₂-Alkoxygruppen substituiert sein kann; Propargyl oder C₂-C₃-Alkenyl;
- R⁵: Wasserstoff oder Methyl
bedeuten.

Ebenso bevorzugte Endprodukte der Formel I sind solche, in deren Formel I
- R¹: C₁-C₃-Alkylamino oder Di(C₁-C₄) -alkylamino;
- R⁵: Wasserstoff oder Methyl
bedeuten.

Ebenso bevorzugte Endprodukte der Formel I sind solche, in deren Formel I
- R¹: Phenyl, das ein bis drei der folgenden Reste tragen darf: Halogen, eine C₁-C₄-Alkylgruppe, Methoxy oder Ethoxy;
- R⁵: Wasserstoff oder Methyl
bedeuten.

Ebenso bevorzugte Endprodukte der Formel I sind solche, in deren Formel I
- R¹: C₁-C₄-Alkyl, das ein bis drei Halogenatomen tragen kann; Propargyl, C₂-C₃-Alkenyl, Methylamino oder Dimethylamino;
- R³: Trifluormethoxy, Chlordifluormethoxy oder Fluor;
- R⁵: Wasserstoff oder Methyl
bedeuten.

Ebenso bevorzugte Endprodukte der Formel I sind solche, in deren Formel I
- R¹: C₁-C₄-Alkyl, das ein bis drei Halogenatome tragen kann; C₂-C₃-Alkenyl, Methylamino oder Dimethylamino;
- R³: Trifluormethoxy, Chlordifluormethoxy oder Fluor;
- R⁴: Methoxy;
- R⁵: Wasserstoff oder Methyl
bedeuten.

Die erfindungsgemäßen Sulfonylharnstoffe der Formel I sind auf verschiedenen Wegen zugänglich, die in der Literatur beschrieben sind. Beispielhaft seien besonders vorteilhafte Wege (A-D) im folgenden näher erläutert. 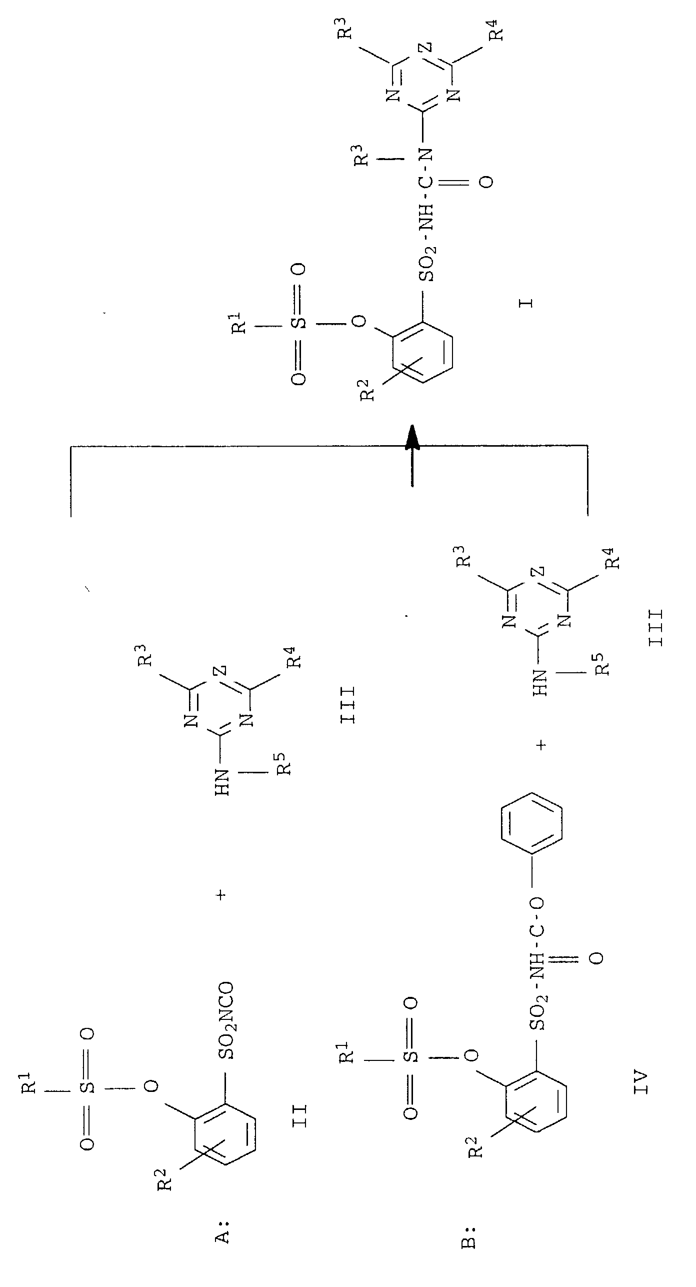 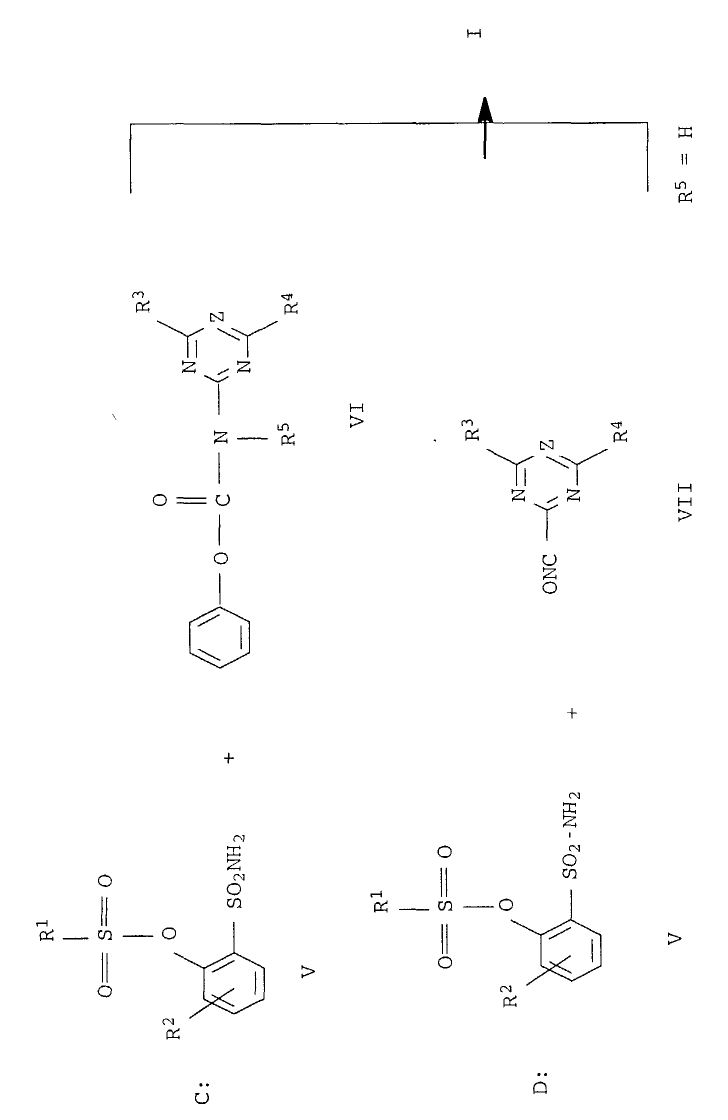
A: Man setzt ein Sulfonylisocyanat II in an sich bekannter weise (EP-A-162 723 oder EP-A-44 212) mit ungefähr der stöchiometrischen Menge eines 2-Amino-1,3,5-triazin- oder pyrimidinderivats III bei einer Temperatur von 0 bis 120°C, vorzugsweise 10 bis 100°C um. Die Reaktion kann unter Normaldruck oder unter Druck (bis 50 bar), vorzugsweise bei 1 bis 5 bar, kontinuierlich oder diskontinuierlich durchgeführt werden.
   Zweckmäßigerweise verwendet man für die Umsetzungen unter den jeweiligen Reaktionsbedingungen inerte Lösungs- und Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, ß,ß'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petro:ather, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2000 Gew.%, vorzugsweise von 200 bis 700 Gew.%, bezogen auf den Ausgangsstoff II.
   Die zur Umsetzung benötigte Verbindung II wird im allgemeinen in etwa äquimolaren Mengen (mit einem über- oder Unterschuß von z.B. 0 bis 20 %, bezogen auf den jeweiligen Ausgangsstoff III) eingesetzt. Man kann den Ausgangsstoff III in einem der vorgenannten Verdünnungsmittel vorlegen und dann den Ausgangsstoff II zugeben.
   Zweckmäßigerweise wird das Verfahren zur Herstellung der neuen Verbindungen jedoch so durchgeführt, daß man den Ausgangsstoff II, gegebenenfalls in einem der vorgenannten Verdünnungsmittel, vorlegt und dann den Ausgangsstoff III zugibt.
   Zur Beendigung der Umsetzung rührt man nach der Zugabe der Komponenten noch 20 Minuten bis 24 Stunden bei 0 bis 120°C, vorzugsweise 10 bis 100°C, insbesondere 10 bis 100°C, nach.
   Als Reaktionsbeschleuniger kann man vorteilhafterweise ein tertiäres Amin, z.B. Pyridin, α,β,γ-Picolin, 2,4-, 2,6-Lutidin, 2,4,6-Collidin, p-Dimethylaminopyridin, Trimethylamin, Triethylamin, Tri(n-propyl)amin, 1,4-Diaza[2,2,2]bicyclooctan [DABCO] oder 1,8-Diazabicylco[5,4,0]-undec-7-en in einer Menge von 0.01 bis 1 Mol pro Mol Ausgangsstoff II verwenden.
   Aus dem Reaktionsgemisch wird der Endstoff I in üblicher Weise, z.B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Rühren in einem organischen Lösungsmittel, das die Verunreinigungen aufnimmt oder Chromatographie gereinigt werden.
   Bevorzugt führt man diese Umsetzung in Acetonitril, Methyl-tert.-butylether, Toluol oder Methylenchlorid in Anwesenheit von 0 bis 100 Moläquivalenten, vorzugsweise 0 bis 50 Moläquivalenten eines tertiären Amins wie 1,4-Diazabicyclo[2,2,2]octan oder Triethylamin durch.
B: Man setzt ein entsprechendes Sulfonylcarbamat der Formel IV in an sich bekannter Weise (EP-A-120 814, EP-A-101 407) in einem inerten organischen Lösungsmittel bei einer Temperatur zwischen 0 und 120°C, vorzugsweise 10 bis 100°C mit einem 2-Amino-1,3,5-triazin- oder pyrimidinderivat III um. Es können hierbei Basen wie tertiäre Amine zugesetzt werden, wodurch die Reaktion beschleunigt und die Produktqualität verbessert wird.
   Geeignete Basen hierfür sind z.B. tertiäre Amine wie unter A angegeben, insbesondere Triethylamin oder 1,4-Diazabicyclo[2,2,2]octan, in einer Menge von 0,01 bis 1 mol pro Ausgangsstoff IV.
   Zweckmäßig verwendet man als Lösungsmittel die unter A angegebenen.
   Man verwendet das Lösungsmittel in einer Menge von 100 bis 2.000 Gew.%, vorzugsweise von 200 bis 700 Gew.%, bezogen auf den Ausgangsstoff IV.
   Die zur Umsetzung benötigte Verbindung IV wird im allgemeinen in etwa äquimolaren Mengen (mit einem über- oder Unterschuß von z.B. 0 bis 20 %, bezogen auf jeweiligen Ausgangsstoff III eingesetzt. Man kann den Ausgangsstoff IV in einem der vorgenannten Verdünnungsmittel vorlegen und dann den Ausgangsstoff III zugeben.
   Man kann jedoch auch den Ausgangsstoff III in einem der genannten Löse- oder Verdünnungsmittel vorlegen und das Sulfonylcarbamat IV zugeben.
   In beiden Fällen kann als Katalysator vor oder während der Reaktion eine Base zugesetzt werden.
   Aus dem Reaktionsgemisch kann das Endprodukt I in üblicher Weise, wie unter A angegeben, gewonnen werden.
C: Man setzt ein Sulfonamid der Formel V in an sich bekannter Weise (EP-A-141 777 und EP-A-101 670) in einem inerten organischen Lösungsmittel mit ungefähr der stöchiometrischen Menge eines Phenylcarbamats VI bei einer Temperatur von 0 bis 120°C, vorzugsweise 20 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei Basen wie tertiäre Amine zugesetzt werden, die die Reaktion beschleunigen und die Produktqualität verbessern. Geeignete Basen sind hierfür die unter A angegebenen, insbesondere Triethylamin, 2,4,6-Collidin, 1,4-Diazabicyclo[2,2,2]octan [DABCO] oder 1,8-Diazabicyclo[5,4,0]undec-7-en (DBU), in einer Menge von 0,01 bis 1 mol pro Ausgangsstoff V.
   Zweckmäßigerweise verwendet man als Löse- oder Verdünnungsmittel die unter A angegebenen.
   Man verwendet das Lösungsmittel in einer Menge von 100 bis 2000 Gew.%, vorzugsweise von 200 bis 700 Gew.%, bezogen auf das Edukt V.
   Die zur Umsetzung benötigte Verbindung V wird im allgemeinen in etwa äquimolaren Mengen (mit einem über- oder Unterschuß von z.B. 0 bis 20 %, bezogen auf die jeweiligen Ausgangsstoffe VI) eingesetzt. Man kann den Ausgangsstoff VI in einem der vorgenannten Verdünnungsmittel vorlegen und dann den Ausgangsstoff V zugeben.
   Man kann jedoch auch den Ausgangsstoff V in einem der genannten Lösungsmittel vorlegen und dann das Carbamat VI zugeben. In beiden Fällen kann als Katalysator vor oder während der Reaktion eine der genannten Basen zugesetzt werden.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach. Man isoliert die Sulfonylharnstoffe der Formel I aus dem Reaktionsgemisch mit den üblichen Methoden, wie unter A beschrieben.
D: Man setzt ein Sulfonamid der Formel V in an sich bekannter Weise (EP-A-234 352) in einem inerten organischen Lösungsmittel mit ungefähr der stöchiometrischen Menge eines Isocyanates VII zu einer Temperatur von 0 bis 150°C, vorzugsweise 10 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei vor oder während der Reaktion Basen wie tertiäre Amine zugesetzt werden, die die Reaktionsbeschleuniger und die Produktqualität verbessern. Geeignete Basen sind hierfür die unter A angegebenen, insbesondere Triethylamin oder 2,4,6-Collidin, in einer Menge von 0,01 bis 1 mol pro Ausgangsstoff V.
   Zweckmäßigerweise verwendet man als Lösungsmittel die unter A angegebenen. Man setzt das Lösungsmittel in einer Menge von 100 bis 2000 Gew.%, vorzugsweise von 200 bis 700 Gew.%, bezogen auf das Edukt V.
   Die zur Umsetzung benötigte Verbindung V wird im allgemeinen in etwa äquimolaren Mengen (mit einem über- oder Unterschuß von z.B. 0 bis 20 %, bezogen auf die Edukte VII) eingesetzt. Man kann den Ausgangsstoff VII in einem der genannten Verdünnungsmittel vorlegen und dann den Ausgangsstoff V zugeben. Man kann aber auch das Sulfonamid vorlegen und dann das Isocyanat VII zugeben.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach. Das Endprodukt I kann aus dem Reaktionsgemisch in der üblichen Weise, wie unter A: beschrieben, gewonnen werden.
   Die als Ausgangsstoffe benötigten Sulfonylisocyanate der Formel II lassen sich in an sich bekannter Weise aus den entsprechenden Sulfonamiden durch Phosgenierung (EP-A 44 212, Houben-Weyl 11/2 (1985) 1106, US 4 379 769) oder durch Umsetzung der Sulfonamide mit Chlorsulfonylisocyanat (DE-OS 3 132 944) gewinnen.
   Die Synthese der heterocyclischen Amine der allgemeinen Formel III und die Umsetzungen der dabei auftretenden Zwischenstufen läßt sich allgemein nach Methoden durchführen, wie sie in Standardwerken der Heterocyclenliteratur beschrieben sind (Pyrimidine: D.J. Brown in "The Chemistry of Heterocyclic Compounds", A. Weissberger, E.C. Taylor (Hrsg.), Wiley, New York, 1985 , Bd. 16; D.J. Brown in "Comprehensive Heterocyclic Chemistry", A. R. Katritzky (Hrsg.), Pergamon Press, New York, 1984, Bd. 3, 57 ff.; Triazine: E.M. Smolin und L. Rapoport in "The Chemistry of Heterocyclic Compounds", A. Weissberger (Hrsg.), Interscience Publ., New York, 1959 , Bd.13; J.E. Quirke in "Comprehensive Heterocyclic Chemistry", A. R. Katritzky (Hrsg.), Pergamon Press, New York, 1984, Bd. 3, 457 ff.).
   2-Aminopyrimidine und 2-Amino-1,3,5-triazine, die in 4- bzw. 6-Stellung einen Trifluormethoxy- oder Chlordifluormethoxy-Rest tragen, lassen sich insbesondere nach den Methoden der deutschen Anmeldungsschriften P 40 07 316.5, P 40 07 317.3, P 40 07 683.0, P 40 24 761.9, P 40 24 755.4 und P 40 24 754.6 herstellen.
   So lassen sich Derivate der Formel IIIa, in der R⁴ für Methylamino, Dimethylamino, Methoxy, Ethoxy oder einen C₂-Haloalkoxyrest steht, gemäß Formel Schema 2 herstellen.

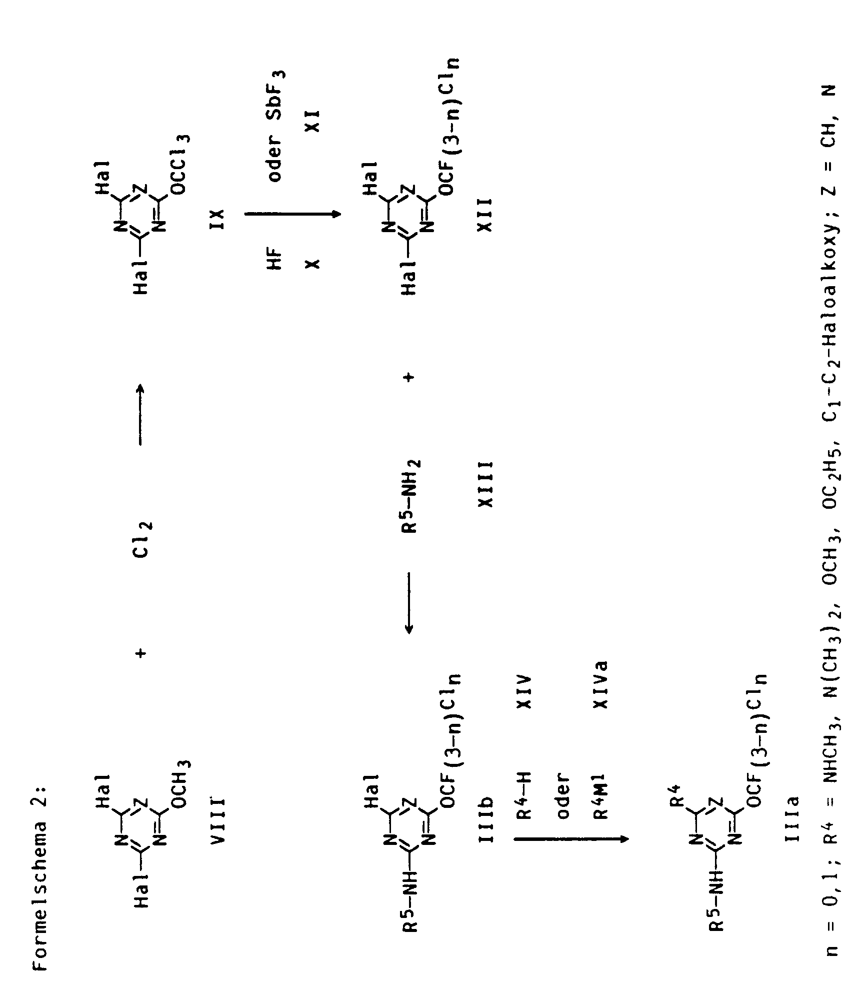

In entsprechender Weise gelangt man zu den 2-Amino-6-trifluormethyl-1,3,5-triazin oder 2-Amino-6-trifluormethylpyrimidin-derivaten IIIc, wenn man 2,4-Dihalogen-6-trichlormethyl-1,3,5-triazin oder 2,4-Dihalogen-6-trifluormethylpyrimidin der Formel XV gemäß Formelschema 3 umsetzt. 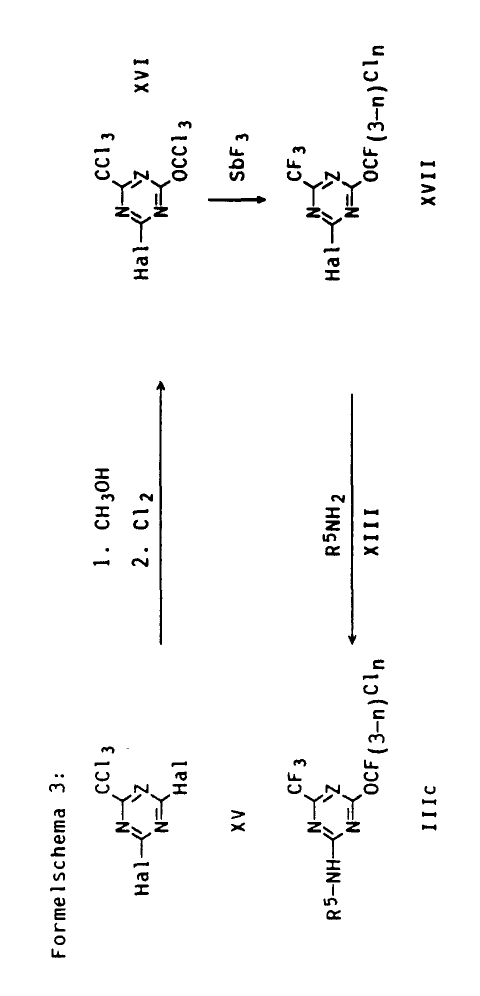 Ausgehend von den Zwischenprodukten XII in Formelschema 2 und Substitution des Halogenatoms in 4-Position durch die in Formelschema 3 beschriebene Reaktionssequenz (1. CH₃OH, 2. Cl₂, 3. SbF₃) sowie anschließende Umsetzung mit R⁵NH₂ gelangt man zu den Zwischenprodukten IIId 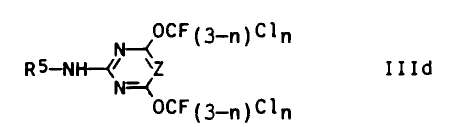 Entsprechend gelangt man zu 4-Alkyl-2-amino-1,3,5-triazinen oder 4-Alkyl-2-aminopyrimidinen IIIe, wenn man 2-Alkoxy-4-alkyl-6-halogen-triazine oder 4-Alkoxy-6-alkyl-2-halogenpyrimidine gemäß Formelschema 4 umsetzt.

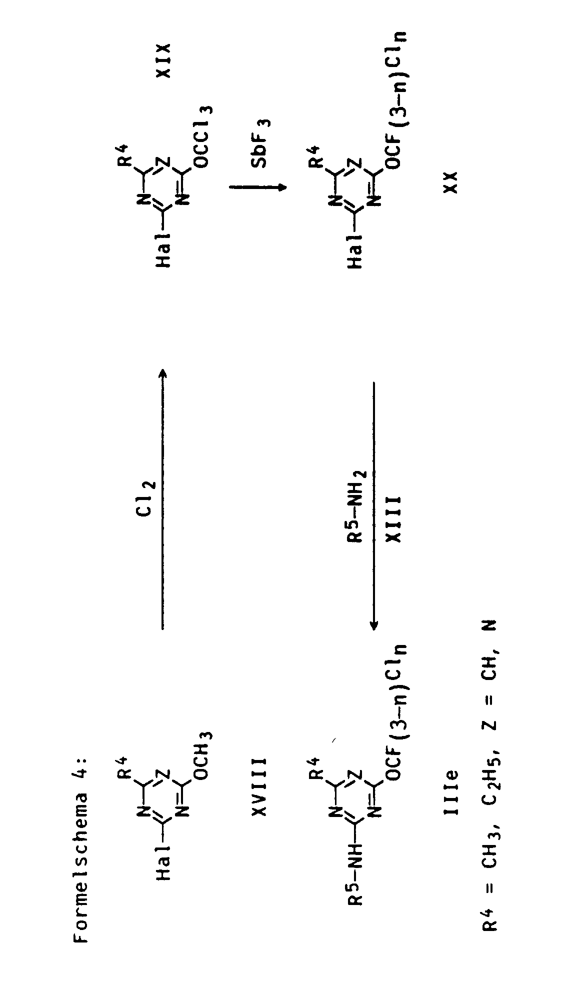

Die als Ausgangsstoffe benötigten 2-Alkoxy-4-alkyl-6-halogen-1,3,5-triazine und 4-Alkoxy-6-alkyl-2-halogenpyrimidine sind literaturbekannt (z.B. 2-Chlor-4-methoxy-6-methylpyrimidin in Bull. Soc. Chim. Belg. 68 (1959) 30; 2-Chlor-4-methoxy-6-methyl-1,3,5-triazin in Monatsh. Chem. 101 (1970) 724 oder lassen sich analog herstellen.

Die Chlorierung der 2-Methoxy-1,3,5-triazine bzw. 2-Methoxypyrimidene VIII, XV oder XVIII mit Chlor zu den Trichlormethoxy-Derivaten IX, XVI oder XIX führt man beispielsweise bei einer Temperatur von 100 bis 180°C durch.

Als Chlorierungsmittel sind elementares Chlor oder Chlor abgebende Substanzen wie Sulfurylchlorid oder Phosphorpentachlorid geeignet.

Die Umsetzung kann in Gegenwart eines inerten Lösungsmittels, beispielsweise einem chlorierten Kohlenwasserstoff wie Chlorbenzol, 1,2-, 1,3- oder 1,4-Dichlorbenzol, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, α-Chlorpropionsäurechlorid, α,α-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes VIII, XV oder XVIII, durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von α,α'-Azoisobutyronitril in zweckmäßig einer Menge von 0,2 bis 7 Mol%, bezogen auf den Ausgangsstoff VIII, XV oder XVIII. Man kann die Reaktion auch durch Zugabe eines Katalysators beschleunigen; als solcher-eignet sich Phosphorpentachlorid, zweckmäßig in einer Menge von 0,5 bis 7 Mol% bezogen auf den Ausgangsstoff VIII, XV oder XVIII. In diesem Fall legt man den Ausgangsstoff VIII, XV oder XVIII zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Die Ausgangsstoffe VIII, XV oder XVIII können mit Chlor in annähernd stöchiometrischer Menge oder vorzugsweise im überschuß, vorteilhaft mit 3,1 bis 11, insbesondere 3,3 bis 5 mol Cl₂ je Equivalent Methoxy in den Ausgangsstoffen VIII, XV oder XVIII umgesetzt werden. Die Umsetzung kann bei einer Temperatur von 100 bis 180°C, vorteilhaft von 120 bis 150°C, drucklos oder unter Druck kontinuierlich oder diskontinuierlich durchgeführt werden.

Chloriert man bei 1 bar, so werden zweckmäßig 3,3 bis 5 mol Chlorgas, bezogen auf ein Equivalent Methoxy in den Ausgangsstoffen VIII, XV oder XVIII, eingesetzt, was einem Chlorumsatz von 91 bis 60 % entspricht. Durch geeignete apparative Maßnahmen, z. B. durch Anwendung von mäßigem überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden die erforderliche Menge Chlorgas unter intensivem Rühren in die flüssigen Ausgangsstoffe VIII, XV oder XVIII einleitet, wobei man zunächst bei einer Temperatur von 120 bis 130°C beginnt und diese - gegebenenfalls unter Ausnutzung des exothermen Charakters der Reaktion - kontinuierlich steigert, so daß gegen Ende die Umsetzung bei einer Temperatur von 135 bis 150°C durchgeführt wird. Bei größeren Reaktionsansätzen muß dem exothermen Charakter durch äußere Kühlung bzw. geeignete Dosierung der Chlormenge Rechnung getragen werden; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Die Umsetzung der Trichlormethoxy-Derivate IX, XVI oder XIX mit einem Halogenaustauschmittel führt man beispielsweise bei einer Temperatur von 0 bis 180°C durch.

Als Halogenaustauschmittel sind Antimontrifluorid in Gegenwart oder Abwesenheit katalytischer Mengen eines Antimon(V)salzes oder Fluorwasserstoff geeignet.

Zweckmäßig verwendet man einen überschuß von 1 bis 200, vorzugsweise 5 bis 25 mol-% Antimontrifluorid pro Trichlormethylequivalent. Die Katalysatormenge an Antimon(V)salz beträgt zwischen 1 bis 20, vorzugsweise 5 bis 18 mol-% pro Trichlormethylequivalent. Vorzugsweise dosiert man die Ausgangsstoffe IX, XVI oder XIX bei 90 bis 130 °C zu der Mischung des Halogenaustauschmittels und erwärmt dann noch zwischen 10 und ca. 240 Minuten auf eine Temperatur zwischen 110 bis 180 °C. Anschließend wird durch Destillation aufgearbeitet.

Man kann die Reaktion jedoch auch kontinuierlich führen, die Ausgangsstoffe IX, XVI oder XIX bei 110 bis 180°C innerhalb 10 bis ca. 240 Minuten zugeben und gleichzeitig unter vermindertem Druck die entstehenden niedriger siedenden Endstoffe XII, XVII oder XX abdestillieren. Spuren mitgerissener Antimonsalze lassen sich durch Extraktion mit konz. Salzsäure beseitigen.

Arbeitet man ohne Antimon(V)salz-Katalyse oder setzt nur geringe Mengen, z. B. 0,5 bis 5 mol-% ein, und reduziert die Menge an Antimontrifluorid auf 60 bis 90 mol-% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 150°C, vorzugsweise 40 bis 120°C, durchgeführt werden. Hierzu versetzt man in einem Autoklaven die Ausgangsstoffe IX, XVI oder XIX mit einem überschuß von 300 bis 700, vorzugsweise 350 bis 400 mol-% Fluorwasserstoff pro Trichlormethylequivalent und rührt 10 Minuten bis 10 Stunden. Gegebenenfalls kann die Reaktion in gleicher Weise, wie für die Verwendung von Antimontrifluorid beschrieben, durch Zusatz eines Katalysators wie Antimonpentachlorid beschleunigt werden. Nach dem Entspannen und der Entfernung flüchtiger Bestandteile wird wie beschrieben aufgearbeitet.

Die Umsetzung der Fluormethoxy-Derivate XII, XVII oder XX mit den Aminen XIII führt man beispielsweise bei einer Temperatur von -80 bis 40°C durch.

Die 2-Halogen-1,3,5-triazine oder -pyrimidine XII, XVII oder XX können in einem aprotisch polaren Lösungsmittel mit den Aminen XIII bei einer Temperatur von -80 bis 40°C umgesetzt werden, wobei man das Amin XIII entweder in einem überschuß einsetzt oder eine organische Hilfsbase verwendet.

Für die Umsetzung der Triazine oder -Pyrimidine XII, XVII oder XX mit dem Amin XIII sind folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf die Ausgangsstoffe XII, XVII oder XX.

Vorteilhaft gibt man 1,8 bis 2,5, insbesondere 1,95 bis 2,2 mol-Equivalent des Amins XIII, bezogen auf die Ausgangsstoffe XII, XVII oder XX innerhalb 0,5 bis 2 Stunden zu einer Mischung von Ausgangsstoffen XII, XVII oder XX in einem der vorgenannten Lösungsmittel bei -80 bis 40°C, vorzugsweise -70 bis 25°C, rührt bis zur Vervollständigung der Reaktion bis zu 3 Stunden nach und läßt dann zur Aufarbeitung auf 25°C erwärmen.

Setzt man nur ungefähr stöchiometrische Mengen des Amins XIII ein, so verwendet man zweckmäßig 0,9 bis 1,1, Equivalente einer organischen Hilfsbase, bezogen auf die Ausgangsstoffe XII, XVII oder XX. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, α-,β-,γ-Picolin, 2,4- und 2,6-Lutidin und Triethylendiamin.

Die Reaktion kann drucklos oder unter Druck, kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z. B. durch Chromatographie. Man kann jedoch auch direkt die organische Phase einengen und den Rückstand mit einem Lösungsmittel verrühren.

Die 2-Amino-4-fluoralkoxy-1,3,5-triazine bzw. 2-Amino-4-fluoralkoxypyrimidine der Formel IIIa erhält man vorteilhaft in der Weise, daß man 2-Amino-4-fluoralkoxy-6-halogen-1,3,5-triazine oder -pyrimidine der Formel IIIb 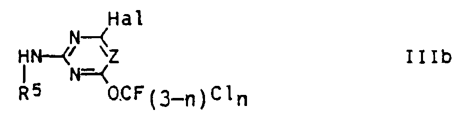 in der Hal für Fluor, Chlor oder Brom steht und R⁵, n und Z die vorgenannte Bedeutung haben, mit einem Nucleophil der Formel XIV

R⁴-H XIV

in der R⁴ für Methylamino, Dimethylamino, Methoxy, Ethoxy oder eine C₁-C₂-Haloalkoxygruppe steht, oder dessen Salz XIVa umsetzt.

Die Umsetzung der 2-Amino-4-fluoralkoxy-1,3,5-triazine oder 2-Amino-4-fluoralkoxypyrimidine IIIb mit einem Nucleophil XIV oder dessen Salz XIVa führt man beispielsweise bei einer Temperatur von -80 bis 80°C durch.

Die 4-Halogen-Derivate IIIb können in einem aprotisch polaren Lösungsmittel mit den Nukleophilen XIV bzw. XIVa bei einer Temperatur von -80 bis +80°C, vorteilhaft -30 bis +20°C umgesetzt werden, wobei man die Nukleophile entweder in einem Überschuß einsetzt oder eine organische Hilfsbase verwendet.

Für die Umsetzung des 4-Halogen-Derivate IIIb mit den Nukleophilen XIV bzw. XIVa sind folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff IIIb.

Vorteilhaft gibt man 1,8 bis 2,5, insbesondere 1,95 bis 2,2 mol-Äquivalente des Nukleophiles XIV bzw. XIVa, bezogen auf den Ausgangsstoff IIIb innerhalb 0,5 bis 2 Stunden zu einer Mischung von Ausgangsstoff IIIb in einem der vorgenannten Lösungsmittel bei (-80) bis 80°C, vorzugsweise -30 bis 25°C, rührt bis zur Vervollständigung der Reaktion (bis zu 3 Stunden) nach und läßt dann zur Aufarbeitung auf 25°C erwärmen.

Setzt man nur ungefähr stöchiometrische Mengen des Nukleophiles XIV bzw. XIVa ein, so muß man zweckmäßig 0,9 bis 1,1 Equivalente einer organischen Hilfsbase, bezogen auf den Ausgangsstoff IIIb, zusetzen. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, α-,β-,γ-Picolin, 2,4- und 2,6-Lutidin und Triethylendiamin.

Die Reaktion kann drucklos oder unter Druck, kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z. B. durch Chromatographie. Die Umsetzungsprodukte sind jedoch meist genügend rein, so daß man nur von dem ausgefallenen Salz abzufiltrieren und die organische Phase einzuengen braucht. 2-Amino- bzw. 2-Alkylamino-, 2-Alkenylamino- oder 2-Alkinylaminosubstituierte Pyrimidine, die in 4- bzw. 6-Stellung ein Fluoratom tragen, lassen sich nach dem oder in Analogie zu den in der
EP-A 378 092 oder in Yakugaku Zasshi 87 (1967) 1315 beschriebenen Verfahren herstellen. Die entsprechenden 1,3,5-Triazine sind in analoger Weise erhältlich. Geeignete Vorstufen wie etwa 2,4-Difluor-6-methoxy-1,3,5-triazin sind literaturbekannt (FR-A 1 561 876 (CA 72, 90530), DE-OS 29 01 498 (CA 91, 194627) oder Chem. Ber. 102 (1969) 2330).

2-Amino- bzw. 2-Alkylamino-, 2-Alkenylamino- oder 2-Alkinylaminosubstituierte Pyrimidine oder 1,3,5-Triazine der Formel III, die in 4- bzw. 6-Stellung eine Bromdifluormethoxygruppe tragen, sind in der EP-A 169 815 beschrieben.

2-Amino- bzw. 2-Alkylamino-, 2-Alkenylamino- oder 2-Alkinylaminosubstituierte Pyrimidine, die in der 4- bzw. 6-Stellung eine Difluormethoxygruppe tragen, sind nach den in der EP-A 84 020 beschriebenen Methoden zugänglich.

Die Sulfonylcarbamate der Formel IV wurden nach oder in Analogie zu an sich bekannten Reaktionen (z.B. EP-A 120 814) hergestellt. Man kann aber auch die Sulfonylisocyanate der FormelII in qlatter Reaktion in einem inerten Lösungsmittel wie Ether oder Dichlormethan mit Phenol in die Carbamate der Formel IV überführen.

Carbamate der Formel VI sind nach oder in Analogie zu bekannten Umsetzungen (z.B. EP-A 101 670) zugänglich, sie lassen sich aber auch aus den entsprechenden Isocyanaten VII durch Umsetzung mit Phenol herstellen.

Die Isocyanate der Formel VII erhält man aus den Aminen der Formel III durch Behandlung mit Oxalylchlorid oder Phosgen (in Analogie nach Angew. Chem. 83 (1971) 407, EP-A 388 873).

Die Sulfonamide der Formel V lassen sich durch Reaktion der entsprechenden Sulfonsäurechloride mit Ammoniak gewinnen (Houben-Weyl, Methoden der organischen Chemie, Band 9 (1955) 605). Die Sulfonsäurechloride erhält man entweder durch Meerwein-Reaktion (Diazotierung geeigneter Amine und Kupfersalz-katalysierte Sulfochlorierung) oder durch Chlorsulfonierung geeigneter Aromaten (F. Muth in "Methoden der organischen Chemie", Houben-Weyl, Thieme-Verlag, Stuttgart (1955) 557 ff.). Die Sulfonamide der Formel V lassen sich auch aus geeignet substituierten 2-Hydroxybenzolsulfonamiden durch Umsetzung mit geeigneter substituierten Sulfonylhalogeniden in Gegenwart einer Hilfsbase herstellen.

Typische Beispiele für die Herstellung der Zwischenstufen sind im experimentellen Teil aufgeführt.

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80°C bis 120°C, vorzugsweise 0°C bis 60°C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar überdruck durchgeführt werden.

Die Verbindungen I bzw. die sie enthaltenden herbiziden Mittel sowie deren umweltverträgliche Salze von Alkalimetallen und Erdalkalimetallen können in Kulturen wie Weizen, Reis und Mais Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische - Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele,
Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:
I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 5 werden in einer Mischung gelöst, die aus 80 Gewichtsteilen Xylol, 10 Gewichtsteilen des Anlagerungsproduktes von 8 bis 10 Mol Ethylenoxid an 1 Mol ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 5 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr.13 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr.13 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 5 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 1,0 kg/ha, vorzugsweise 0,01 bis 0,5 kg/ha aktive Substanz (a.i.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| | |
|---|---|
| Botanischer Name | Deutscher Name |
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Citrus limon | Zitrone |
| Citrus sinensis | Apfelsine, Orange |
| Coffea arabica (Coffea canephora, | Kaffee |
| Coffea liberica) | |
| Cucumis sativus | Gurke |
| Cynodon dactylon | Bermudagras |
| Daucus carota | Möhre |
| Elaeis guineensis | ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süβkirsche |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Sulfonylharnstoffderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazin, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxyphenylpropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische öle und Ölkonzentrate zuge-etzt werden. Nachstehend sind Beispiele für die Synthese der Verbindungen I wiedergegeben.

### Herstellung der Vorstufen

### Methansulfonsulfonsäure[(2-amino-4-chlor)phenyl]ester

Man tropft 174,5 g Methansulfonylchlorid (1.52 mol) bei 10-15°C zu einer Lösung von 218,7 g 2-Amino-4-chlorphenol (1.52 mol) und 153.8 g Triethylamin (1.52 mol) in 400 ml Methylenchlorid. Man rührt 18 h bei 25°C nach, trägt den Ansatz in Eiswasser ein und trennt die organische Phase ab. Diese wird 2x mit Wasser gewaschen und über Na₂SO₄ getrocknet. Nach Abdestillieren des Lösungsmittels verbleibt ein allmählich kristallisierender Rückstand des Produkts, der für weitere Umsetzung rein genug ist (329,5 g, 98 % d.Th.). Das Produkt kann aus Methanol/Wasser umkristallisiert werden (Fp. 70-71°C).
¹H-NMR-Spektrum (250 MHz, CDCl₃, int. TMS): 7.10 d (1H), 6.75 d (1H), 6.67 dd (1H), 4.10 br (2H), 3.16 s (3H).

### Methansulfonsäure[(2-chlorsulfonyl)phenyl]ester

Eine Diazoniumsalzlösung, hergestellt durch gleichzeitiges Eintragen einer Lösung von 39,5 g Natriumnitrit (0.57 mol) in 60 ml Wasser und 105 g Methansulfonsäure(2-aminophenyl)ester (0.57 mol) in 200 ml konz. Salzsäure bei 0-5°C und 1h Nachrühren bei 0°C, wird in eine mit Schwefeldioxid gesättigte Lösung von 1,7 g CuCl₂ und 4,5 g Benzyltriethylammoniumchlorid in 200 ml 1,2-Dichlorethan und 10 ml Wasser bei 0-10°C getropft. Man entfernt die Kühlung und rührt 30 min bei 25°C, dann unter langsamer Steigerung der Temperatur des Reaktionsgemisches noch 1h bei 50°C. Die organische Phase wird abgetrennt, mit Eiswasser gewaschen und über Na₂SO₄ getrocknet. Nach Entfernen des Lösungsmittels verbleibt ein öliger Rückstand, der durch Anreiben mit etwas Methanol kristallisiert werden kann. Man erhält so 139 g der Titelverbindung (90 % d. Th.) mit Fp. 94-95°C. 1H-NMR (300 MHz, CDCl3, int. TMS): 8.10 d (1H), 7.68-7.85 m (2H), 7.51 (1H), 3.42 s (3H).

### Methansulfonsäure[(2-aminosulfonyl)phenyl]ester

Man leitet Ammoniak bei -30°C in eine Lösung von 130,3 g Methansulfonsäure[(2-chlorsulfonyl)phenyl]ester in 11 Tetrahydrofuran und kontrolliert den Umsatz dünnschichtchromatographisch. Nach erfolgter Umsetzung wird das Tetrahydrofuran im Wasserstrahlvakuum abdestilliert und der Rückstand mit Wasser und Diethylether verrieben. Man erhält so 110,2 g der Titelverbindung (91 %) mit Fp.: 131-132°C.
1H-NMR (300 MHz, CD₃SOCD₃, int. TMS): 7.90 d (1H), 7.61 br (2H), 7.45-7.74 m (3H), 3.50 s (H).

### 2-(Dimethylaminosulfonyloxy)benzolsulfonamid

Eine Lösung von 5,0 g 2-Hydroxybenzolsulfonamid (29 mmol) in 200 ml Acetonitril wird bei 25°C mit 4,0 g Kaliumcarbonat (29 mmol) versetzt. Nach 10 min Rühren tropft man 4,1 g Dimethylaminosulfonylchlorid (29 mmol) bei 25°C zu und rührt weitere 16 h bei dieser Temperatur. Man entfernt die flüchtigen Anteile im Wasserstrahlvakuum, nimmt den Rückstand in Essigsäureethylester auf, trocknet die Lösung über Na₂SO₄ und entfernt das Lösungsmittel im Wasserstrahlvakuum. Der verbleibende Rückstand wird 1 h mit 100 ml Diethylether kräftig gerührt. Man saugt das kristalline Produkt aus und trocknet bei 40°C im Vakuum. Man erhält so 5,7 g der Titelverbindung.
¹H-NMR-Spektrum (250 MHz, CD₃SOCD₃, int. TMS, δ): 7.91 d (1H); 7.57-7.74 m (2H); 7.49 br (2H); 7.36-7.52 m (1H); 3.02 s (6H).

### Herstellung der Zwischenprodukte III

### 2,4-Difluor-6-trichlormethoxy-1,3,5-triazin

In eine Mischung von 300 g (2,041 mol) 2,4-Difluor-6-methoxy-1,3,5-triazin und 0,3 g α,α'-Azoisobutyronitril leitete man bei 130°C und unter UV-Bestrahlung einen Strom von Chlorgas so ein, daß sich während 2 Stunden eine Temperatur von 140 bis 145°C einstellte. Nach NMR-spektroskopischer Kontrolle des Reaktionsverlaufs wurde unter äußerer Beheizung noch weitere 3 Stunden bei 135 bis 140°C mit Chlor begast. Nach dem Absaugen von ausgefallenem Niederschlag und Destillation des Filtrats im Vakuum erhielt man 444 g (87 % d. Th.) der Titelverbindung vom Sdp. 40 - 46°C/0,3 mbar.

### 2,4-Difluor-6-trifluormethoxy-1,3,5-triazin

Zu einer Mischung von 187,4 g (1,048 mol) Antimontrifluorid und 35,2 g (0,117 mol) Antimonpentachlorid wurden die Hälfte von 210 g (0,838 mol) 2,4-Difluor-6-trichlormethoxy-1,3,5-triazin zunächst bei 110°C unter Rühren so zugegeben, daß sich anfangs eine Temperatur von 125°C einstellte; mit dem sich einstellenden Rückfluß mußte bei weiterer Zugabe außen beheizt werden. Es wurde eine Stunde bei 125 -130°C gerührt und eine bei 100 bis 105°C siedende Fraktion über eine 25-cm-Füllkörperkolonne abdestilliert. Nach dem Abklingen der Reaktion wurde die restliche Hälfte der Trichlormethoxyverbindung innerhalb 30 Minuten zugetropft und die bei 100 bis 105°C übergehende Fraktion kontinuerliche abdestilliert. Die Gesamtreaktionszeit betrug 3 Stunden. Man erhielt 134,4 g (79,8 % d. Th.) der Titelverbindung mit n_{D}²⁴ = 1.3650.

### 6-Chlordifluormethoxy-2,4-difluor-1,3,5-triazin

210 g (0,838 mol) 2,4-Difluor-6-trichlormethoxy-1,3,5-triazin wurden innerhalb 10 Minuten unter Rühren bei 110°C zu 110 g (0,614 mol) Antimontrifluorid gegeben. Nach Zugabe von 3/4 von 9,38 g (0,0313 mol) Antimonpentachlorid wurde auf 145 °C erwärmt und 1 Stunde gerührt. Restlicher Katalysator wurde zugegeben und nochmals 2 Stunden gerührt, wobei als niedersiedende Fraktion über eine 30-cm-Füllkörperkolonne zwischen 95 bis 105°C 20 g (11,8 % d.Th.) 2,4-Difluor-6-trifluormethoxy-1,3,5-triazin erhalten wurde. Der Destillationsrückstand wurde ohne Kolonne destilliert und ergab 94,8 g (52 % d.Th.) der Titelverbindung vom Sdp. 125 - 130°C; n_{D}²⁴ = 1.4042.

### 2,4-Dichlor-6-trifluormethoxy-1,3,5-triazin

Zu einer Mischung von 40,9 g (0,229 mol) Antimontrifluorid und 7,03 g (0,0234 mol) Antimonpentachlorid wurden 52 g (0,183 mol) 2,4-Dichlor-6-trichlormethoxy-1,3,5-triazin innerhalb 5 Minuten unter Rühren bei 90°C zugegeben, wobei sich die Temperatur bis auf 180°C erhöhte. Es wurde noch 20 Minuten bei 170 bis 180°C nachgerührt und dann das Rohprodukt bei 90 - 103°C/70 mbar abdestilliert. Durch nochmalige Destillation erhielt man 32,3 g (75,5 % d.Th.) der Titelverbindung vom Sdp. 165 - 173°C.

### 2-Amino-4-fluor-6-trifluormethoxy-1,3,5-triazin

4,4 g (0,259 mol) Ammoniakgas wurden innerhalb 45 Minuten bei -70 bis -65°C unter Rühren in eine Mischung von 26,0 g (0,1293 mol) 2,4-Difluor-6-trifluormethoxy-1,3,5-triazin in 100 ml Tetrahydrofuran eingeleitet. Es wurde 2 Stunden bei -70°C und über Nacht unter Erwärmung bis auf 22°C gerührt. Nach dem Einengen im Vakuum wurde der Rückstand mit Wasser verrührt, abgesaugt und gewaschen. Nach dem Trocknen erhielt man 22 g (85,9 % d.Th.) der Titelverbindung vom Fp. 138 - 139°C.

### 2,4-Bismethylamino-6-trifluormethoxy-1,3,5-triazin und 2-Methylamino-4-fluor-6-trifluormethoxy-1,3,5-triazin

5,9 g (0,189 mol) Methylamin wurden bei -70°C innerhalb 30 Minuten unter Rühren in eine Mischung von 19,0 g (0,0945 mol) 2,4-Difluor-6-trifluormethoxy-1,3,5-triazin in 100 ml Diethylether eingegast. Es wurde 2 Stunden bei -70°C und über Nacht unter Erwärmung bis auf 22°C gerührt. Das Reaktionsgemisch wurde im Vakuum eingeengt, in Methylenchlorid aufgenommen und mit Wasser gewaschen. Nach dem Trocknen wurde über eine Kieselgelsäule fraktioniert chromatographiert, wobei man in den ersten beiden Fraktionen 5,0 g (25 % d.Th.) 2-Methylamino-4-fluor-6-trifluormethoxy-1,3,5-triazin vom Fp. 68 -72°C erhielt. In den weiteren Fraktionen 4 - 7 isolierte man 10,7 g (51 % d. Th.) des schwerer löslichen 2,4-Bismethylamino-6-trifluormethoxy-1,3,5-triazins vom Fp. 150 -152°C.

### 2-Amino-4-chlordifluormethoxy-6-fluor-1,3,5-triazin und 2,4-Diamino-6-chlordifluormethoxy-1,3,5-triazin

7,8 g (0,46 mol) Ammoniak wurden innerhalb 45 Minuten unter Rühren bei -70°C in eine Mischung von 50,0 g (0,23 mol) 2,4-Difluor-6-chlordifluormethoxy-1,3,5-triazin ir 150 ml Tetrahydrofuran eingeleitet. Es wurde 2 Stunden bei -70°C und über Nacht unter Erwärmung bis auf 22°C gerührt. Das Reaktionsgemisch wurde im Vakuum eingeengt, mit Wasser gewaschen und getrocknet. Anschließend wurde das Reaktionsprodukt mit Methylenchlorid auf eine Kieselgelsäule geschlämmt und mit gleichem Lösungsmittel eluiert. In den Fraktionen 1 bis 8 erhielt man 21,5 g (43,6 % d.Th.) 2-Amino-4-fluor-6-chlordifluormethoxy-1,3,5-triazin vom Fp. 131 - 133°C. Durch Nachspülen mit Essigester isolierte man dann in den Fraktionen 9 bis 14 das schwerer lösliche 2,4-Diamino-6-chlordifluormethoxy-1,3,5-triazin (11,2 g, 23 % d.Th.) vom Fp. 114°C.

### 2-Chlordifluormethoxy-4-fluor-6-methylamino-1, 3, 5-triazin und 2,4-Bismethylamino-6-chlordifluormethoxy-1,3,5-triazin

5,2 g (0,166 mol) Methylamin wurden innerhalb 20 Minuten unter Rühren bei -70°C in eine Mischung von 18,1 g (0,083 mol) 4-Difluorchlormethoxy-2,6-difluor-1,3,5-triazin eingeleitet. Es wurde 2 Stunden bei -70°C und über Nacht unter Erwärmung bis auf 22°C gerührt. Das Reaktionsgemisch wurde im Vakuum eingeengt, in Methylenchlorid aufgenommen, mit Wasser gewaschen und getrocknet. Nach Chromatographie über Kieselgel erhielt man in den ersten Fraktionen 5,5 g (29 % d.Th.) 2-Chlordifluormethoxy-4-fluor-6-methylamino-1,3,5-triazin vom Fp. 62 -64°C. Im Nachlauf isolierte man 8,7 g (44 % d.Th.) 2,4-Bismethylamino-6-chlordifluormethoxy-1,3,5-triazin vom Fp. 118 - 120°C.

### 2-Amino-4-methoxy-6-trifluormethoxy-1,3,5-triazin

9,1 g (0,05 mol) 30%iges Natriummethylat wurden bei 0°C innerhalb 15 Minuten unter Rühren zu einer Mischung von 10 g (0,05 mol) 2-Amino-4-fluor-6-trifluormethoxy-1,3,5-triazin in 100 ml Methanol gegeben. Nach einer Stunde Rühren bei 0°C wurde im Vakuum eingeengt, in Methylenchlorid aufgenommen und mit Wasser extrahiert. Nach dem Trocknen und Einengen erhielt man 10,5 g (99 % d.Th.) der Titelverbindung vom Fp. 96 - 101°C.

### 2-Amino-4-chlordifluormethoxy-6-methoxy-1,3,5-triazin

8,4 g (0,047 mol) 30%iges Natriummethylat wurden bei 0°C innerhalb 15 Minuten unter Rühren zu einer Mischung von 10 g (0,047 mol) 2-Amino-4-chlordifluormethoxy-6-fluor-1,3,5-triazin in 100 ml Methanol gegeben. Nach einer Stunde Rühren bei 0°C wurde im Vakuum eingeengt, in Methylenchlorid aufgenommen und mit Wasser extrahiert. Nach dem Trocknen und Einengen erhielt man 10,4 g (98,5 % d. Th.) der Titelverbindung vom Fp. 109 - 110°C.

### 2-Amino-4-methoxy-6-trifluormethoxy-1,3,5-triazin

9,1 g (0,05 mol) 30%iges Natriummethylat wurden bei 0°C innerhalb 15 Minuten unter Rühren zu einer Mischung von 10 g (0,05 mol) 2-Amino-4-fluor-6-trifluormethoxy-1,3,5-triazin in 100 ml Methanol gegeben. Nach einer Stunde Rühren bei 0°C wurde im Vakuum eingeengt, in Methylenchlorid aufgenommen und mit Wasser extrahiert. Nach dem Trocknen und Einengen erhielt man 10,5 g (99 % d. Th.) der Titelverbindung vom Fp. 96 bis 101°C.

### 2-Amino-4-chlordifluormethoxy-6-methoxy-1,3,5-triazin

8,4 g (0,047 mol) 30%iges Natriummethylat wurden bei 0°C innerhalb 15 Minuten unter Rühren zu einer Mischung von 10 g (0,047 mol) 2-Amino-4-chlordifluormethoxy-6-fluor-1,3,5-triazin in 100 ml Methanol gegeben. Nach einer Stunde Rühren bei 0°C wurde im Vakuum eingeengt, in Methylenchlorid aufgenommen und mit Wasser extrahiert. Nach dem Trocknen und Einengen erhielt man 10,4 g (98,5 % d. Th.) der Titelverbindung vom Fp. 109 bis 111°C.

### 2-Amino-4-ethoxy-6-trifluormethoxy-1,3,5-triazin

2,3 g (0,093 Mol) 97 % Natriumhydrid wurden portionsweise bei 20 bis 35°C zu 300 ml Ethanol gegeben und 15 Minuten bis zur Lösung gerührt. Bei 0°C wurden dann unter Rühren 18,5 g (0,093 Mol) 2-Amino-4-fluor-6-trifluormethoxy-1,3,5-triazin innerhalb 10 Minuten zugegeben, 1 Stunde bei 0°C und über Nacht bei 22°C gerührt. Nach dem Einengen im Vakuum wurde der Rückstand in Methylenchlorid aufgenommen, mit Wasser extrahiert und getrocknet. Nach dem Einengen erhielt man 17,9 g (85,9 % d. Th.) der Titelverbindung vom Fp. 69 bis 91°C.

### 2-Amino-4-chlordifluormethoxy-6-ethoxy-1, 3, 5-triazin

1,2 g (0,047 mol) 97 % Natriumhydrid wurden bei 20 bis 35°C portionsweise zu 150 ml Ethanol gegeben und 15 Minuten bis zur Lösung gerührt. Anschließend wurde bei 0°C unter Rühren 10,0 g (0,047 mol) 2-Amino-4-chlordifluormethoxy-6-fluor-1,3,5-triazin zugegeben, 1 Stunde bei 0°C und über Nacht bei 22°C gerührt. Nach dem Einengen im Vakuum wurde der Rückstand in Methylenchlorid aufgenommen, mit Wasser extrahiert und getrocknet. Nach dem Einengen erhielt man 10,6 g (94,6 % d. Th.) der Titelverbindung vom Fp. 63 bis 65°C.

### 2-Amino-4-methylamino-6-trifluormethoxy-1, 3, 5-triazin

3,5 g (0,111 mol) Methylamin wurden bei 0°C innerhalb 20 Minuten unter Rühren in eine Lösung von 11 g (0,055 Mol) 2-Amino-4-fluor-6-trifluormethoxy-1,3,5-triazin in 150 ml Tetrahydrofuran eingegast. Es wurde eine Stunde bei 0°C und über Nacht bei 22°C gerührt. Das Reaktionsgemisch wurde im Vakuum eingeengt, mit Wasser verrührt und getrocknet. Man erhielt 10,8 g (93,1 % d. Th.) der Titelverbindung vom Fp. 155 bis 157°C (Zers.).

### 2-Amino-4-chlordifluormethoxy-6-methylamino-1,3,5-triazin

2,9 g (0,093 mol) Methylamin wurden innerhalb 20 Minuten unter Rühren bei 0°C in eine Lösung von 10 g (0,047 mol) 2-Amino-4-chlordifluormethoxy-6-fluor-1,3,5-triazin in 150 ml Diethylether eingegast. Es wurde eine Stunde bei 0°C und über Nacht bei 22°C gerührt. Nach dem Waschen mit Wasser, Trocknen und Einengen erhielt man 9,4 g (89,5 % d. Th.) der Titelverbindung vom Fp. 143°C (Zers.).

### 2-Amino-4-dimethylamino-6-trifluormethoxy-1,3,5-triazin

5,0 g (0,111 mol) Dimethylamin wurden innerhalb 20 Minuten unter Rühren bei 0°C in eine Lösung von 11 g (0,055 mol) 2-Amino-4-fluor-6-trifluormethoxy-1,3,5-triazin in 150 ml Tetrahydrofuran eingegast. Es wurde eine Stunde bei 0°C und über Nacht bei 22°C gerührt. Nach dem Einengen, Waschen mit Wasser und Trocknen erhielt man 9,9 g (80,7 % d. Th.) der Titelverbindung vom Fp. 114 bis 118°C (Zers.).

### 2-Amino-4-chlordifluormethoxy-6-dimethylamino-1,3,5-triazin

4,2 g (0,093 mol) Dimethylamin wurden innerhalb 20 Minuten unter Rühren bei 0°C in eine Lösung von 10 g (0,047 mol) 2-Amino-4-chlordifluormethoxy-6-fluor-1,3,5-triazin in 150 ml Diethylether eingeleitet. Es wurde eine Stunde bei 0°C und über Nacht bei 22°C gerührt. Nach dem Waschen mit Wasser, Trocknen und Einengen erhielt man 9,8 g (87,8 % d. Th.) der Titelverbindung vom Fp. 130 bis 133°C (Zers.).

### 2-Chlor-4-trichlormethoxy-6-trichlormethylpyrimidin

a) 2-Chlor-4-methoxy-6-trichlormethylpyrimidin
   293,1 g (1,692 mol) 30 %ige Natriummethylatlösung wurden innerhalb 1 1/2 Stunden bei 0 bis 5°C unter Rühren zu einer Lösung von 434 g (1,692 mol) 2,6-Dichlor-4-trichlormethylpyrimidin in 1 1 1,2-Dichlorethan gegeben. ES wurde 1 Stunde bei 0 bis 5°C und 12 Stunden bei 25°C gerührt. Das Reaktionsgemisch wurde 4x mit Wasser und 3x mit gesättigter Kochsalzlösung extrahiert. Nach dem Trocknen über Magnesiumsulfat und Einengen erhielt man 423 g (95 % d. Th.) der Titelverbindung als fast farbloses Öl. ¹H-NMR (CDCl₃) (ppm) OCH₃ (s/3H) 4,1; CH (s/1H) 7,25.
b) 2-Chlor-4-trichlormethoxy-6-trichlormethylpyrimidin
   In eine Mischung von 210 g (0,802 mol) 2-Chlor-4-methoxy-6-trichlormethylpyrimidin und 260 mg (0,0016 mol), α,α'-Azoisobutyronitril wurden unter UV-Bestrahlung und gaschromatographischer Kontrolle des Reaktionsverlaufs bei einer Temperatur von zunächst 110°C Chlor eingeleitet, wobei sich auch nach Entfernen der Heizbades eine Reaktionstemperatur von 140°C einstellte. Nach dem Abklingen der Reaktion wurden während 5 1/2 Stunden bei 120°C insgesamt 341 g (4,8 mol) Chlor eingeleitet. Zu dem erkaltenden Reaktionsgemisch rührte man ab 40°C 70 ml n-Pentan zur Ausfällung hinzu. Der Niederschlag wurde abgesaugt, mit Petrolether gewaschen und getrocknet, wobei man 163 g (55 % d. Th.) der Titelverbindung vom Fp. 67-69°C erhielt.
   Das Filtrat (113,8 g) bestand nach dem Gaschromatogramm zu 83 % aus der Titelverbindung, 4 % 2-Chlor-4-dichlormethoxy-6-trichlormethylpyrimidin und 9 % 2,4-Dichlor-6-trichlormethylpyrimidin. Die Gesamtausbeute der Titelverbindung betrug 87,6 % der Theorie.

### 2, 4-Difluor-6-trichlormethoxy-pyrimidin

210 g (2,96 mol) Chlor wurden unter UV-Bestrahlung und gaschromatographischer Kontrolle des Reaktionsverlaufs während 2 1/2 Stunden unter Rühren bei 130°C in 123 g (0,843 mol) 2,4-Difluor-6-methoxy-pyrimidin, das nach dem in der EP-A 378 089 beschriebenen Verfahren hergestellt wurde, eingeleitet. Das Reaktionsgemisch wurde über eine 10-cm-Vigreux-Kolonne im Vakuum destilliert, wobei man 190,2 g (90,5 % d. Th.) der Titelverbindung vom Kp. 40-43°C/0,2 mbar erhielt.

### 2,4-Dichlor-6-trichlormethoxy-pyrimidin

Unter Rühren, UV-Bestrahlung und gaschromatographischer Kontrolle des Reaktionsverlaufs wurden 303 g (4,27 mol) Chlor während 1/2 Stunde bei 80°C, 1 Stunde bei 100°C, 3 Stunden bei 120°C und 3 Stunden bei 150°C in eine Mischung von 209 g (1,168 mol) 2,6-Dichlor-4-methoxy-pyrimidin und 2 g (0,012 mol) α,α'-Azoisobutyronitril eingeleitet. Anschließend wurde das Reaktionsgemisch im Vakuum destilliert. Man erhielt 241,3 g (73 % d. Th.) der Titelverbindung vom kp. 87-88°C/0,4 mbar; Fp. 55-56°C.

### 2,4-Difluor-6-trifluormethoxy-pyrimidin

49,9 g (0,2 mol) 2,4-Difluor-6-trichlormethoxy-pyrimidin wurde bei 100°C innerhalb 15 Minuten unter Rühren zu einer Mischung von 39,3 g (0,22 mol) Antimontrifluorid und 9,38 g (0,031 mol) Antimonpentachlorid gegeben. Innerhalb 25 Minuten wurde die Badtemperatur von 100 auf 150°C erhöht und 30 Minuten nachgerührt, wobei sich zwischen 120 bis 125°C Rückfluβ einstellte. Durch anschließende Destillation erhielt man 37,1 g (92,7 % d. Th.) der Titelverbindung vom Kp. 125-127°C.

### 6-Chlordifluormethoxy-2,4-difluor-pyrimidin

93 g (0,373 mol) 2,4-Difluor-6-trichlormethoxy-pyrimidin wurden bei 100°C innerhalb 10 Minuten unter Rühren zu einer Mischung von 44,5 g (0,249 mol) Antimontrifluorid und 0,94 g (0,0031 mol) Antimonpentachlorid gegeben. Innerhalb 25 Minuten wurde die Badtemperatur von 100 auf 175°C erhöht, wobei sich bei 145°C Rückfluß einstellte. Nach 1 1/2 Stunden Rühren wurde das Reaktionsprodukt bei 146-150°C abdestilliert. Das Destillat wurde in Methylenchlorid gelöst, mit 6 n Salzsäure extrahiert und über Magnesiumsulfat getrocknet. Nach dem Einengen im Vakuum erhielt man als Rückstand die Titelverbindung in einer Ausbeute von 63,7 g = 78,8 % d. Th.

### 2-Fluor-4-trifluormethoxy-6-trifluormethyl-pyrimidin

80 g (0,219 mol) 2-Chlor-4-trichlormethyl-6-trichlormethoxy-pyrimidin wurden innerhalb 5 Minuten unter Rühren bei 100° zu einer Mischung von 93,9 g (0,525 mol) Antimontrifluorid und 18,7 g (0,0627 mol) Antimonpentachlorid gegeben. Innerhalb 10 Minuten wurde die Badtemperatur auf 140°C erhöht und 1 Stunde nachgerührt, wobei sich starker Rückfluß einstellte. Das Reaktionsprodukt wurde bei 135-140°C, gegen Schluß bei 95°C/50 mbar, überdestilliert. Das Destillat wurde in Methylenchlorid aufgenommen, mit 6 n Salzsäure extrahiert und über Magnesiumsulfat getrocknet. Nach dem Einengen im Vakuum erhielt man die Titelverbindung in einer Ausbeute von 35,9 g (65,5 % d. Th.).

### 2,4-Dichlor-6-trifluormethoxy-pyrimidin

115 g (0,407 mol) 2,4-Dichlor-6-trichlormethoxy-pyrimidin wurden innerhalb 5 Minuten unter Rühren bei 100°C zu einer Mischung von 80 g (0,447 mol) Antimontrifluorid und 18,77 g (0,0627 mol) Antimonpentachlorid gegeben, wobei sich die Reaktionstemperatur bis auf 140°C erhöhte. Es wurde noch 45 Minuten bei 150°C gerührt. Zur Destillation wurde ein Druck von 210 mbar eingestellt, wobei die Titelverbindung bei 128°C überging; letzte flüchtige Bestandteile wurden bei 110°C/22 mbar übergetrieben. Das Destillat wurde in Methylenchlorid gelöst, mit 6 n Salzsäure extrahiert und über Magnesiumsulfat getrocknet. Nach dem Einengen im Vakuum erhielt man die Titelverbindung in einer Ausbeute von 80 g (84,4 % d. Th.) als farbloses öl vom n_{D}²⁵ = 1,4604.

### 2-Amino-4-chlordifluormethoxy-6-fluor-pyrimidin

9,8 g (0,578 mol) gasförmiges Ammoniak wurden innerhalb einer Stunde bei -75 bis -70°C unter Rühren in eine Mischung von 62,5 g (0,289 mol) 2,4-Difluor-6-chlordifluormethoxy-pyrimidin in 300 ml Tetrahydrofuran eingeleitet. Es wurde eine Stunde bei -70°C gerührt und dann auf Raumtemperatur erwärmt. Der ausgefallene Niederschlag wurde abgesaugt, zwischen Essigester und Wasser verteilt und die organische Phase über Magnesiumsulfat getrocknet. Das Reaktionsfiltrat wurde eingeengt, in der obigen Essigesterphase gelöst, über Kieselgel mit Petrolether:Ether = 5:1 chromatographiert und eingeengt. Man erhielt 46,5 g (75,3 % d. Th.) der Titelverbindung als farblose Kristalle vom Fp. 77-80°C.

### 2-Amino-4-fluor-6-trifluormethoxy-pyrimidin

8,7 g (0,51 mol) gasförmiges Ammoniak wurden innerhalb 1 Stunde bei -75 bis -70°C unter Rühren in eine Mischung von 51 g (0,255 mol) 2,4-Difluor-6-trifluormethoxy-pyrimidin in 200 ml Diethylether eingeleitet. Es wurde noch 1 1/2 Stunden bei -70°C und 1 Stunde bei Raumtemperatur gerührt. Das Reaktionsgemisch wurde im Vakuum eingeengt, in Methylenchlorid aufgenommen und mit Wasser extrahiert. Nach dem Trocknen der organischen Phase, Einengen und Chromatographieren über Kieselgel mit Petrolether:Ether = 8:1 erhielt man 38,1 g (75,6 % d. Th.) der Titelverbindung als farblose Kristalle vom Fp. 86-89°C.

### 2-Amino-4-chlor-6-trifluormethoxy-pyrimidin

4,3 g (0,25 mol) gasförmiges Ammoniak wurden innerhalb 45 Minuten unter Rühren bei -50 bis -45°C in eine Mischung von 23,3 g (0,1 mol) 2,4-Dichlor-6-trifluormethoxy-pyrimidin in 150 ml Methyl-tert.-butylether eingeleitet. Es wurde 30 Minuten bei -50°C, 1 Stunde bei -30°C und 1 Stunde bei 25°C gerührt. Der ausgefallene Niederschlag wurde abgesaugt, mit Wasser gewaschen und getrocknet, wobei man 5,4 g (33,1 % d. Th.) 4-Amino-2,4-dichlorpyrimidin vom Fp. 270-272°C als Nebenprodukt erhielt. Das Filtrat wurde mit Wasser gewaschen, getrocknet, teilweise im Vakuum eingeengt und mit Petrolether:Ether = 5:1 frakioniert chromatographiert, wobei man in den ersten Fraktionen 3 g (12,8 % d. Th.) Ausgangsmaterial als farbloses Öl und im Nachlauf 9 g (42 % d. Th.) der Titelverbindung als farblose Kristalle vom Fp. 55-56°C erhielt. Der Umsatz betrug 48,3 %.

### 4-Chlordifluormethoxy-6-fluor-2-methylamino-pyrimidin

20,3 g (0,0938 mol) 4-Chlordifluormethoxy-2,6-difluor-pyrimidin wurden in 150 ml Tetrahydrofuran vorgelegt und bei -70 bis -60°C innerhalb 30 Minuten unter Rühren mit 5,8 g (0,188 mol) gasförmigem Methylamin versetzt. Es wurde jeweils 1 Stunde bei -70°C, 0°C und 25°C gerührt. Nach dem Einengen des Reaktionsgemisches im Vakuum wurde der Rückstand mit Wasser verrührt, 2x mit Essigester extrahiert und der Extrakt über Magnesiumsulfat getrocknet. Es wurde zum Teil im Vakuum eingeengt und dann über Kieselgel mit Ether/Petrolether 1:5 fraktioniert chromatographiert. Die ersten Fraktionen enthielten die Titelverbindung vom Fp. 57-61 °C in einer Ausbeute von 12,5 g (58,5 %).

### 2-Amino-4-trifluormethoxy-6-trifluormethyl-pyrimidin

4,7 g (0,278 mol) gasförmiges Ammoniak wurden unter Rühren bei -75 bis -70°C innerhalb 1 Stunde in eine Mischung von 38,0 g (0,147 mol) 2-Fluor(chlor)-4-trifluormethoxy-6-trifluormethyl-pyrimidin in 150 ml Diethylether eingeleitet. Es wurde jeweils 2 Stunden bei -75°C und nach dem Erwärmen bei 25°C gerührt. Nach dem Absaugen von dem ausgefallenen Niederschlag wurde die organische Phase mit Wasser extrahiert, getrocknet und teilweise eingeengt. Nach dem Chromatographieren mit Methyl-tert.-butylether über Kieselgel erhielt man 20,4 g (56,1 % d. Th.) der Titelverbindung vom Fp. 47-49°C.

### 2-Amino-4-methoxy-6-trifluormethoxy-pyrimidin

2,7 g (0,015 mol) 30%iges Natriummethylat wurden innerhalb 15 Minuten unter Rühren bei -5 bis 0°C zu 2,95 g (0,015 mol) 2-Amino-4-fluor-6-trifluormethoxy-pyrimidin in 50 ml Methanol gegeben. Nach 1 Stunde Rühren bei 0°C und Erwärmen auf 25°C wurde das Reaktionsgemisch im Vakuum eingeengt, mit Wasser verrührt und 2x mit Methylenchlorid extrahiert. Nach dem Trocknen und Einengen im Vakuum erhielt man 3,1 g (98 % d. Th.) der Titelverbindung mit n_{D}²⁵ = 1,4770.

### 2-Amino-4-chlordifluormethoxy-6-methoxy-pyrimidin

26,1 g (0,145 mol) 30%iges Natriummethylat wurden innerhalb 15 Minuten unter Rühren bei -10 bis 0°C zu 31,0 g (0,145 mol) 2-Amino-4-chlordifluormethoxy-6-fluorpyrimidin in 300 ml Methanol gegeben. Es wurde 30 Minuten bei 0°C und 1 Stunde bei 25°C gerührt. Das Reaktionsgemisch wurde im Vakuum eingeengt und wie oben aufgearbeitet. Man erhielt 31,6 g (96,6 % d. Th.) der Titelverbindung als farbloses Öl mit n_{D}²² = 1,5039.

### 4-Chlordifluormethoxy-2-methylamino-6-methoxy-pyrimidin

4,7 g (0,026 mol) 30%iges Natriummethylat wurden innerhalb 10 Minuten unter Rühren bei 0°C zu 6,0 g (0,0263 mol) 4-Chlordifluormethoxy-6-fluor2-methylamino-pyrimidin in 100 ml Methanol gegeben. Es wurde jeweils 1 Stunde bei 0°C und bei 25°C gerührt. Nach üblicher Aufarbeitung erhielt man 6,3 g (100 % d. Th.) der Titelverbindung vom Fp. 49-53°C.

### 4-Chlordifluormethoxy-6-dimethylamino-2-methylamino-pyrimidin

1,9 g (0,0417 mol) gasförmiges Dimethylamin wurden innerhalb 10 Minuten unter Rühren bei 0°C in eine Mischung von 8,9 g (0,0417 mol) 2-Amino-4-chlordifluormethoxy-6-fluor-pyrimidin in 100 ml Tetrahydrofuran eingeleitet. Es wurde 1 Stunde bei 0°C und 2 Stunden bei 25°C gerührt. Nach üblicher Aufarbeitung erhielt man 9,7 g (97,5 % d. Th.) der Titelverbindung vom Fp. 127-130°C.

### Herstellung der Endprodukte I

### 1. Methansulfonsäure[2-[[(4-methoxy-6-trifluormethoxypyrimidin-2-yl)aminocarbonyl]aminosulfonyl]phenyl]ester

Eine Lösung von 3 g 2-Amino-4-methoxy-6-trifluormethoxypyrimidin (14 mmol) in 10 g 1,2-Dichlorethan wird bei 25°C mit 4 g Methansulfonsäure(2-isocyanatosulfonylphenyl)ester (14 mmol) versetzt. Man rührt 10 min nach, entfernt das Lösungsmittel im Ether/Pentan (1:1, v:v). Das kristalline Produkt wird abgesaugt und im Wasserstrahlvakuum bei 40°C getrocknet. Man erhält so 5 g der Titelverbindung (73 % d. Th.) mit Fp.146-149°C.

### 2. Methansulfonsäure[2-[[(4-fluor-6-methoxypyrimidin-2-yl)amino carbonyl]aminosulfonyl]phenyl]ester

Eine Suspension von 2 g 2-Amino-4-methoxy-6-trifluormethoxypyrimidin (14 mmol) in 10 g 1,2-Dichlorethan wird bei 25°C mit 4 g Methansulfonsäure(2-isocyanatosulfonylphenyl)ester (14 mmol) versetzt. Es bildet sich eine homogene Lösung, aus der sich nach etwa 30 min ein voluminöser, weißer Niederschlag abscheidet. Das Produkt wird abgesaugt, mit wenig 1,2-Dichlorethan gewaschen und im Wasserstrahlvakuum bei 40°C getrocknet. Man erhält so 2 g der Titelverbindung (34 % d. Th.) mit Fp. 168-169°C.

### 3. Natrium[methansulfonsäure[2-[[(4-methoxy-6-trifluormethoxy-1 ,3,5-tri-azin-2-yl)aminocarbonyl]aminosulfonyl]phenyl]ester]

Eine Lösung von 3 g Methansulfonsäure[2-[[(4-methoxy-6-trifluormethoxy-1,3,5-triazin-2-yl)aminocarbonyl]aminosulfonyl]phenyl]ester (6.2 mmol) in 30 ml Methanol wird bei 25°C mit 1.1 g (6.2 mmol) einer Lösung von Natriummethanolat (30 gew.-proz.) in Methanol versetzt. Man rührt 2 min bei 25°C und entfernt das Lösungsmittel bei 80°C im Wasserstrahlvakuum. Man erhält so die Titelverbindung in quantitativer Ausbeute mit Zersetzungspunkt 130-135°C.

Die in der nachfolgenden Tabelle 1 genannten Wirkstoffe wurden auf analogem Herstellungsweg erhalten.

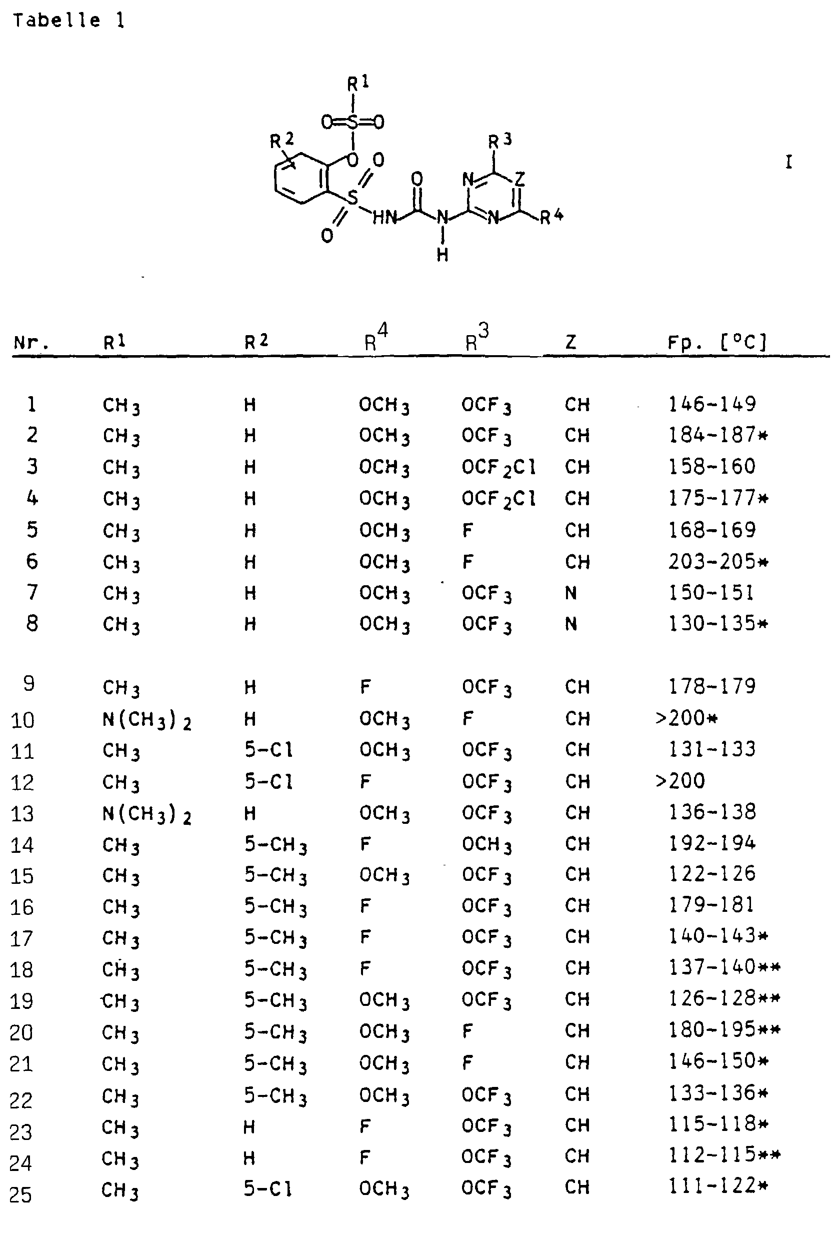

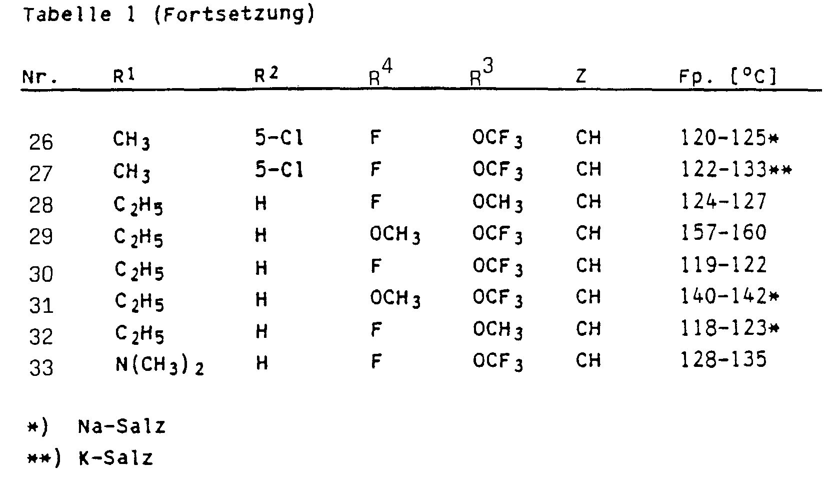

Beispiele für weitere herbizid wirksame Sulfonylharnstoffderivate I, die in analoger Weise erhältlich sind, sind nachfolgend in Tabelle 2 aufgeführt. Hierbei wurden die vereinfachte Formeln I' und I" zugrundegelegt,

A_{x-y}-SO₂-NH-CO-NH-Tₙ I'

A_{x-y}-SO₂-NH-CO-NH-Pₙ Iʺ

in denen A_{x-y} für einen aromatischen Rest der Formel 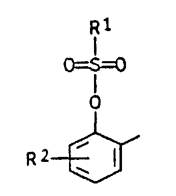 steht, die Laufvariable x den Rest R¹ und die Laufvariable y den Rest R² kennzeichnen. Es bedeuten:

| x | R¹ | y | R² |
|---|---|---|---|
| 1 | CH₃ | 1 | H |
| 2 | C₂H₅ | 2 | 3-F |
| 3 | n-C₃H₇ | 3 | 5-F |
| 4 | i-C₃H₇ | 4 | 6-F |
| 5 | n-C₄H₉ | 5 | 3-Cl |
| 6 | i-C₄H₉ | 6 | 5-Cl |
| 7 | S-C₄H₉ | 7 | 6-Cl |
| 8 | t-C₄H₉ | 8 | 3-OCH₃ |
| 9 | CF₃ | 9 | 5-OCH₃ |
| 10 | ClCH₂ | 10 | 6-OCH₃ |
| 11 | CH₃OCH₂ | 11 | 3-CH₃ |
| 12 | NHCH₃ | 12 | 5-CH₃ |
| 13 | NHC₂H₅ | 13 | 6-CH₃ |
| 14 | N(CH₃)₂ | | |
| 15 | N(C₂H₅)₂ | | |
| 16 | Phenyl | | |
| 17 | 2-F-Phenyl | | |
| 18 | 3-F-Phenyl | | |
| 19 | 4-F-Phenyl | | |
| 20 | 2-Cl-Phenyl | | |
| 21 | 3-Cl-Phenyl | | |
| 22 | 4-Cl-Phenyl | | |
| 23 | 2-CH₃-Phenyl | | |
| 24 | 3-CH₃-Phenyl | | |
| 25 | 4-CH₃-Phenyl | | |
| 26 | 2-t-C₄H₉-Phenyl | | |
| 27 | 3-t-C₄H₉-Phenyl | | |
| 28 | 4-t-C₄H₉-Phenyl | | |
| 29 | 2-OCH₃-Phenyl | | |
| 30 | 3-OCH₃-Phenyl | | |
| 31 | 4-OCH₃-Phenyl | | |

Pn bzw. Tn kennzeichnen die Pyrimidin- oder 1,3,5-Triazinreste. Dabei bedeutet:

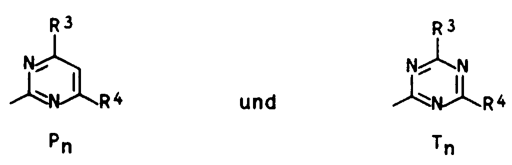

| n | R³ | R⁴ |
|---|---|---|
| 1 | F | F |
| 2 | F | Cl |
| 3 | F | OCH₃ |
| 4 | F | OC₂H₅ |
| 5 | F | CH₃ |
| 6 | F | C₂H₅ |
| 7 | F | CF₃ |
| 8 | F | OCF₃ |
| 9 | F | OCF₂Cl |
| 10 | F | OCF₂H |
| 11 | F | N(CH₃)₂ |
| 12 | OCF₃ | F |
| 13 | OCF₃ | Cl |
| 14 | OCF₃ | OCH₃ |
| 15 | OCF₃ | OC₂H₅ |
| 16 | OCF₃ | CH₃ |
| 17 | OCF₃ | C₂H₅ |
| 18 | OCF₃ | CF₃ |
| 19 | OCF₃ | OCF₃ |
| 20 | OCF₃ | OCF₂Cl |
| 21 | OCF₃ | OCF₂H |
| 22 | OCF₃ | NHCH₃ |
| 23 | OCF₃ | N(CH₃)₂ |
| 24 | OCF₂Cl | F |
| 25 | OCF₂Cl | Cl |
| 26 | OCF₂Cl | OCH₃ |
| 27 | OCF₂Cl | OC₂H₅ |
| 28 | OCF₂Cl | CH₃ |
| 29 | OCF₂Cl | C₂H₅ |
| 30 | OCF₂Cl | CF₃ |
| 31 | OCF₂Cl | OCF₃ |
| 32 | OCF₂Cl | OCF₂Cl |
| 33 | OCF₂Cl | OCF₂H |
| 34 | OCF₂Cl | NHCH₃ |
| 35 | OCF₂Cl | N(CH₃)₂ |
| 36 | OCF₂Br | F |
| 37 | OCF₂Br | Cl |
| 38 | OCF₂Br | OCH₃ |
| 39 | OCF₂Br | OC₂H₅ |
| 40 | OCF₂Br | CH₃ |
| 41 | OCF₂Br | C₂H₅ |
| 42 | OCF₂Br | CF₃ |
| 43 | OCF₂Br | OCF₃ |
| 44 | OCF₂Br | OCF₂Cl |
| 45 | OCF₂Br | OCF₂H |
| 46 | OCF₂Br | NHCH₃ |
| 47 | OCF₂Br | N(CH₃)₂ |

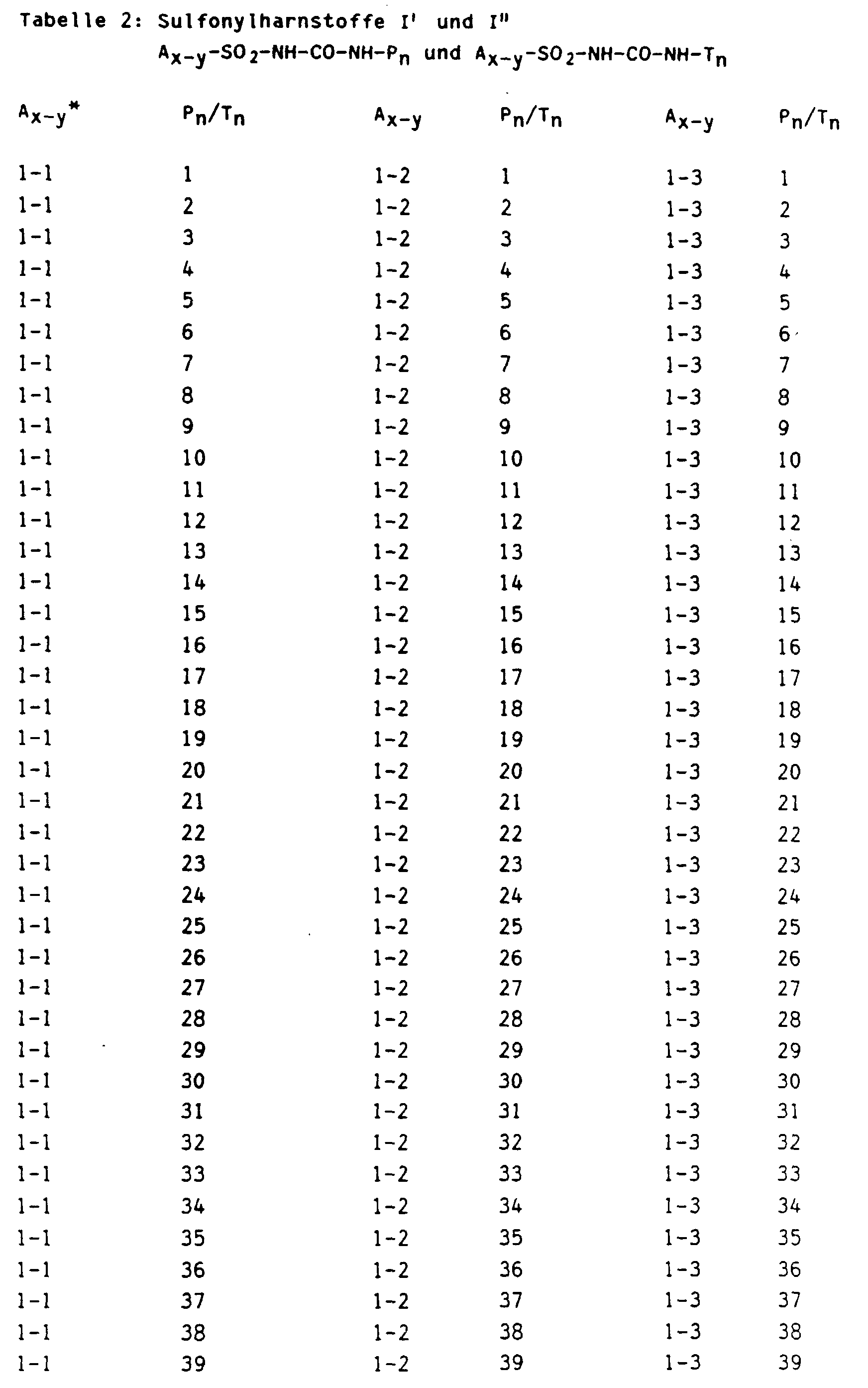

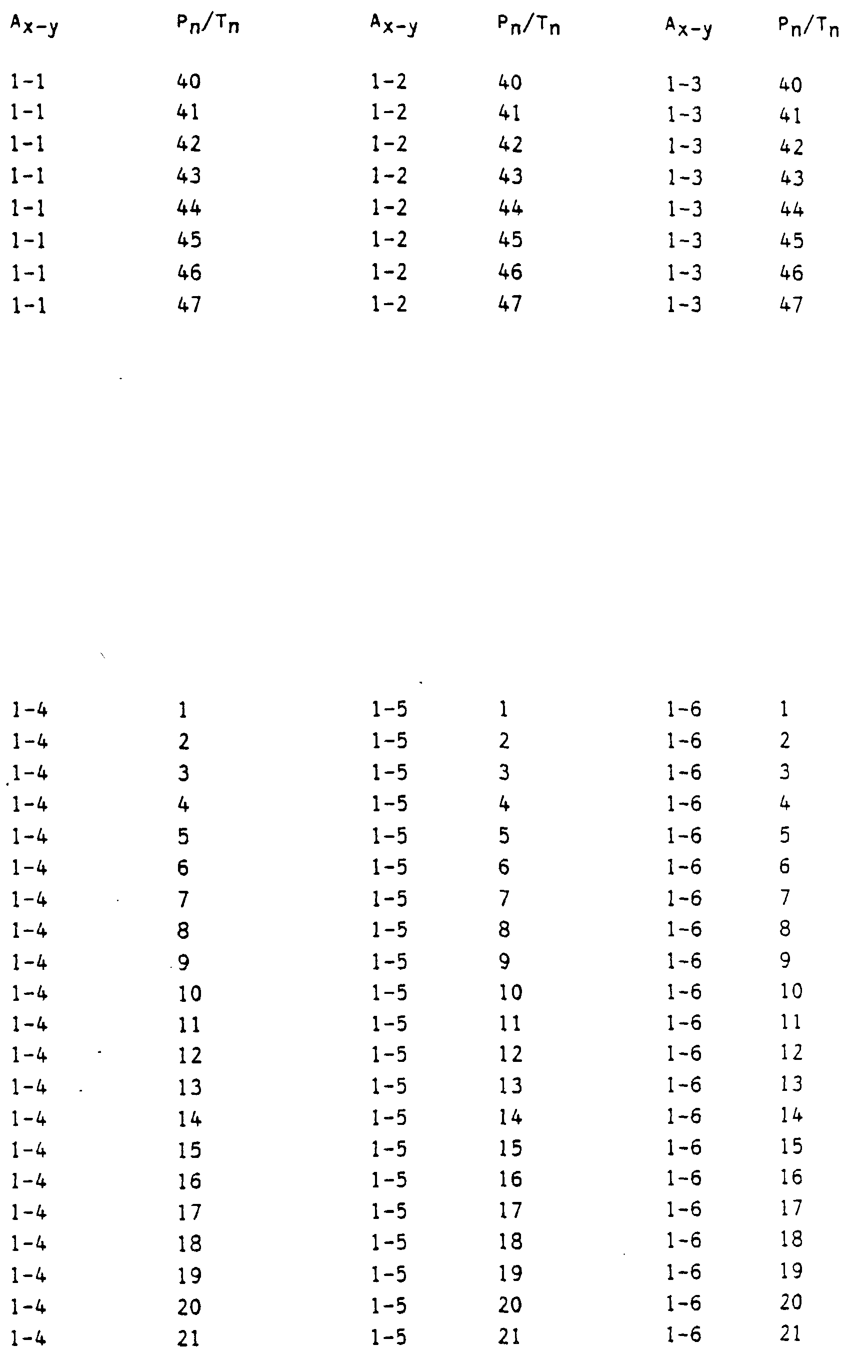

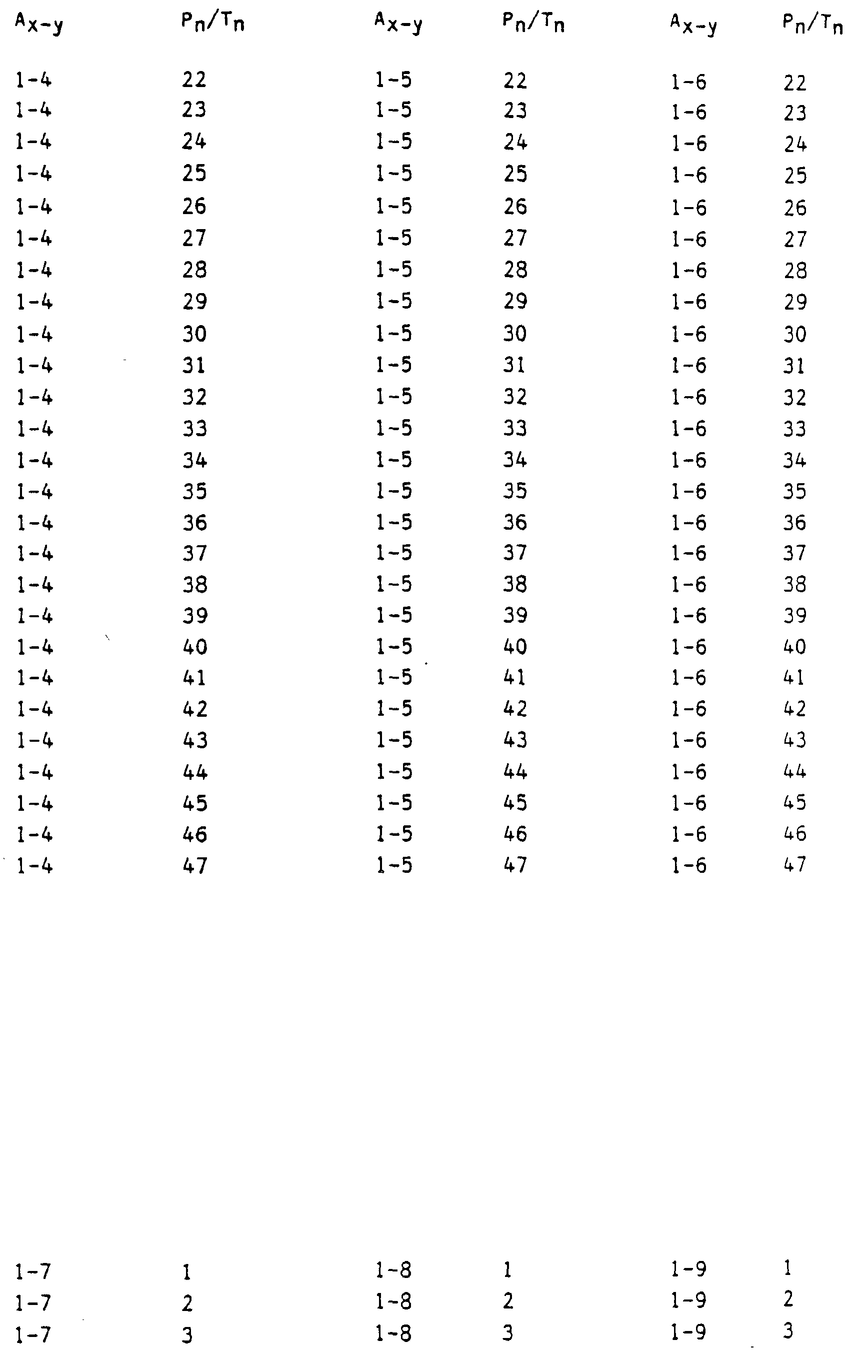

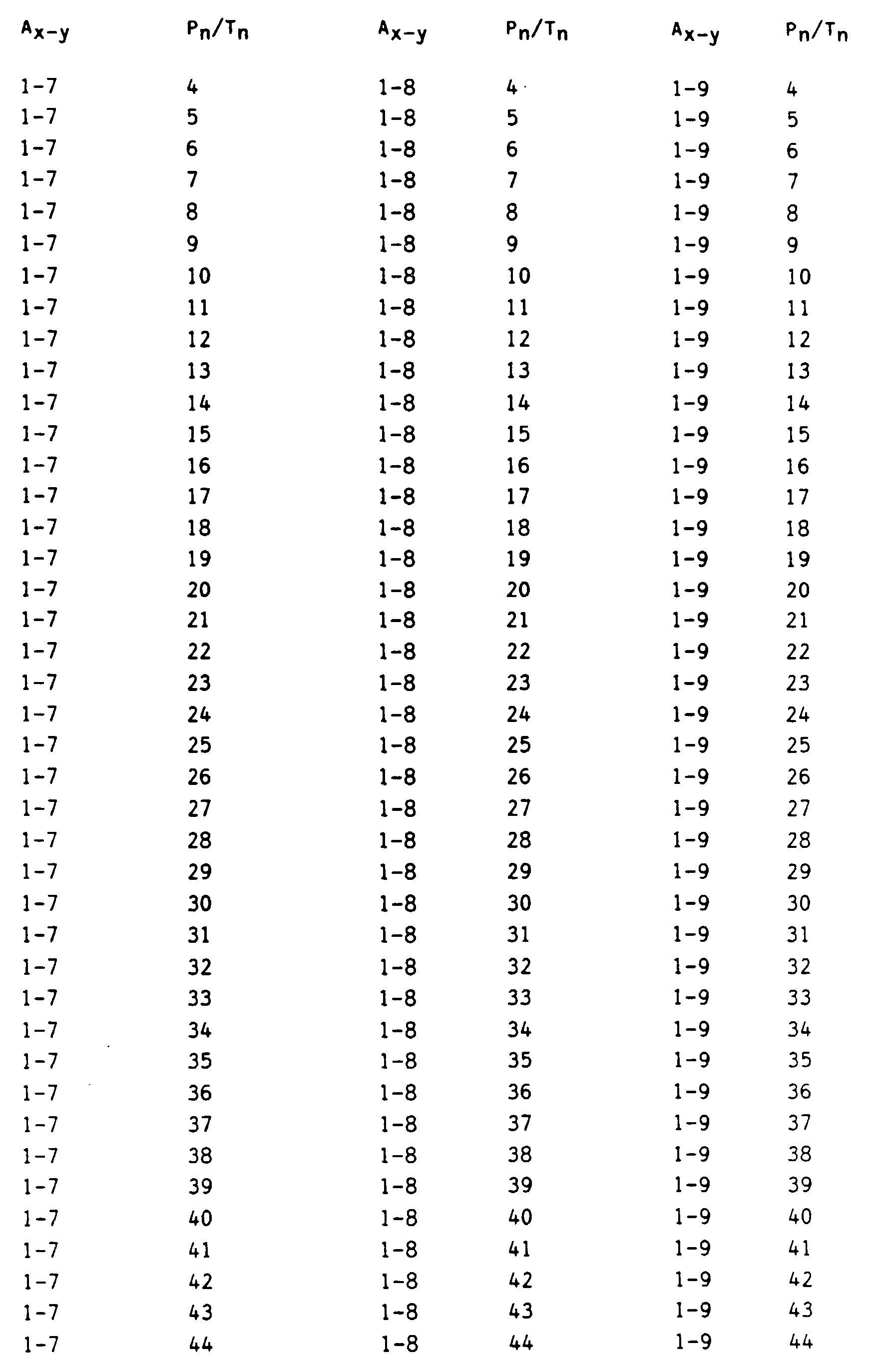

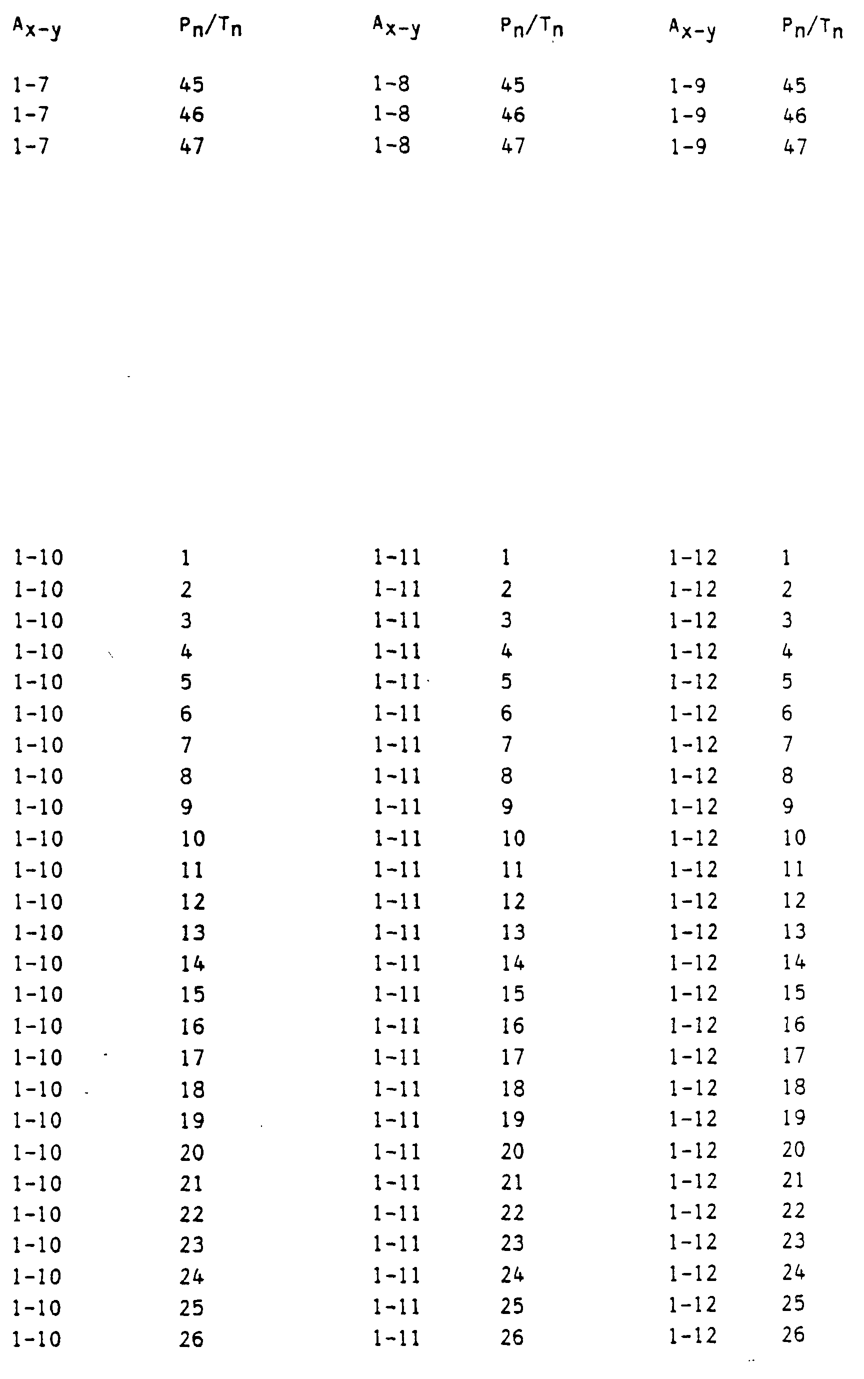

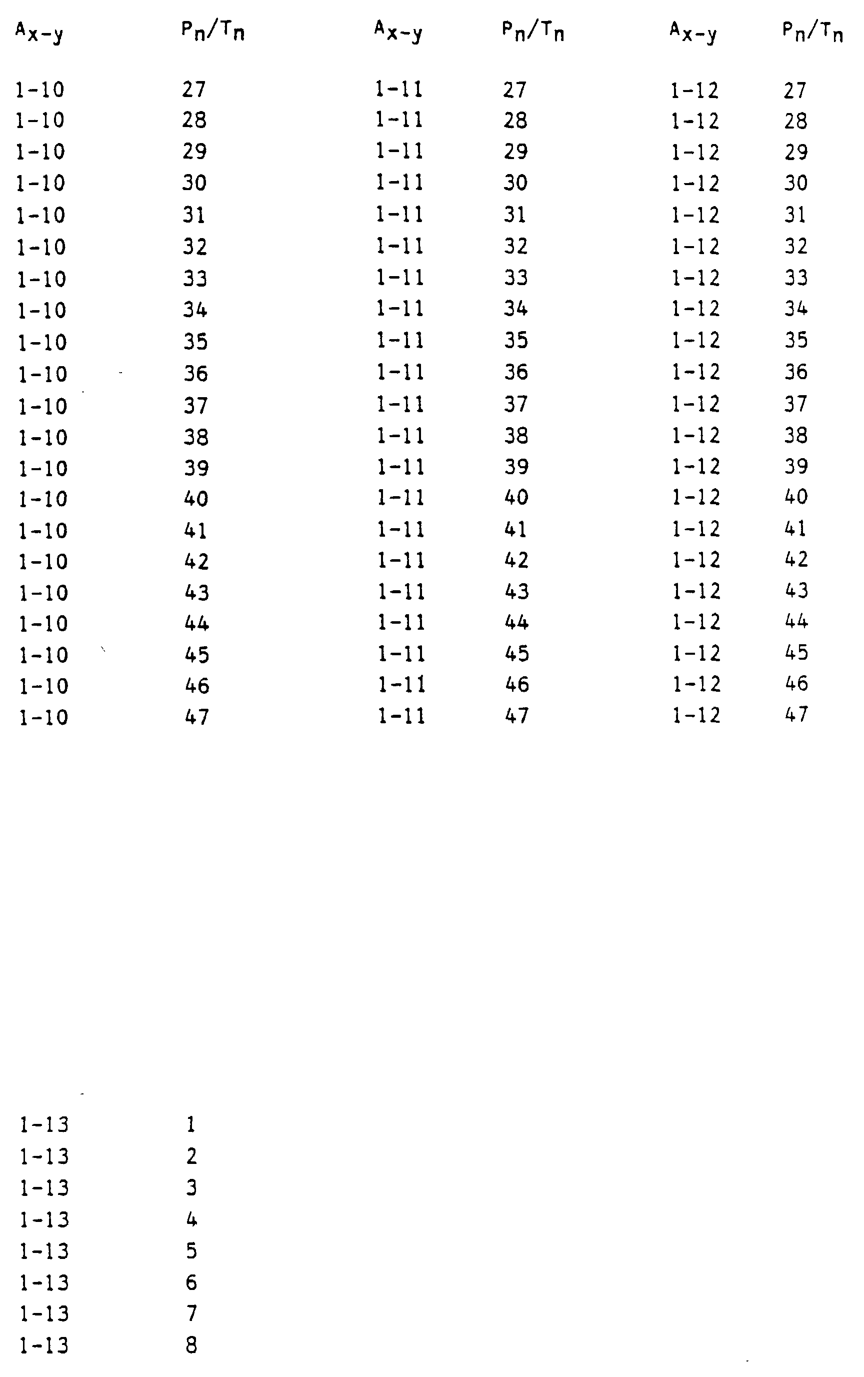

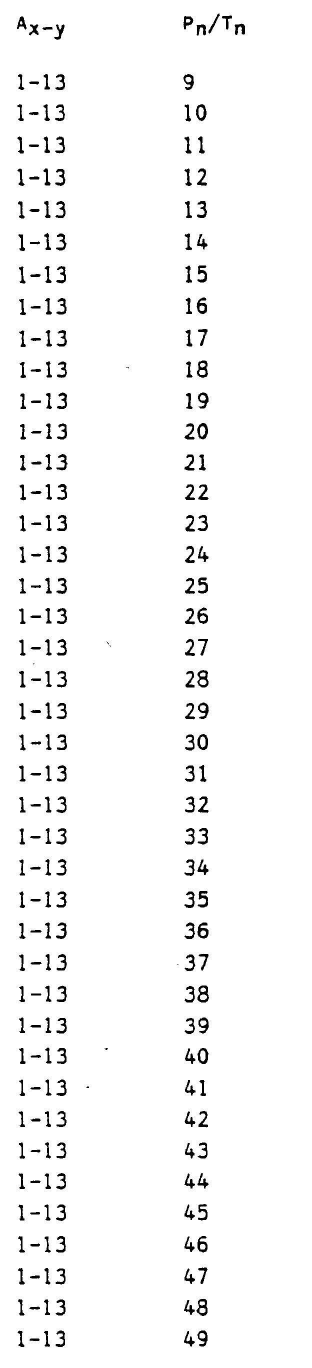

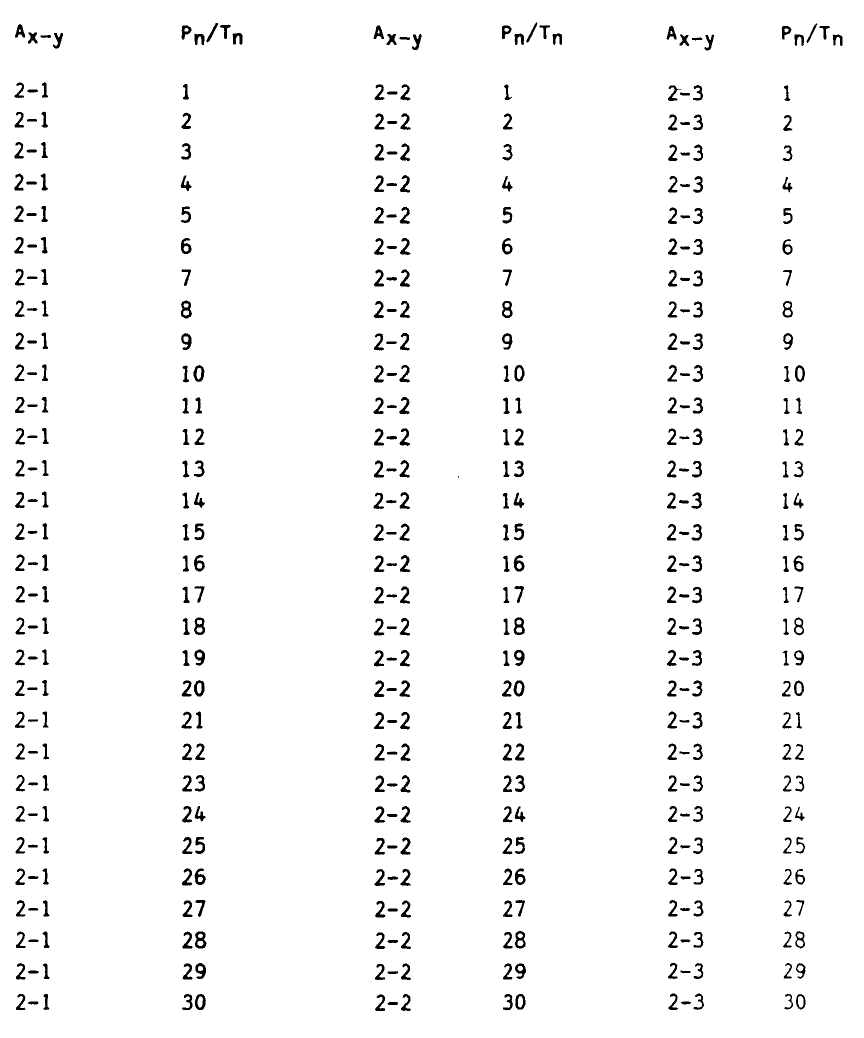

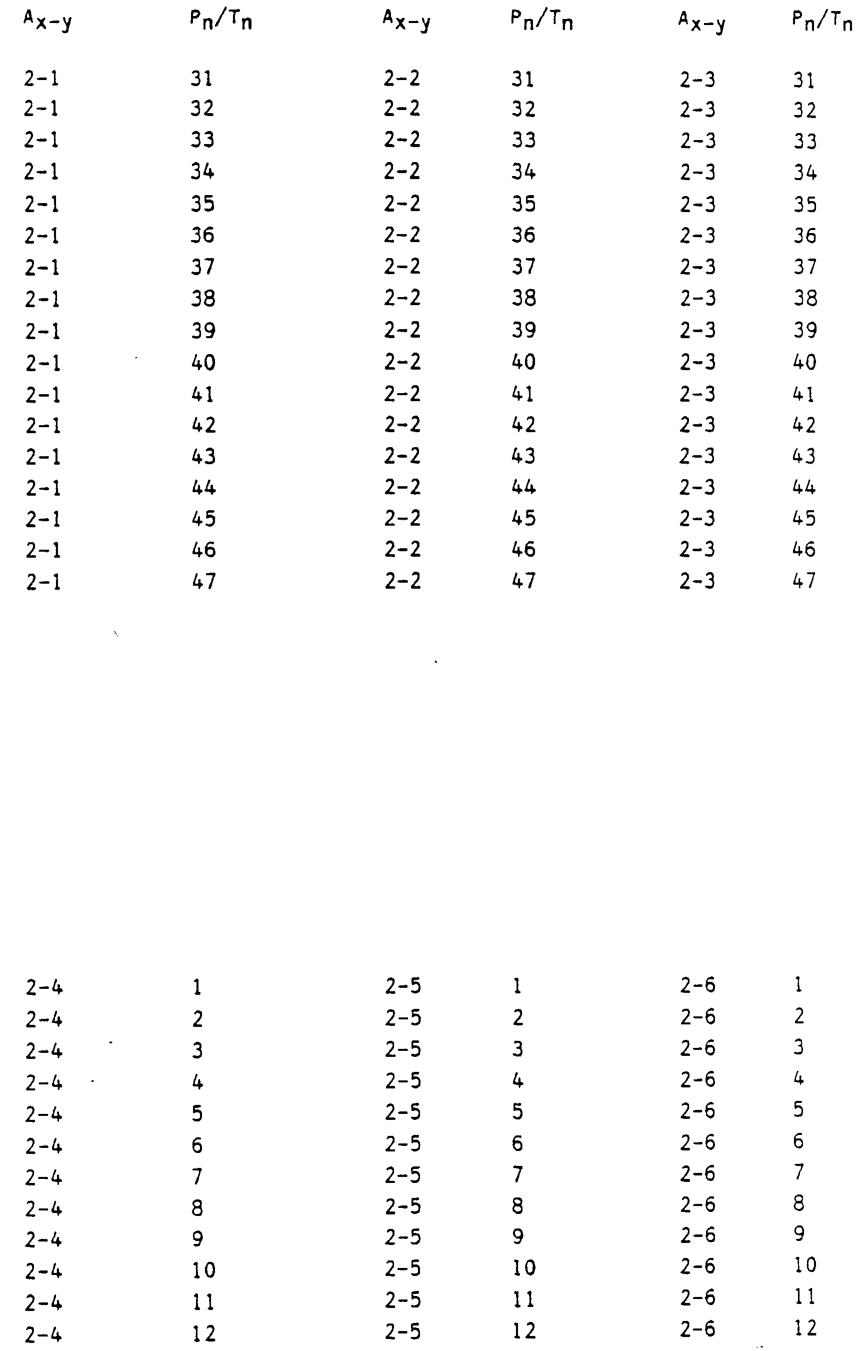

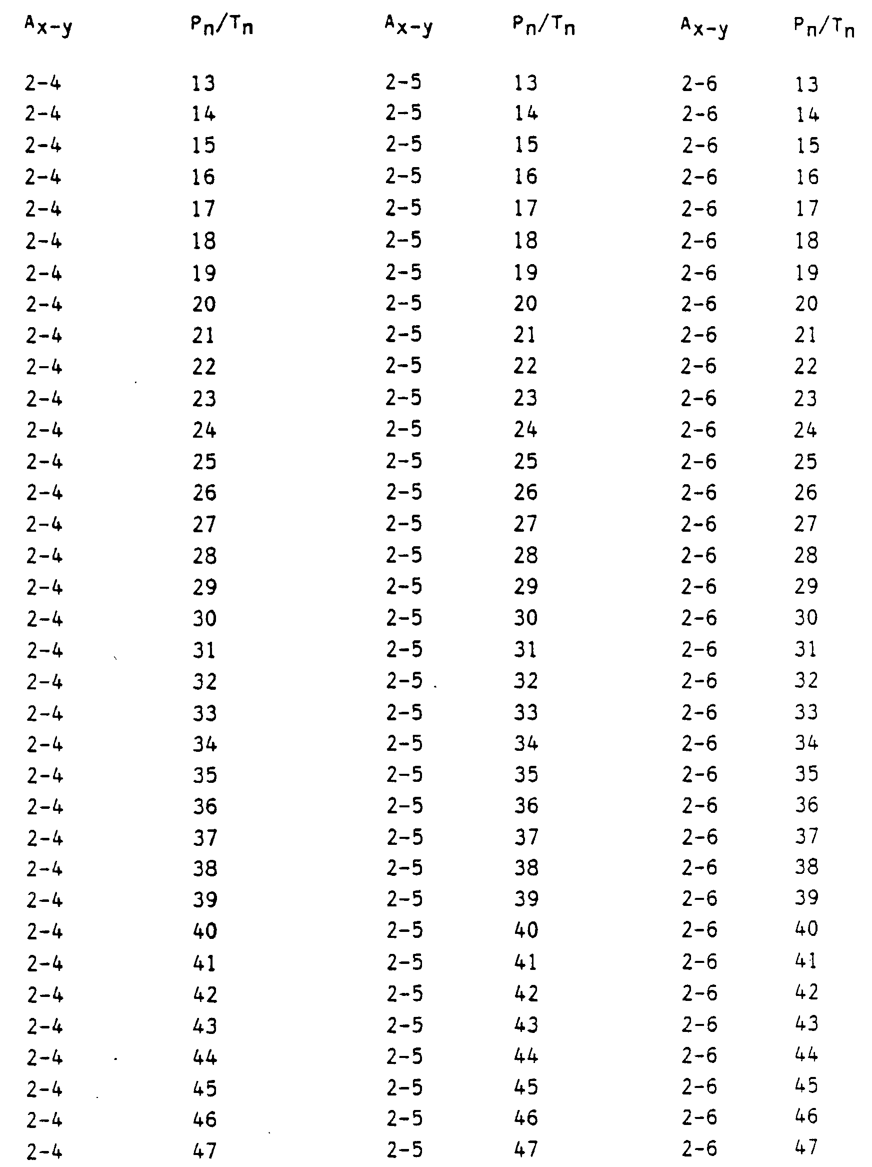

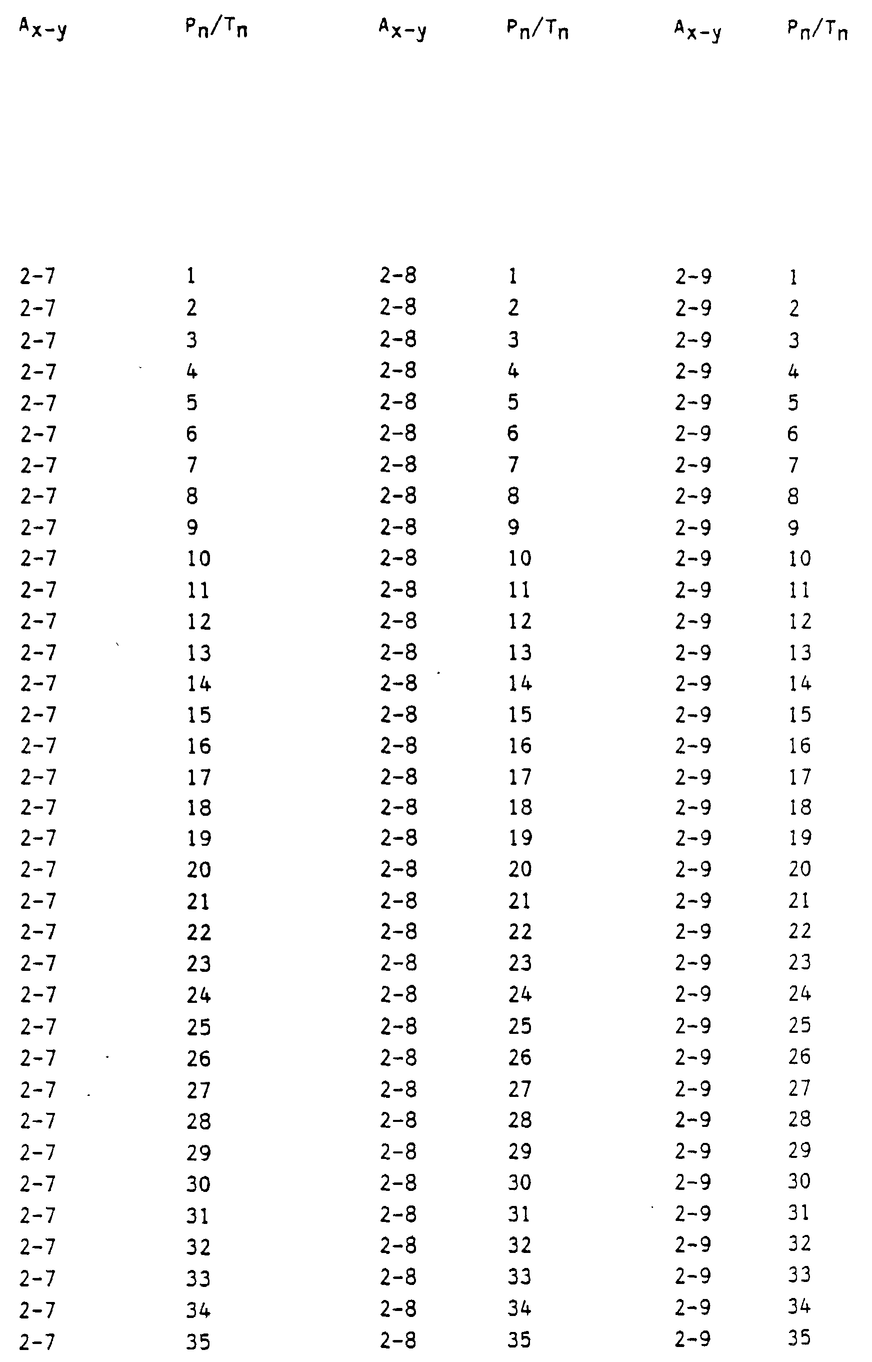

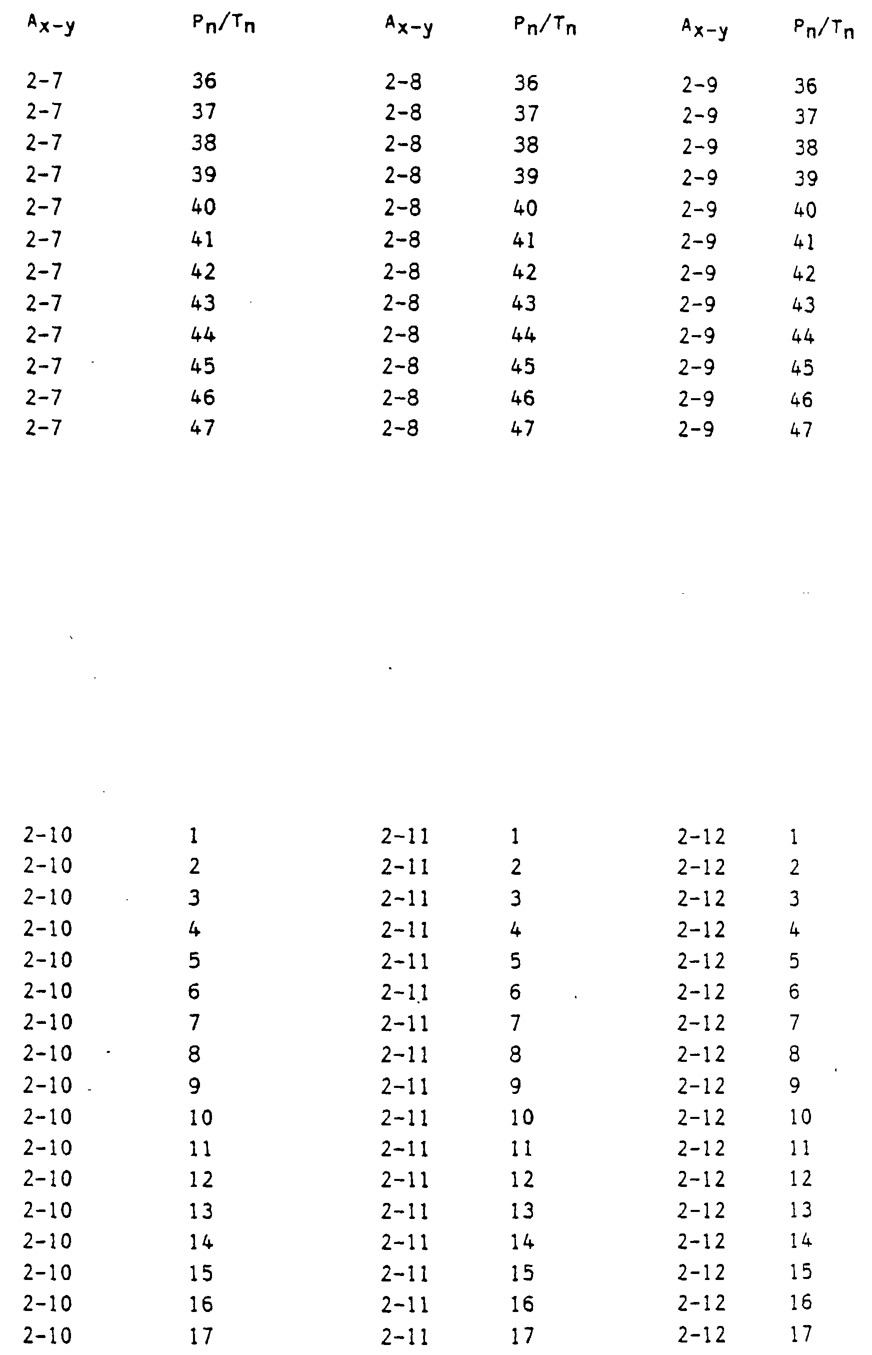

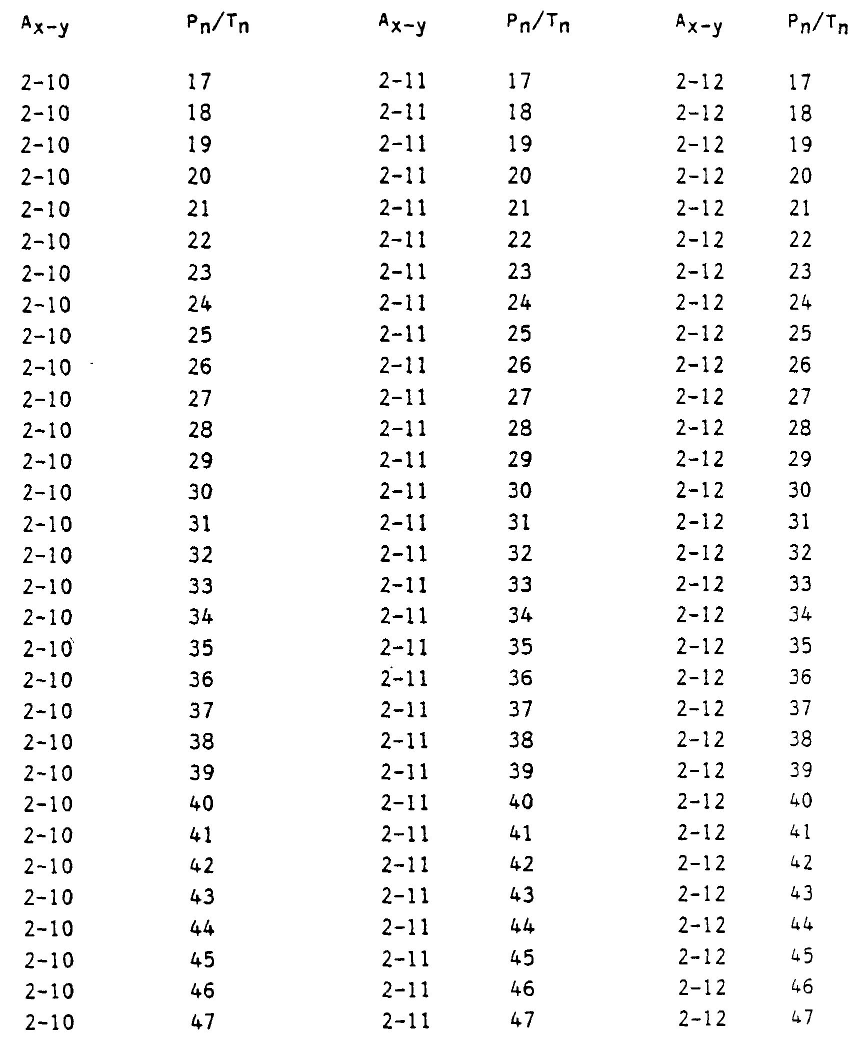

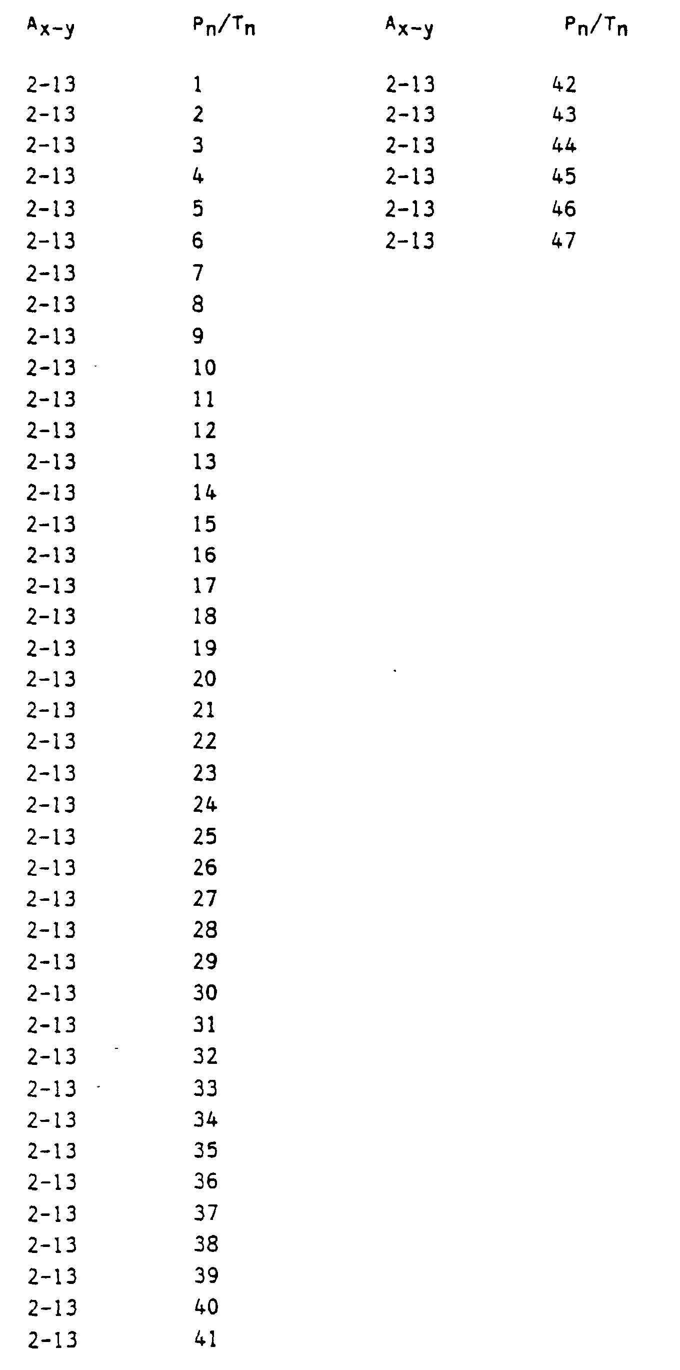

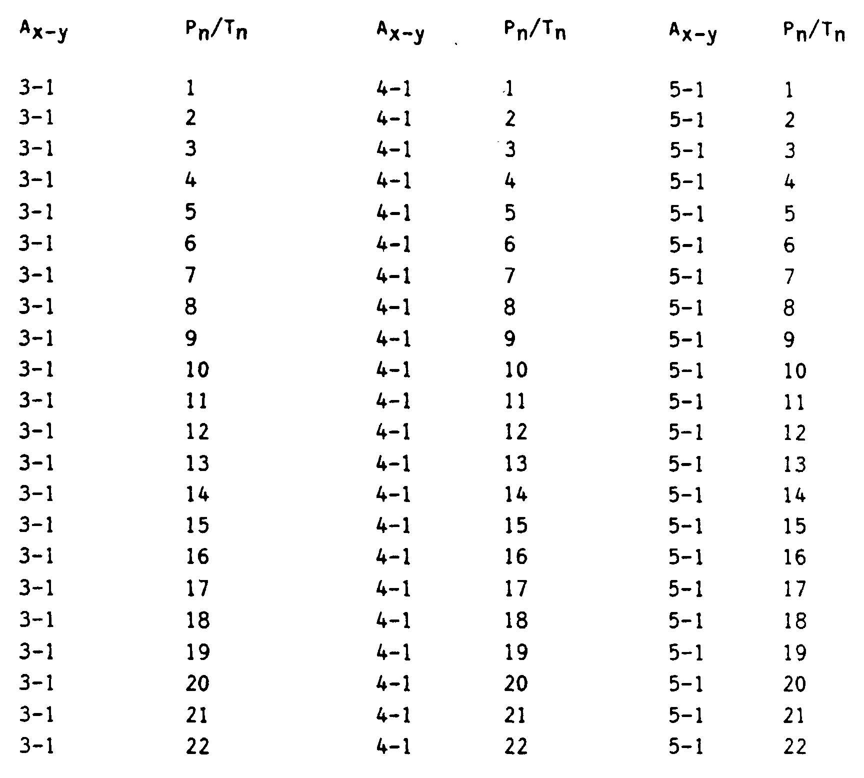

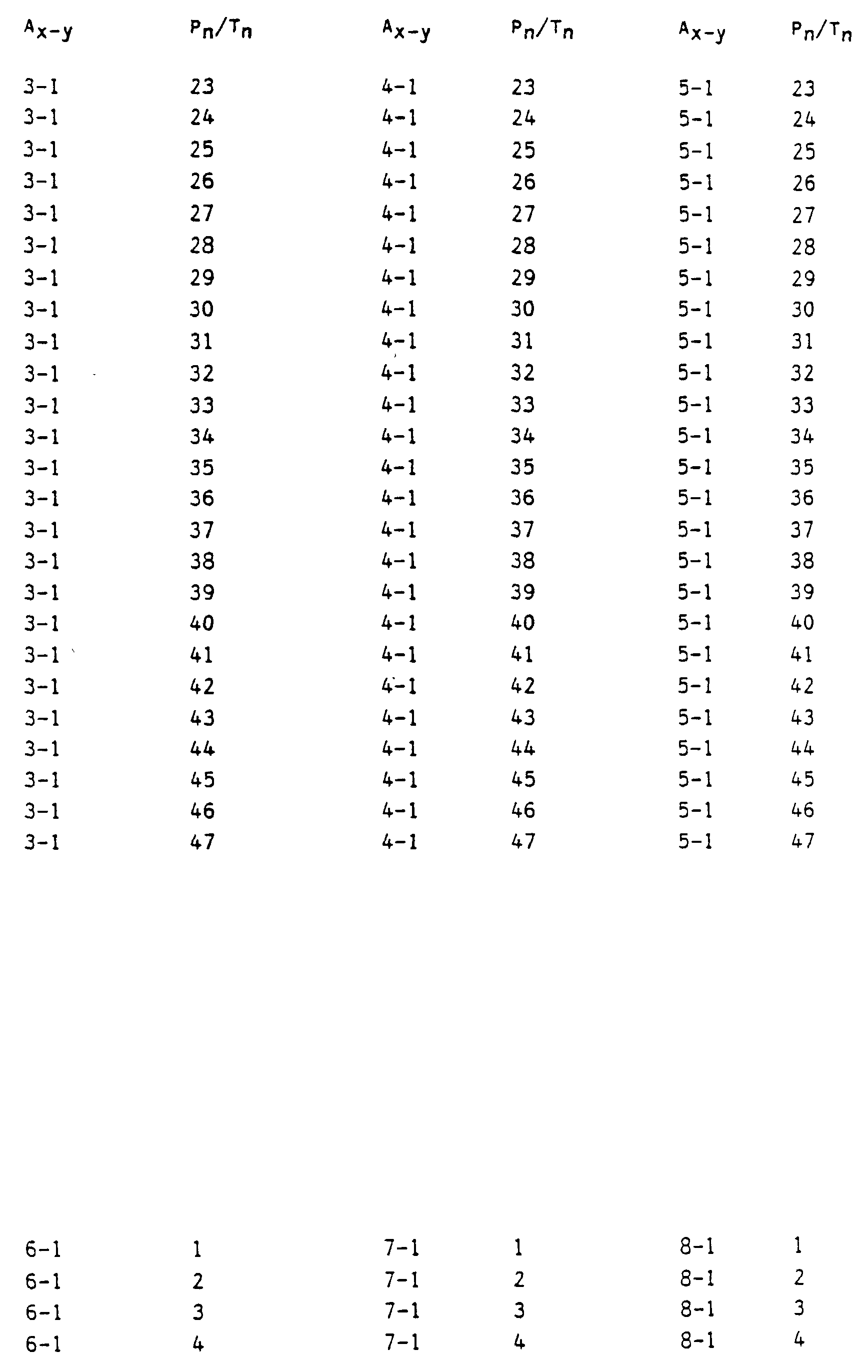

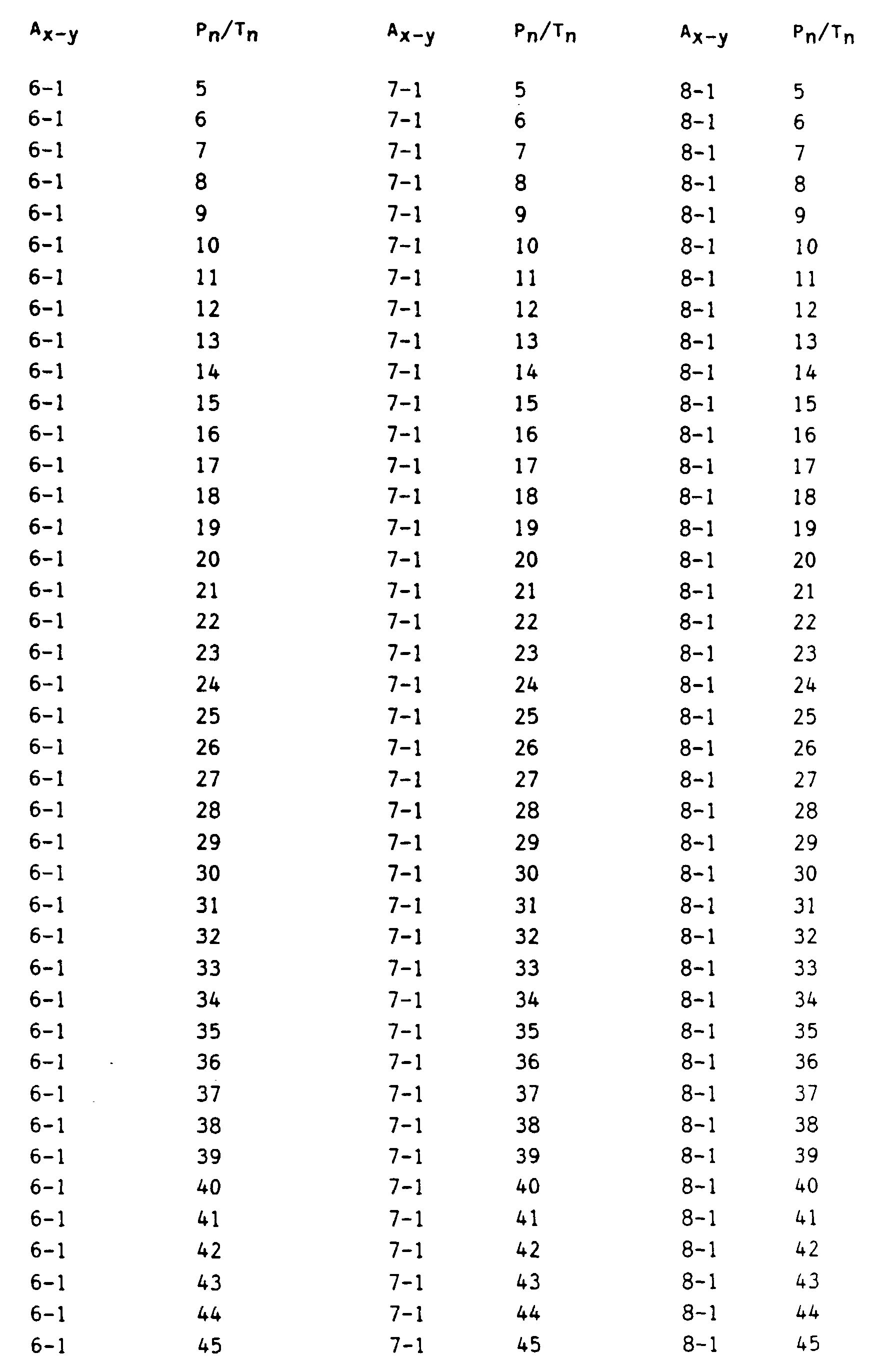

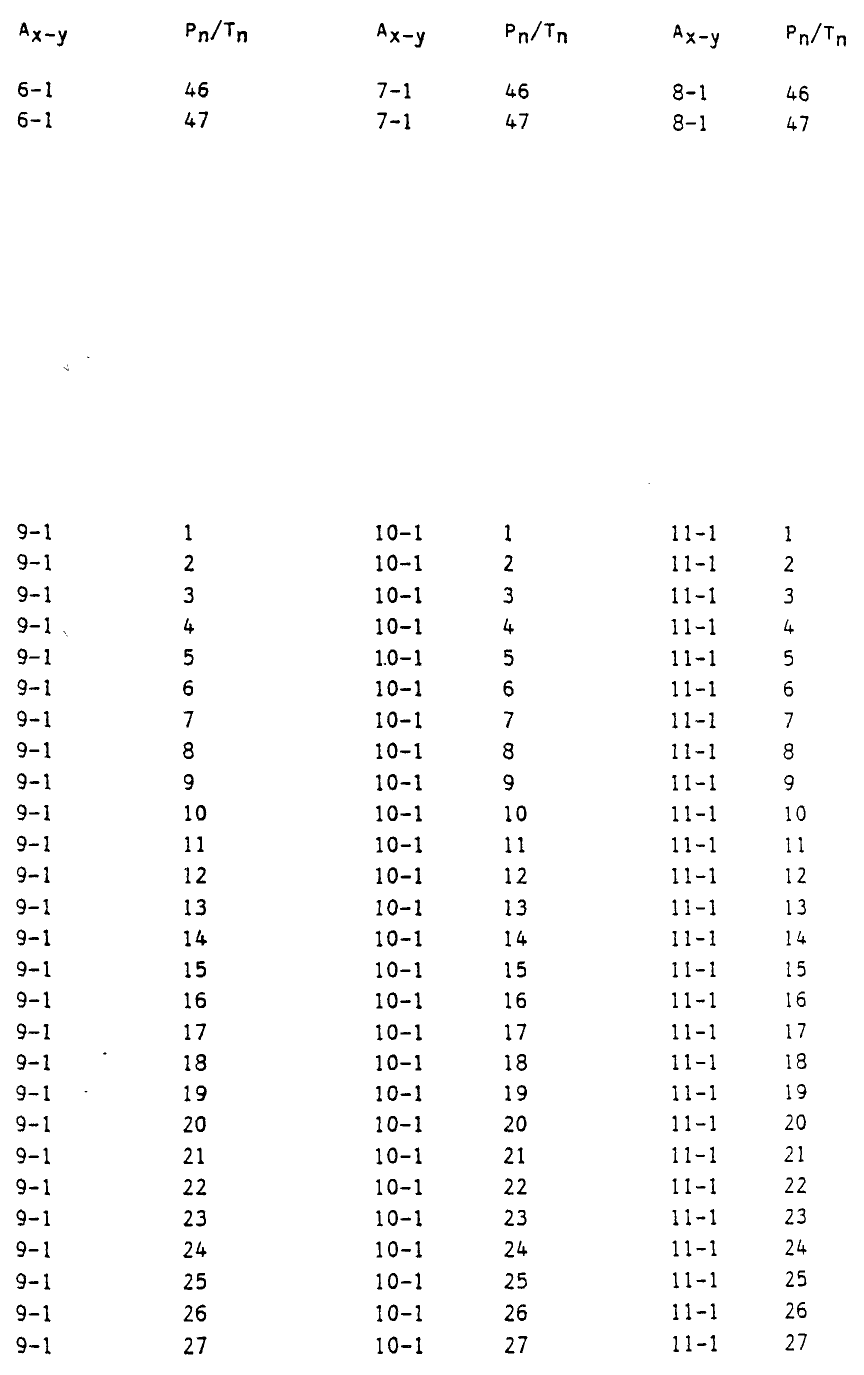

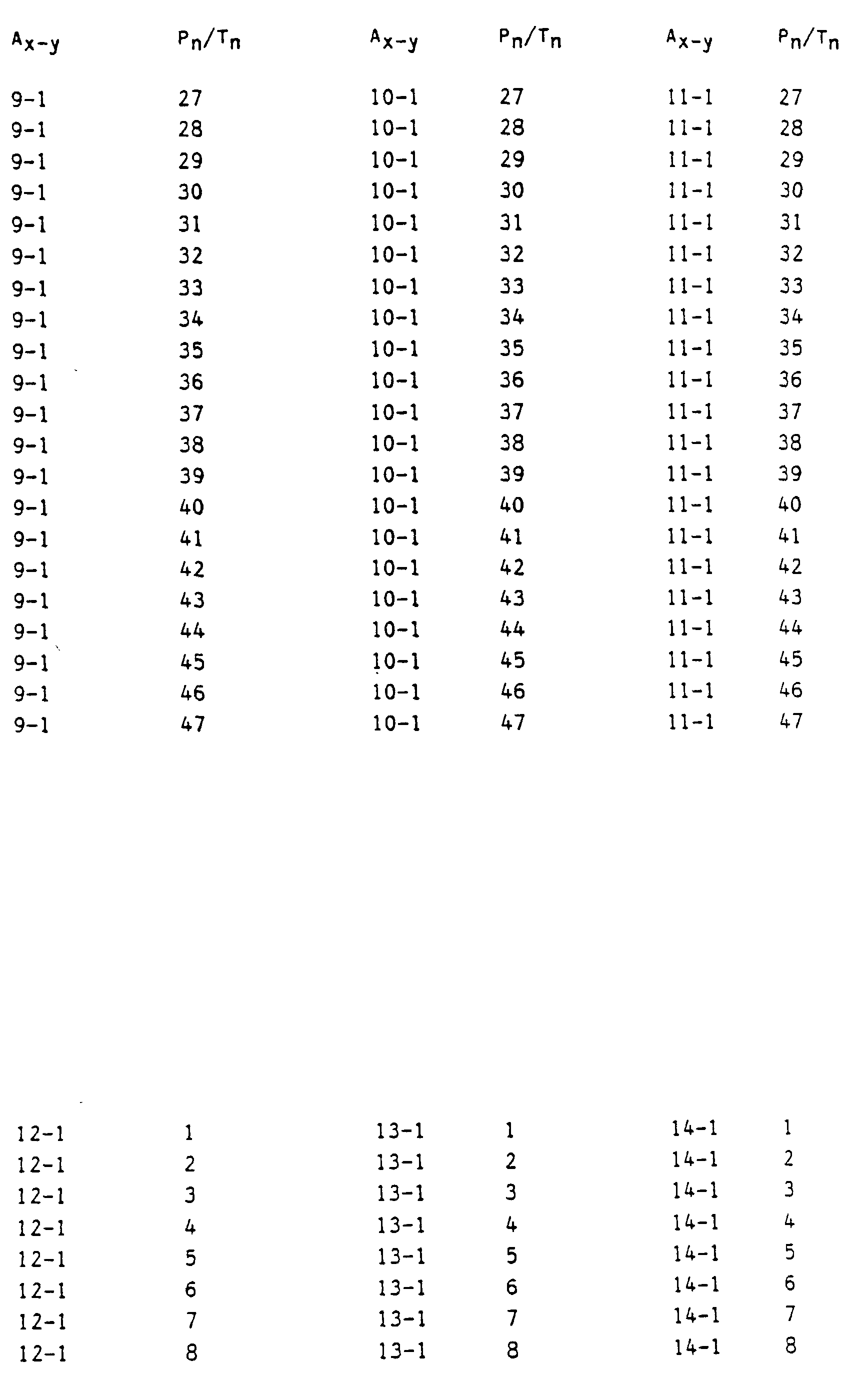

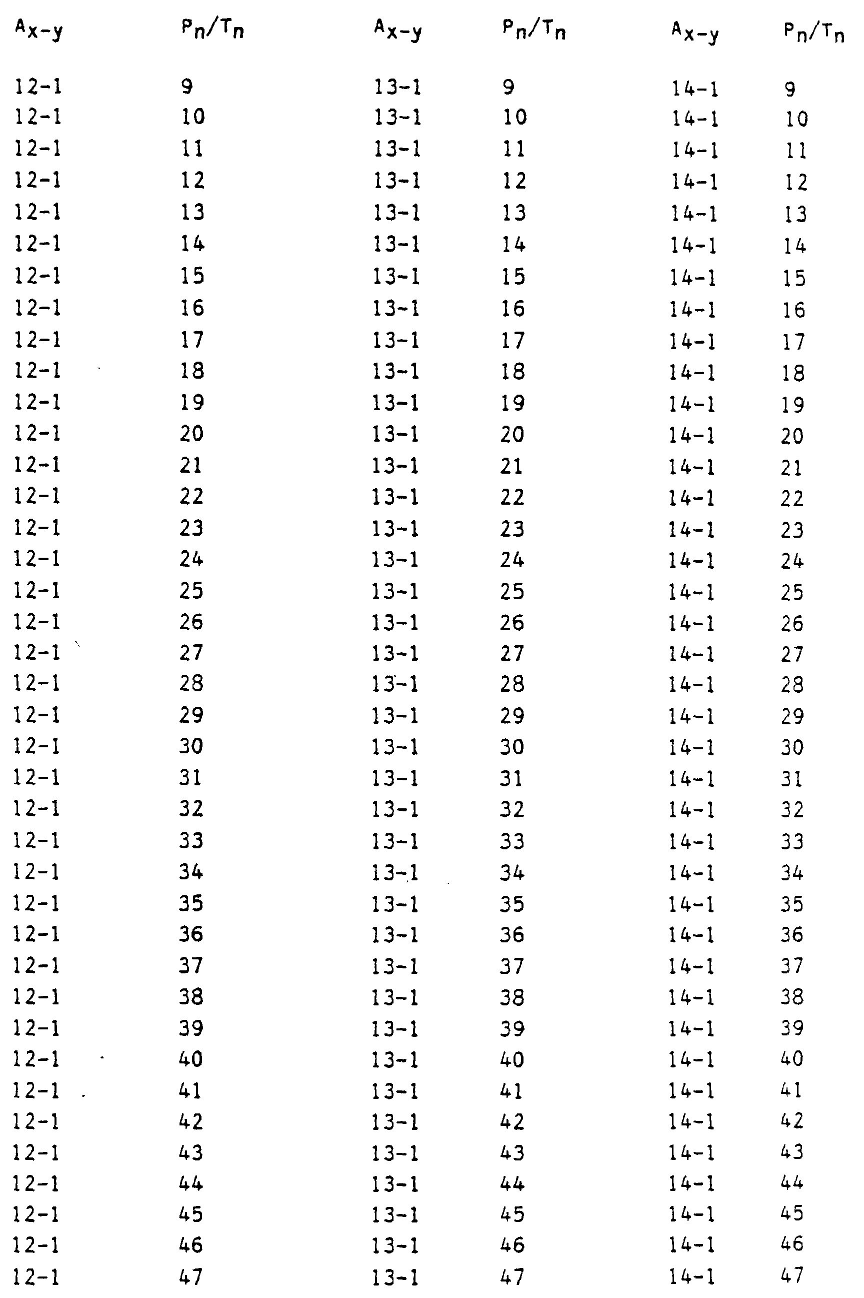

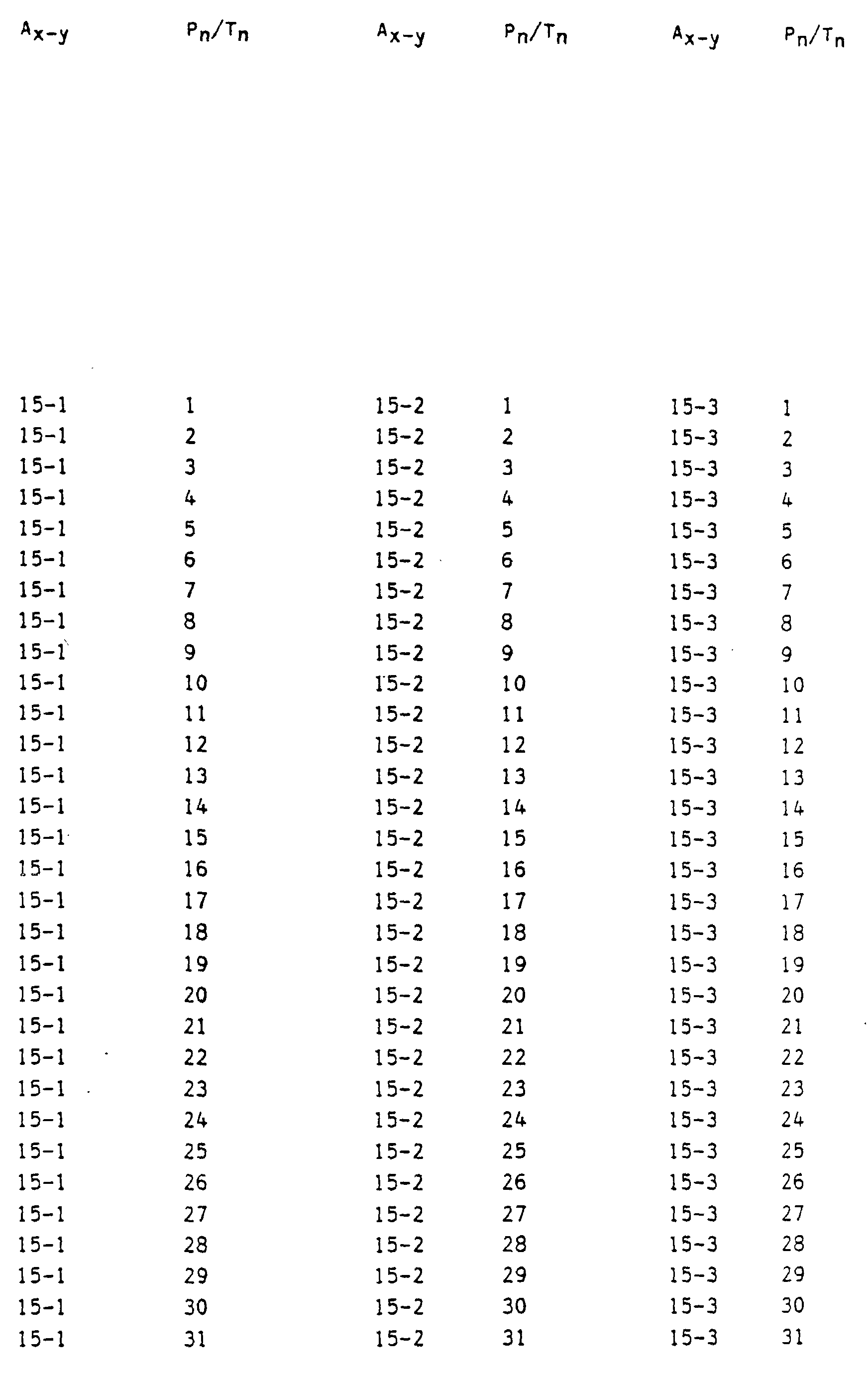

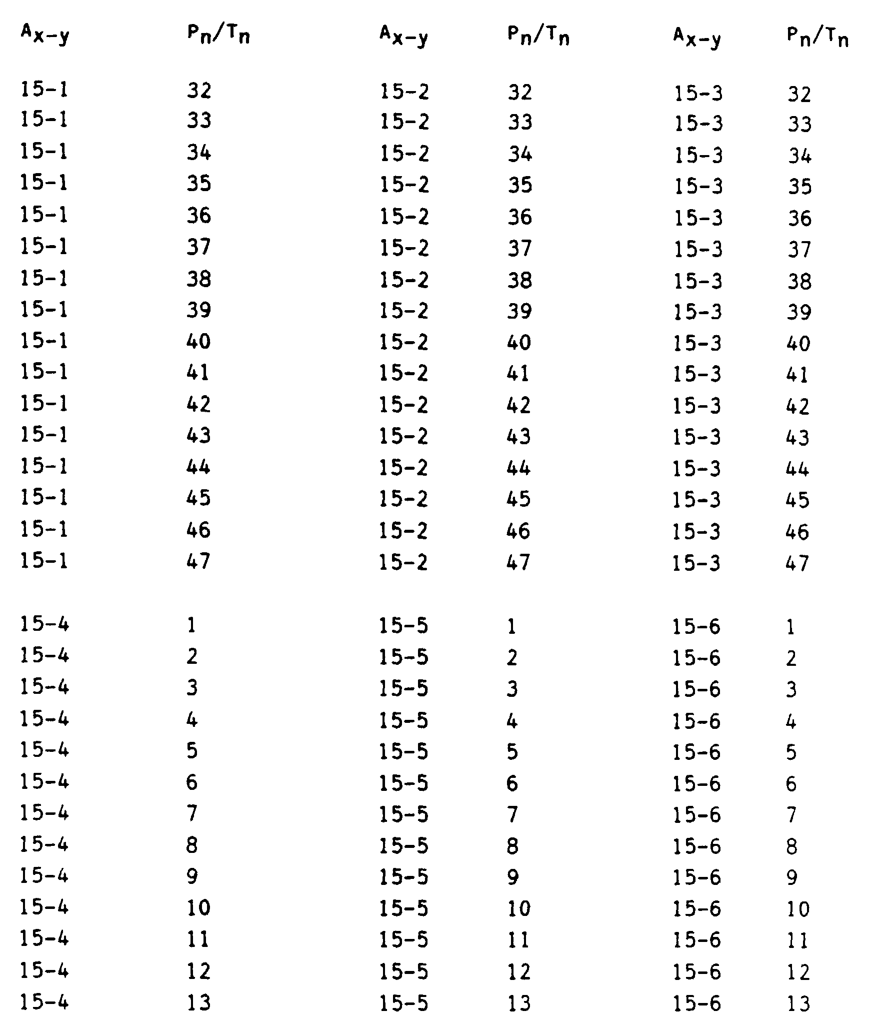

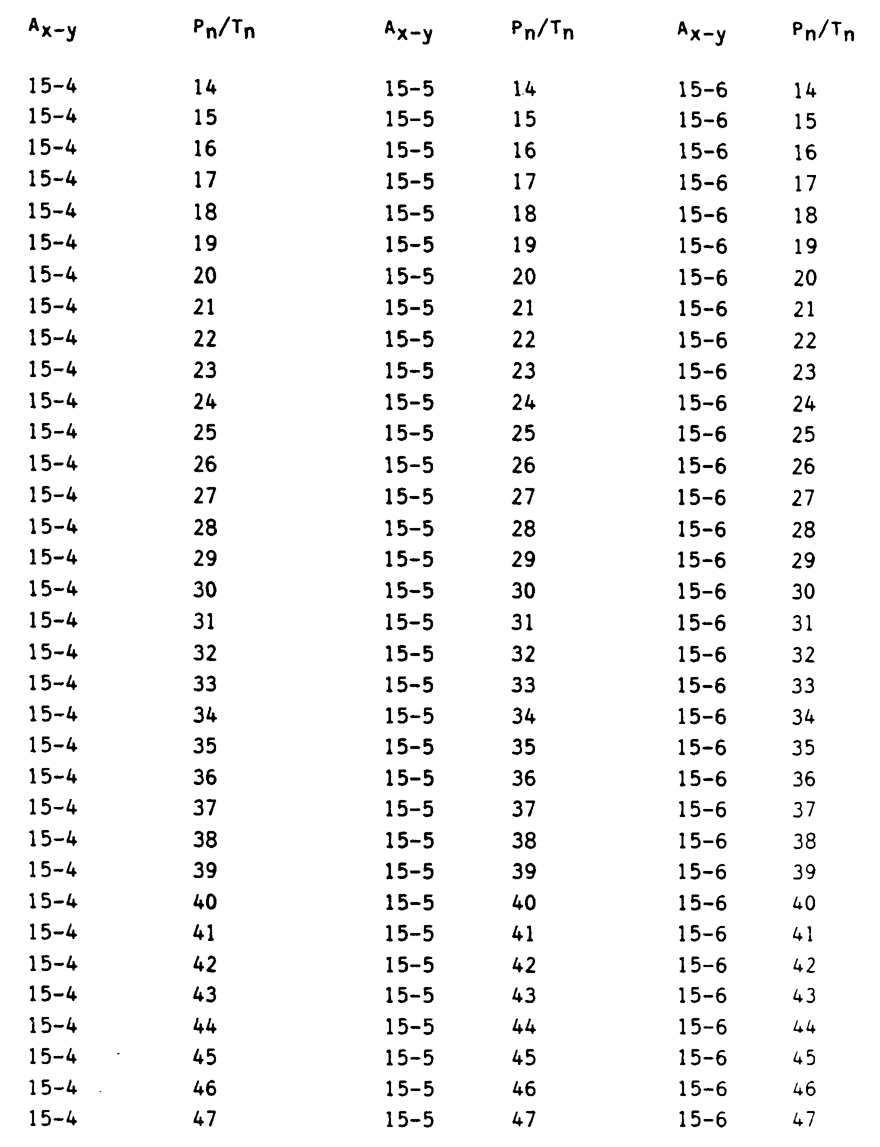

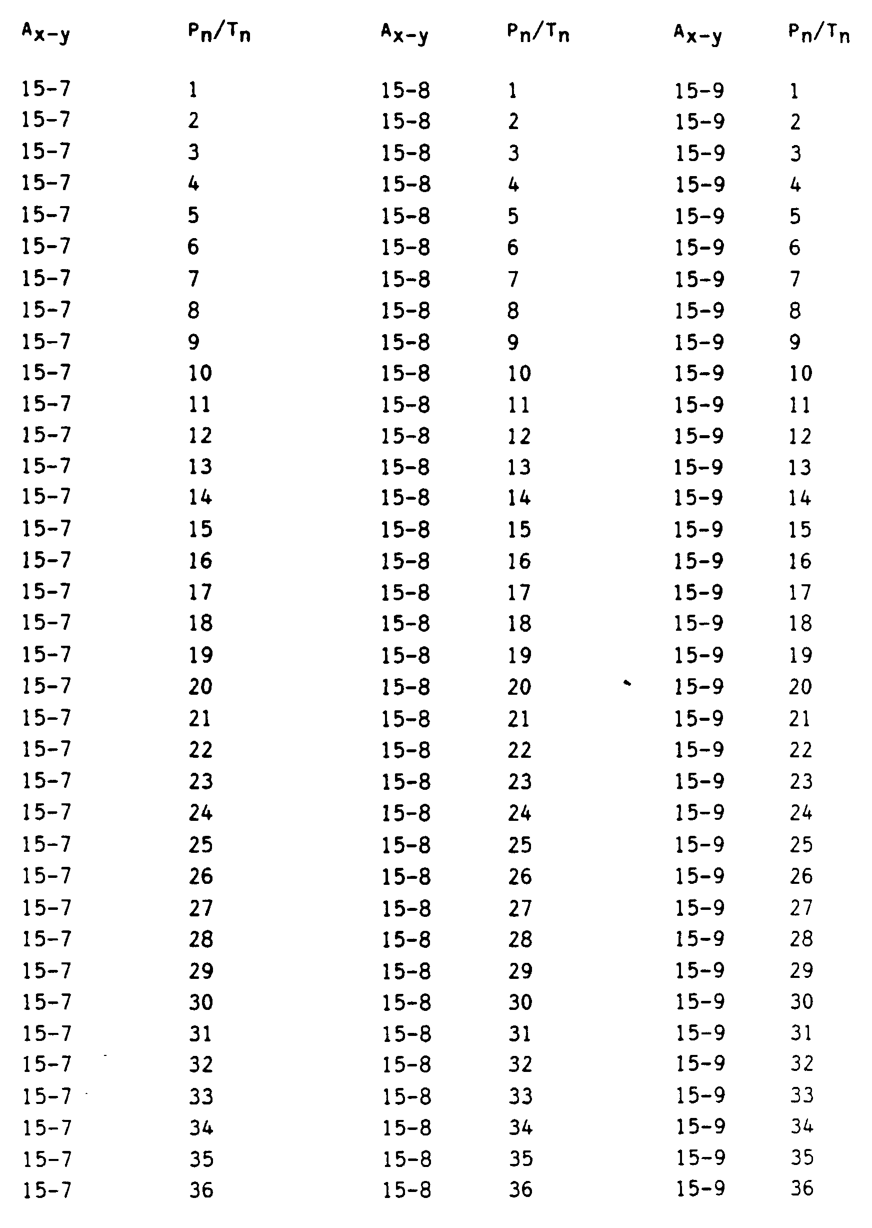

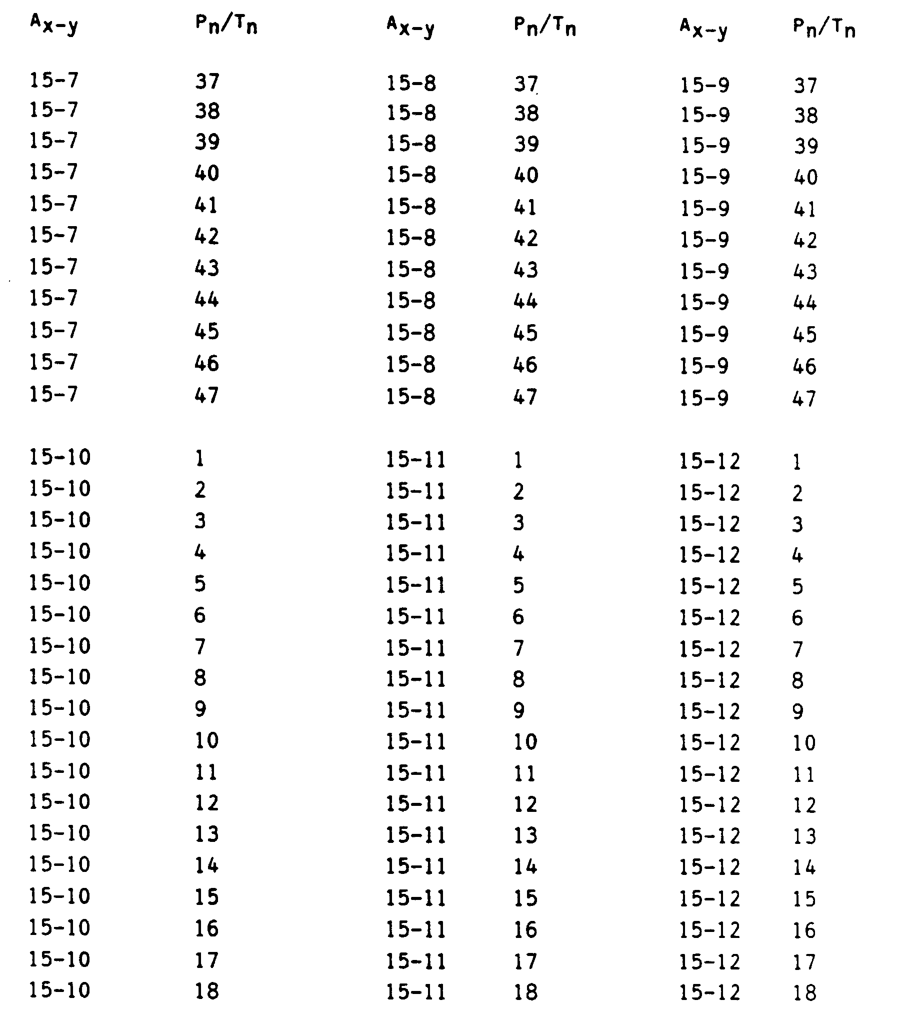

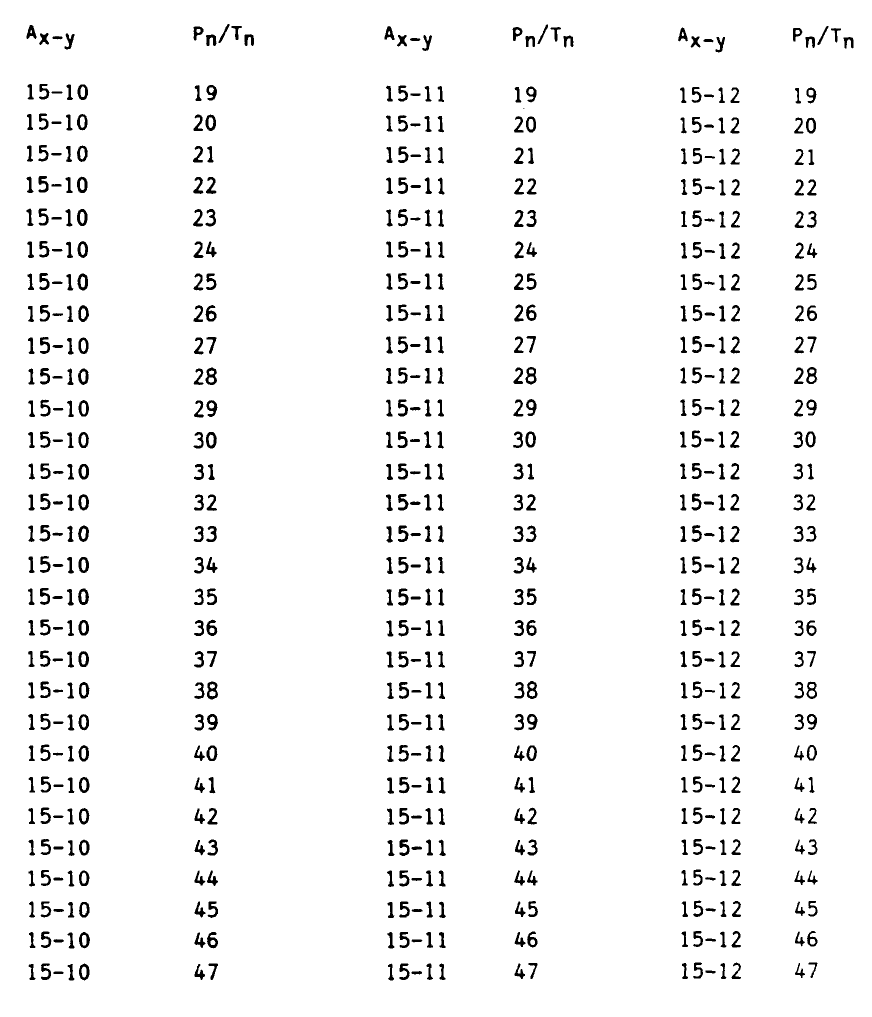

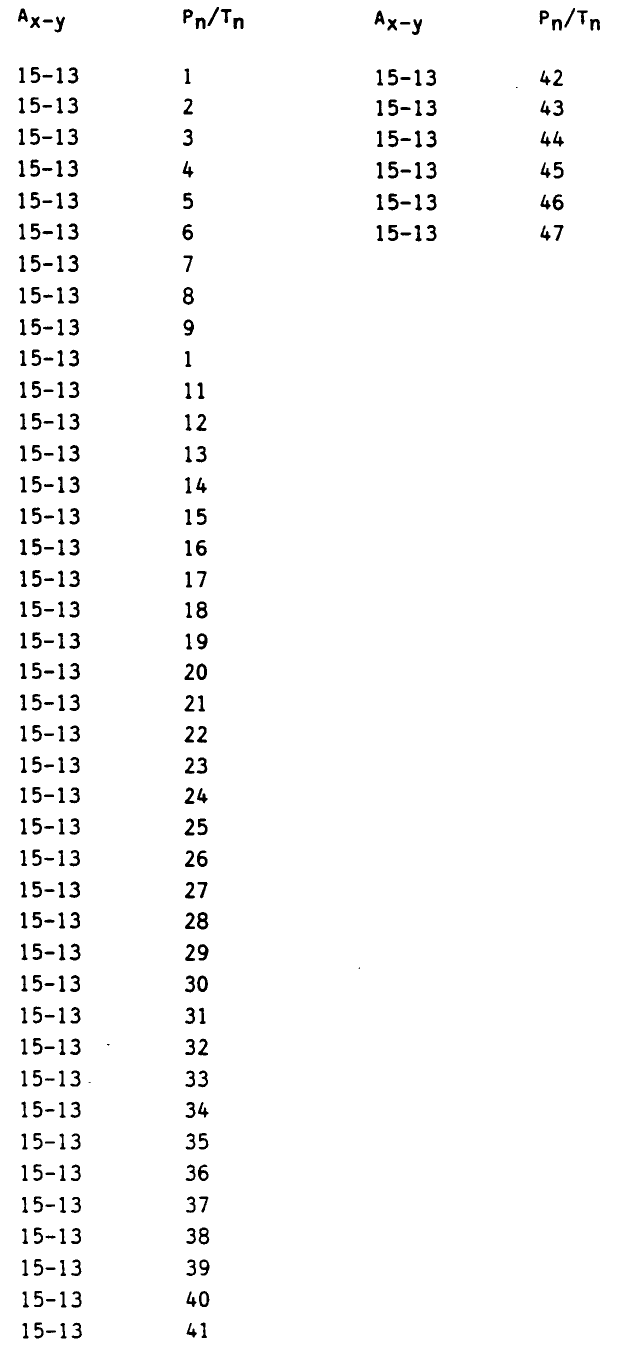

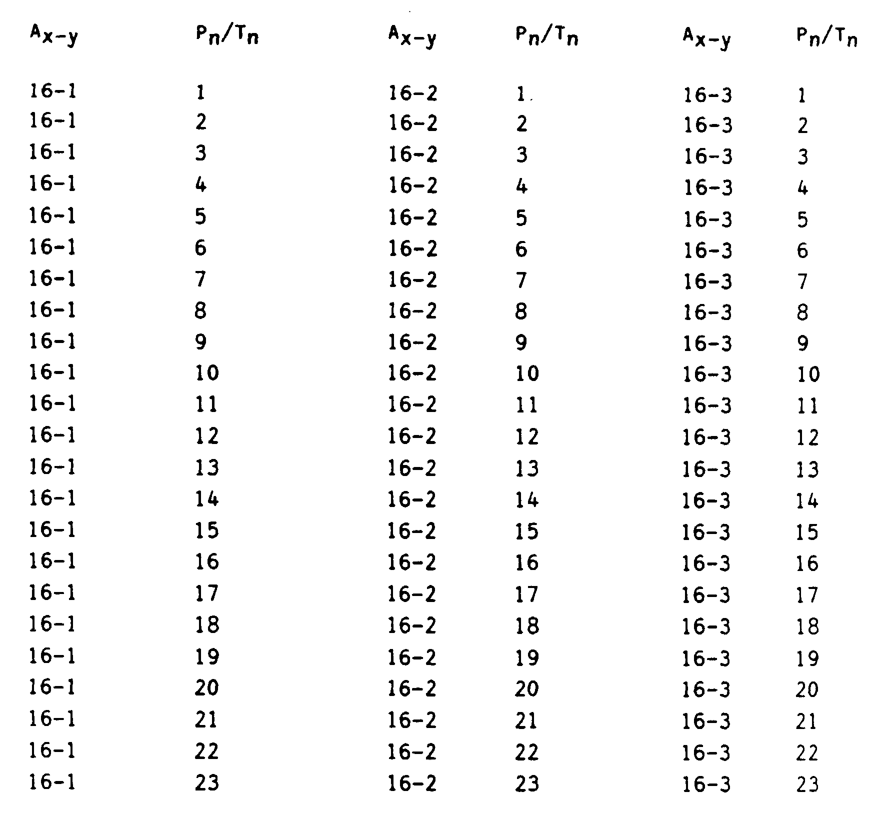

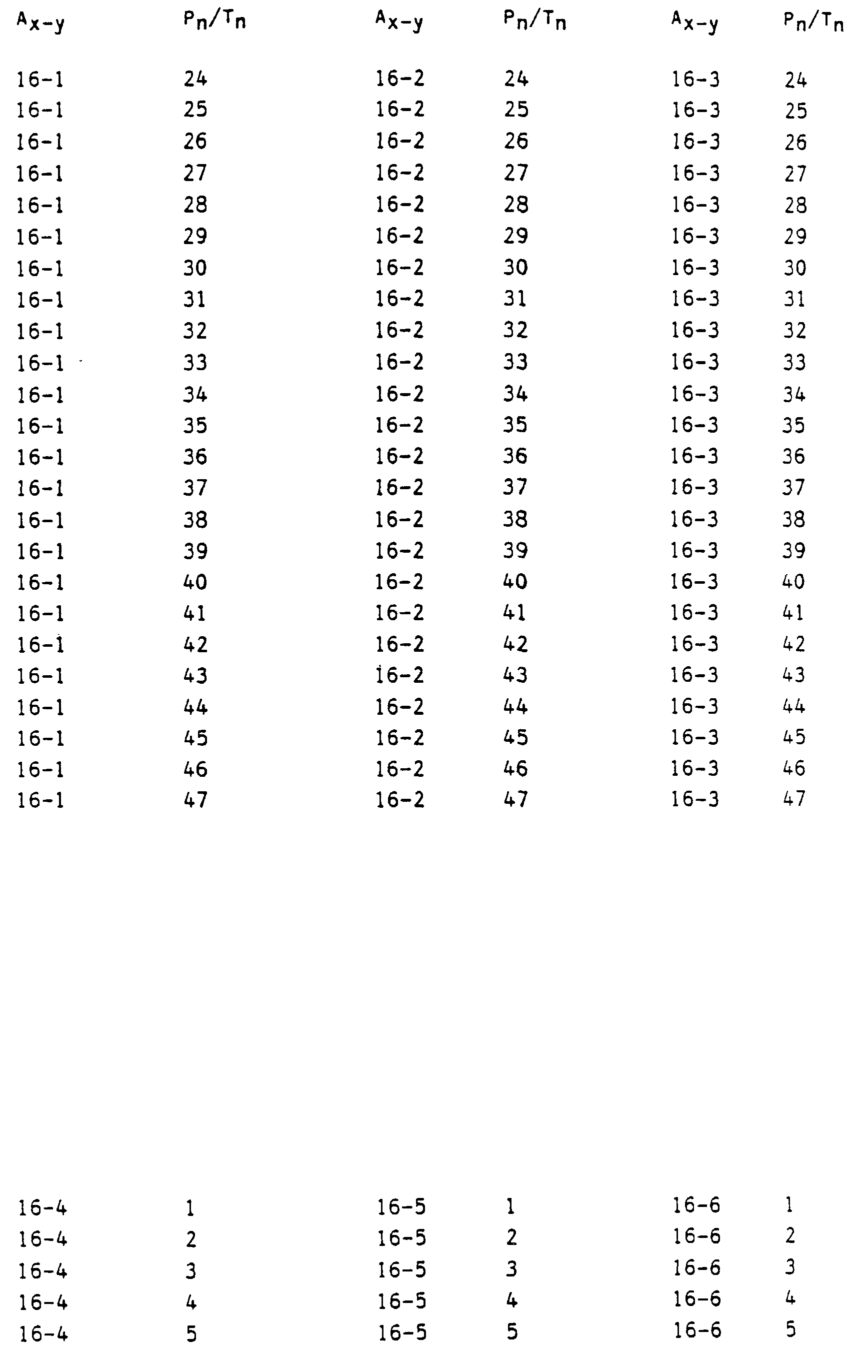

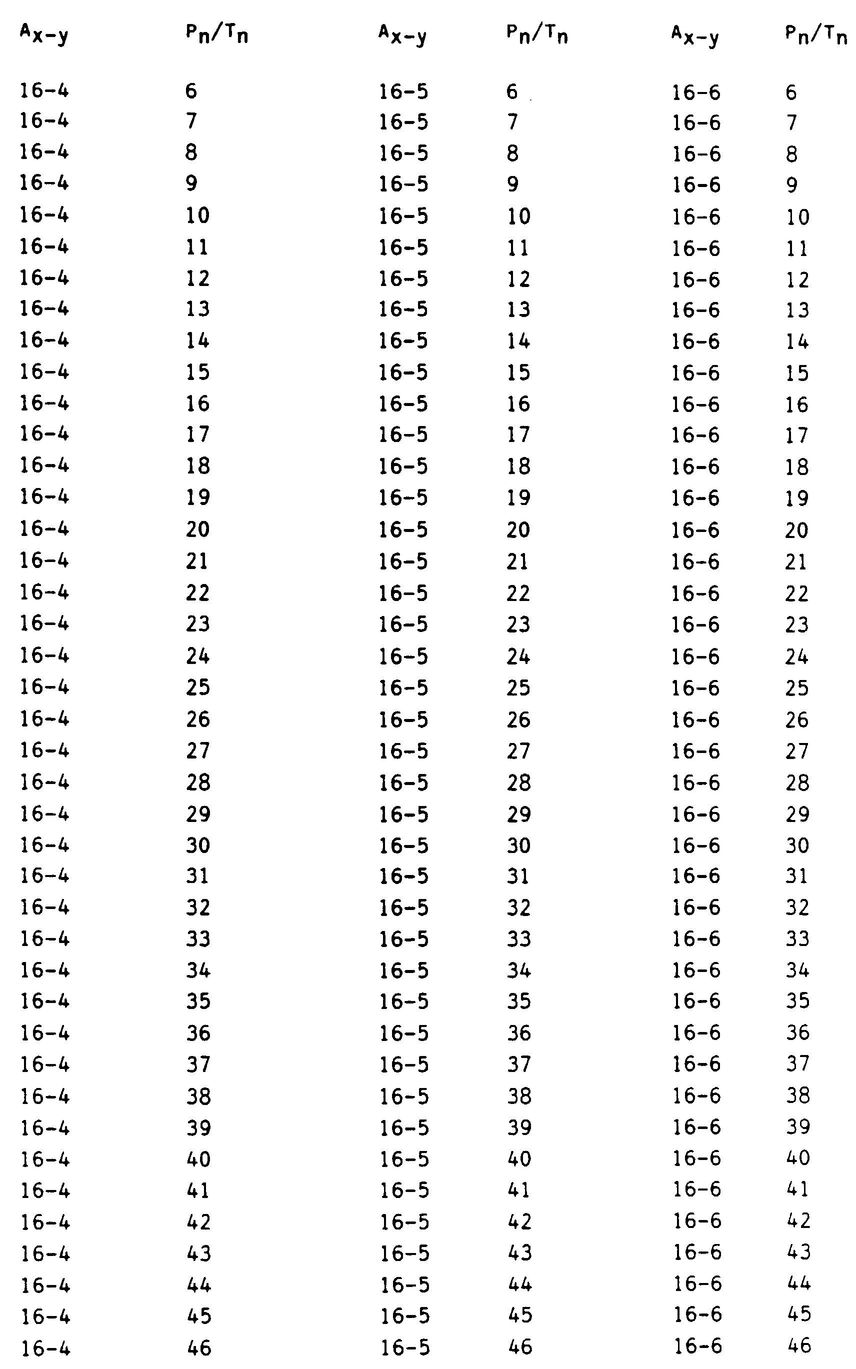

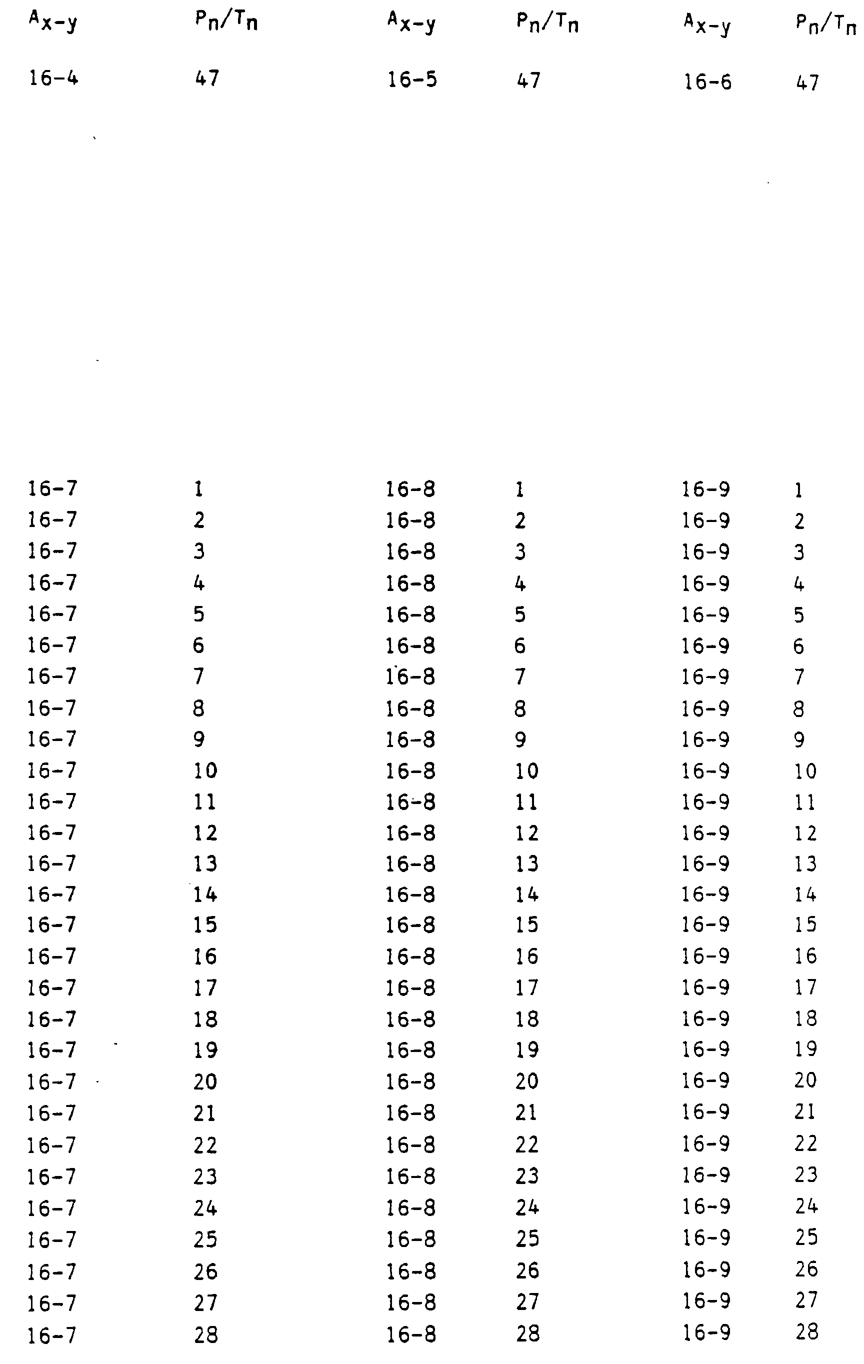

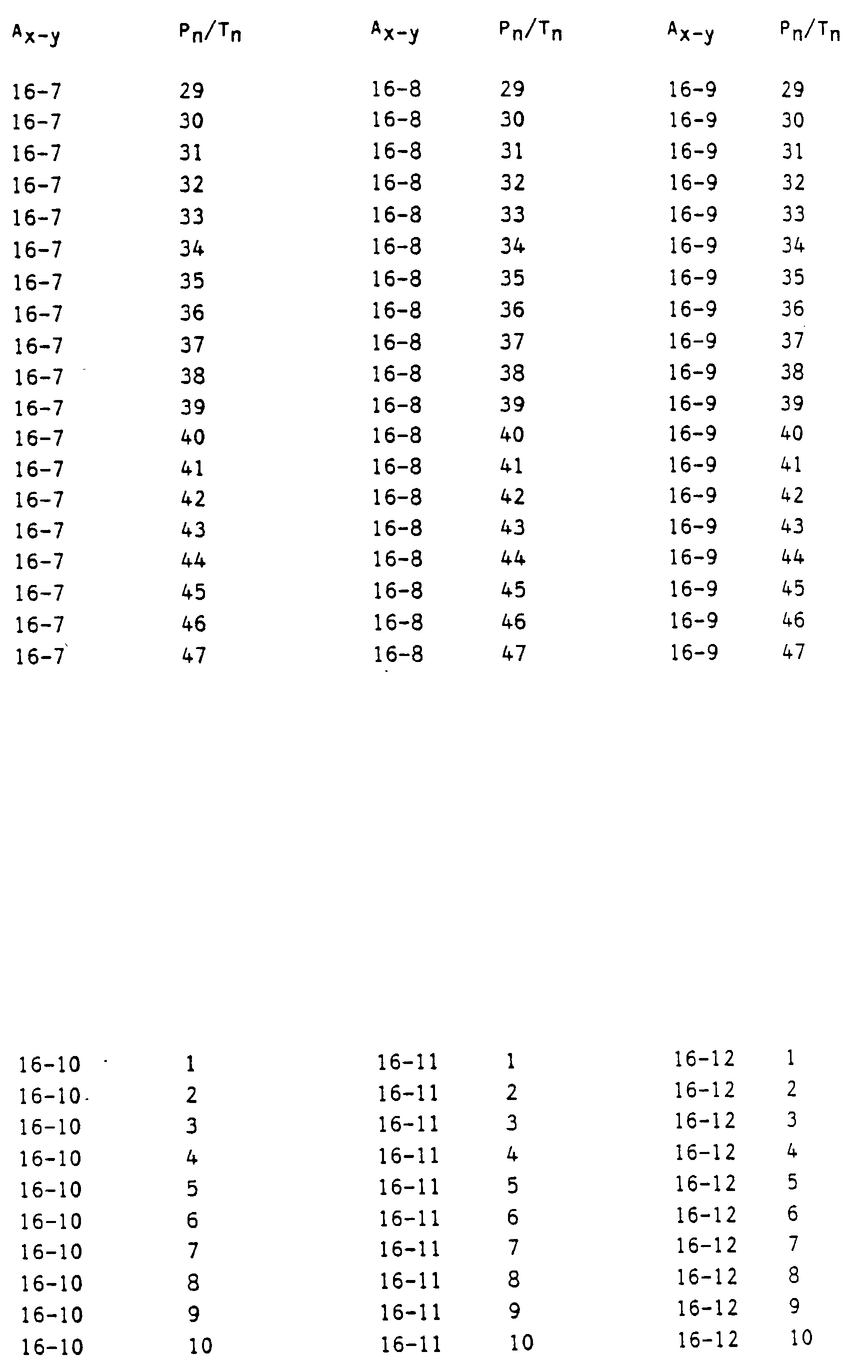

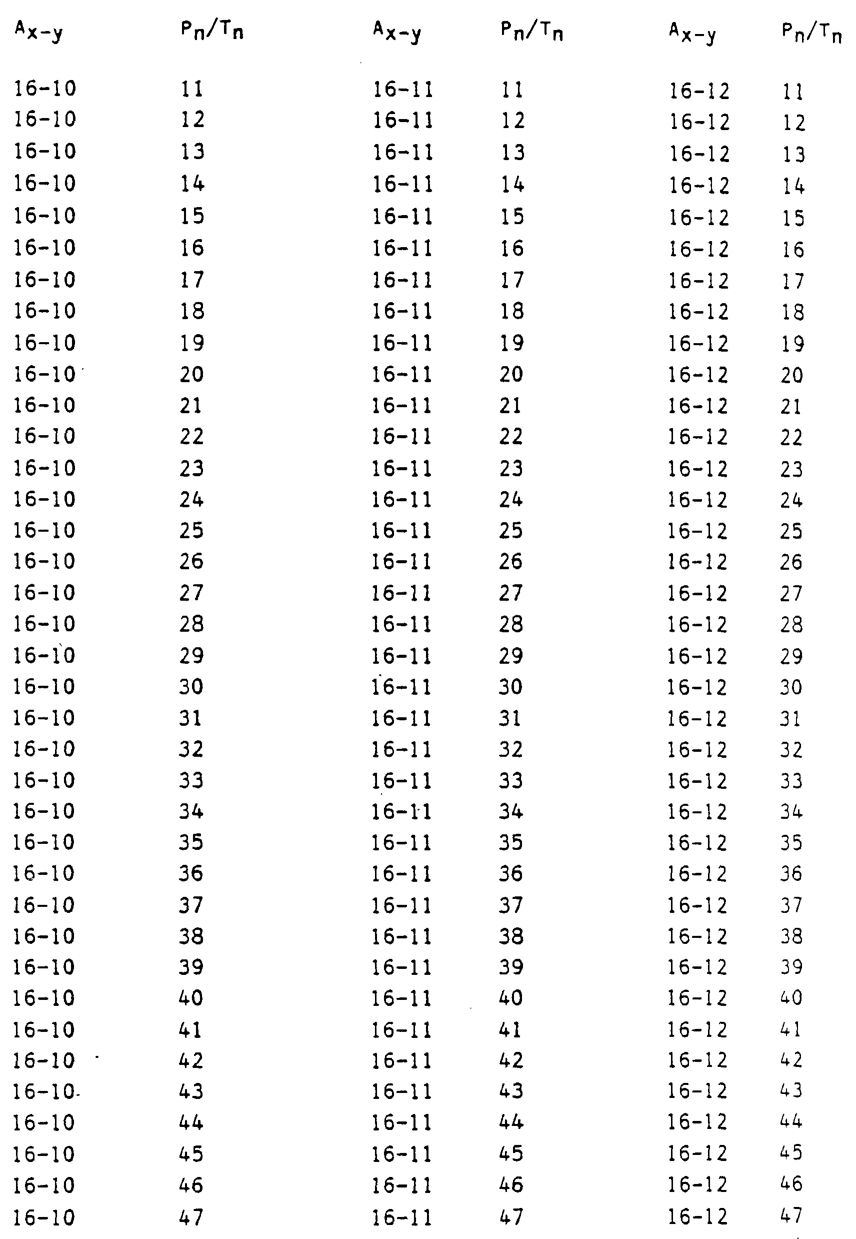

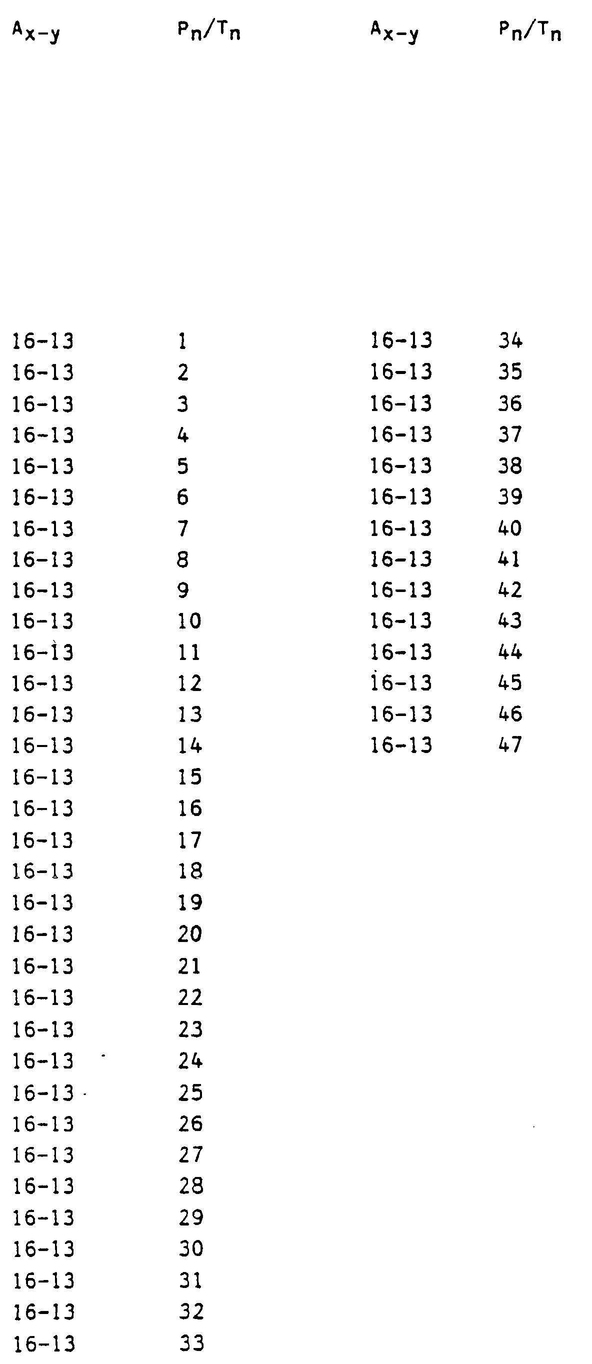

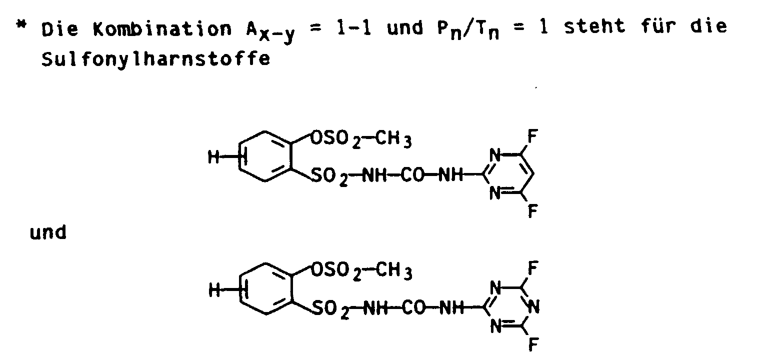

In entsprechender Weise sind die übrigen Zahlenkombinationen einfach den zugehörigen Sulfonylharnstoffderivaten zuzuordnen.

### Anwendungsbeispiele

Die herbizide Wirkung der Sulfonylharnstoffderivate der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäßte verpflanzt. Die Aufwandmenge für die Nachauflaufbehandlung betrug 0,015 kg/ha a.S. (aktive Substanz) bzw. 0,5 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstums verlauf.

Die in den Gewächshausversuchen verwendeten Pflanzen setzten sich aus folgenden Arten zusammen:

| Abkürz. | Lateinischer Name | Deutscher Name |
|---|---|---|
| CHYCO | Chrysanthemum | Kronenwucherblume |
| SINAL | Sinapis alba | Weißer Senf |
| SOLNI | Solanum nigrum | Schwarzer Nachtschatten |
| STEME | Stellaria media | Vogelmiere |
| XANPE | Xanthium pennsylvanicum | Amerikanische Spitzklette |
| | | |
| TRZAW | Triticum aestivum | Winterweizen |
| ZEAMX | Zea mays | Mais |

Mit 0,015 kg/ha a.S. im Nachauflaufverfahren eingesetzt, lassen sich mit den Beispielen 1 und 5 breitblättrige unerwünschte Pflanzen sehr gut bekämpfen bei gleichzeitiger Verträglichkeit an den Beispielkulturen Weizen und Mais.

Die Beispielverbindung 13 zeigt bei einer Aufwandmenge von 0,5 kg/ha im Nachauflaufverfahren sehr gute herbizide Wikrung gegen die unerwünschten Pflanzen Amaranthus retroflexus, Galium aparine und Ceantaurea cyanus.

In den nachfolgenden Vergleichsbeispielen wurden dem Beispiel Nr. 1 die aus EP-A 44 212 bekannte Verbindung A und die unter den dort angegebenen allgemeinen Anspruch fallende Verbindung B gegenübergestellt.

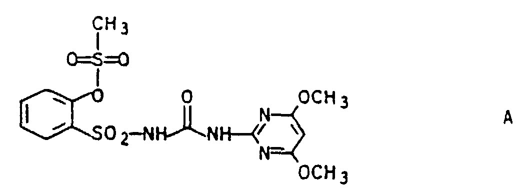

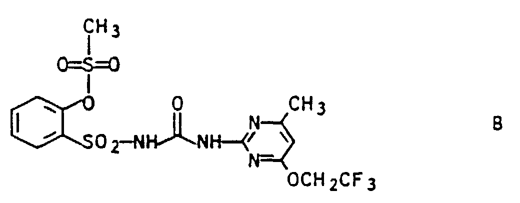

Die Versuchsergebnisse, die in den Tabellen 3 und 4 zusammengefaßt sind, zeigen deutlich die überraschend hohe Selektivität der beanspruchten Verbindung gegenüber den Vergleichssubstanzen bei gleichzeitig sehr guter herbizider Wirksamkeit.

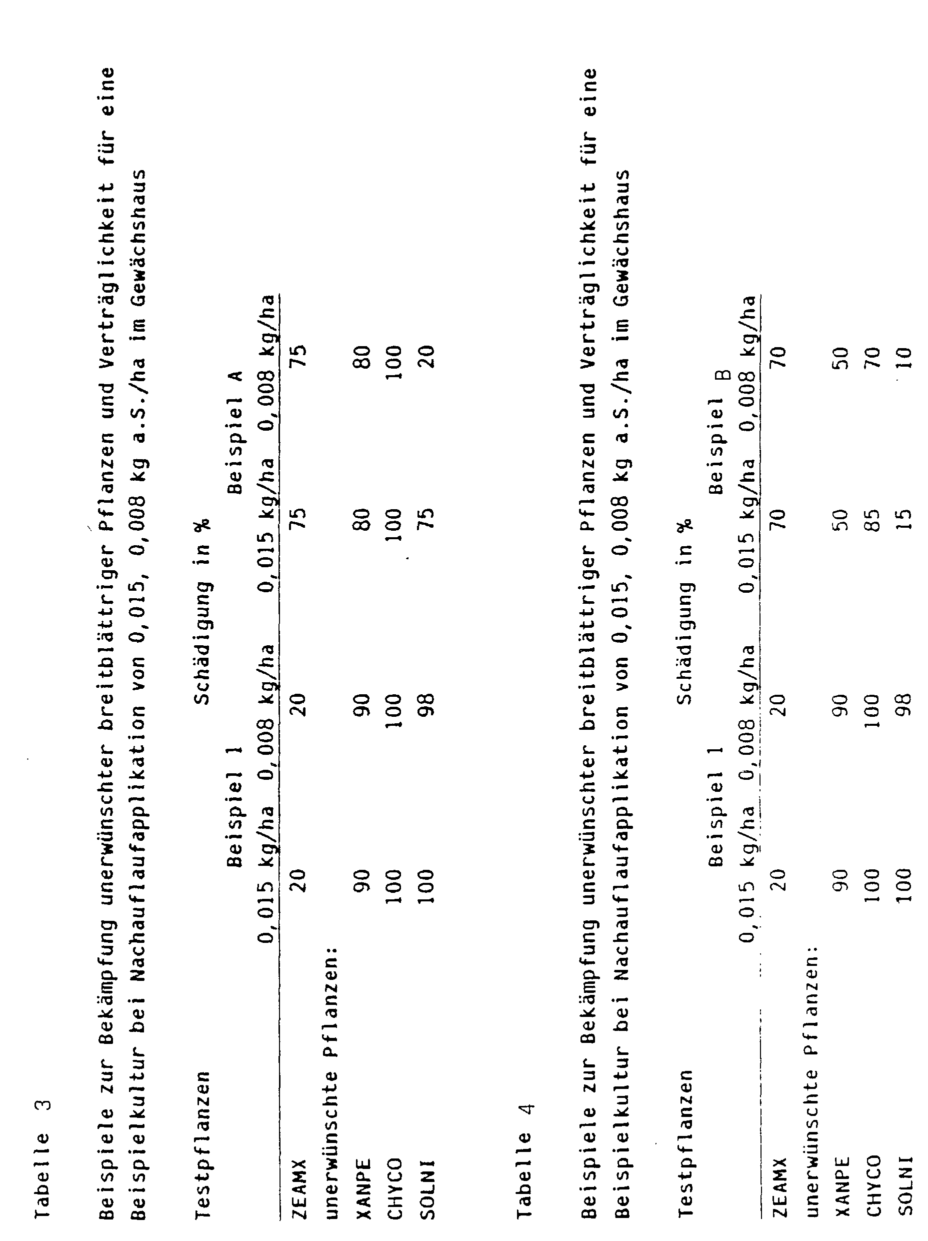

## Patentansprüche

1. Substituierte Sulfonylharnstoffderivate der allgemeinen Formel I, 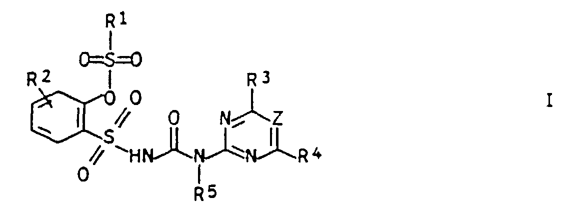 in der
R¹ eine C₁-C₄-Alkylgruppe, die bis zu drei der folgenden Reste tragen kann: Halogen oder eine C₁-C₂-Alkoxygruppe; eine C₂-C₃-Alkenylgruppe; die Propargylgruppe; eine C₁-C₃-Alkylaminogruppe oder eine Di(C₁-C₄-alkyl)aminogruppe; einen Phenylrest, der bis zu drei der folgenden Reste tragen darf: Halogen, eine C₁-C₄-Alkylgruppe oder eine C₁-C₂-Alkoxygruppe,
R² Wasserstoff, Halogen, die Methyl-, Methoxy- oder Ethoxygruppe, die jeweils 1 bis 3 Halogenatome tragen können, eine C₁-C₂-Alkylsulfonylgruppe, die Nitrogruppe oder die Cyanogruppe;
R³ die e Trifluormethoxygruppe,
die Bromdifluormethoxygruppe, die Chlordifluormethoxygruppe oder Fluor;
R⁴ Halogen, die Methyl- oder Ethylgruppe, eine C₁-C₂-Haloalkylgruppe, eine C₁-C₂-Haloalkoxygruppe, die Methoxy- oder Ethoxygruppe oder die Methyl- oder Dimethylaminogruppe;
R⁵ Wasserstoff, eine C₁-C₃-Alkylgruppe, eine C₂-C₃-Alkenylgruppe oder eine C₃-C₄-Alkinylgruppe;
Z CH oder N
bedeuten, mit der Maßgabe, daß, wenn R³ ein Fluoratom bedeutet und Z für N steht, R⁴ keine Alkylaminogruppe bedeutet,
sowie deren landwirtschaftlich brauchbare Salze.

2. Substituierte Sulfonylharnstoffderivate der Formel I gemäß Anspruch 1, in der R¹ bis R⁴ und Z die in Anspruch 1 genannte Bedeutung besitzen und R⁵ für Wasserstoff oder die Methylgruppe steht.

3. Substituierte Sulfonylharnstoffderivate der Formel I gemäß Anspruch 1, in der R¹ eine C₁-C₄-Alkylgruppe, die ein- bis dreifach durch Halogen oder eine C₁-C₂-Alkoxygruppe substituiert sein kann; die Propargylgruppe oder eine C₂-C₃-Alkenylgruppe bedeutet, R⁵ für Wasserstoff oder die Methylgruppe steht und R² bis R⁴ und Z die in Anspruch 1 genannte Bedeutung haben.

4. Substituierte Sulfonylharnstoffderivate der Formel I gemäß Anspruch 1, in der R¹ eine C₁-C₃-Alkylamino- oder Di(C₁-C₄)alkylaminogruppe bedeutet, R⁵ für Wasserstoff oder die Methylgruppe steht und R² bis R⁴ und Z die in Anspruch 1 genannte Bedeutung haben.

5. Substituierte Sulfonylharnstoffderivate der Formel I gemäß Anspruch 1, in der R¹ einen Phenylrest, der ein bis drei der folgenden Reste tragen darf: Halogen, eine C₁-C₄-Alkylgruppe, Methoxy oder Ethoxy bedeutet, R⁵ für Wasserstoff oder die Methylgruppe steht und R² bis R⁴ und Z die in Anspruch 1 genannte Bedeutung haben.

6. Substituierte Sulfonylharnstoffderivate der Formel I gemäß Anspruch 1, in der R¹ eine C₁-C₄-Alkylgruppe, die ein bis drei Halogenatome tragen kann; die Propargylgruppe; eine C₂-C₃-Alkenylgruppe; die Methylamino- oder Dimethylaminogruppe bedeutet, R³ die Trifluormethoxygruppe, die Chlordifluormethoxygruppe oder Fluor bedeutet, R⁵ für Wasserstoff oder die Methylgruppe steht und R², R⁴, und Z die in Anspruch 1 genannte Bedeutung besitzen.

7. Substituierte Sulfonylharnstoffderivate der Formel I gemäß Anspruch 1, in der R¹ eine C₁-C₄-Alkylgruppe, die ein bis drei Halogenatome tragen kann; die Propargylgruppe; eine C₂-C₃-Alkenylgruppe; die Methylamino- oder Dimethylaminogruppe bedeutet, R³ die Trifluormethoxygruppe, die Chlordifluormethoxygruppe oder Fluor bedeutet, R⁴ die Methoxygruppe bedeutet, R⁵ für Wasserstoff oder die Methylgruppe steht und R² und Z die in Anspruch 1 genannte Bedeutung besitzen.

8. Verfahren zur Herstellung von Sulfonylharnstoffderivaten der Formel I gemäß Anspruch 1, dadurch gekennzeichnet, daß man ein entsprechend substituiertes Benzolsulfonylisocyanat der allgemeinen Formel II, 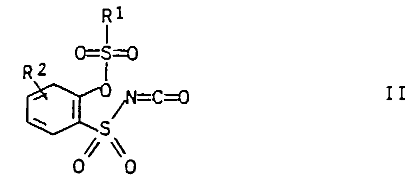 in der R¹ und R² die in Anspruch 1 genannte Bedeutung haben, mit ungefähr stöchiometrischen Mengen eines Amins der allgemeinen Formel III, 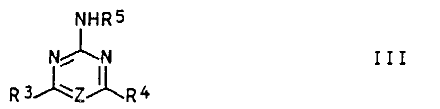 in der R³ bis R⁵ die in Anspruch 1 genannte Bedeutung haben, in an sich bekannter Weise in einem inerten organischen Lösungsmittel umsetzt.

9. Verfahren zur Herstellung von Sulfonylharnstoffderivaten der Formel I gemäß Anspruch 1, dadurch gekennzeichnet, daß man ein Carbamat der Formel IV 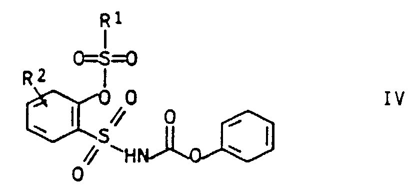 in der R¹ und R² die in Anspruch 1 genannte Bedeutung haben, mit ungefähr stöchiometrischen Mengen eines Amins der Formel III, 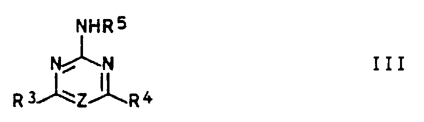 in der R³ bis R⁵ die in Anspruch 1 genannte Bedeutung haben, in an sich bekannter Weise in einem inerten organischen Lösungsmittel umsetzt.

10. Verfahren zur Herstellung von Sulfonylharnstoffderivaten der Formel I gemäß Anspruch 1, dadurch gekennzeichnet, daß man ein entsprechend substituiertes Benzolsulfonamid der allgemeinen Formel V, 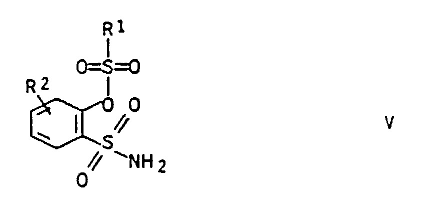 in der R¹ und R² die in Anspruch 1 genannte Bedeutung haben, mit ungefähr sthöchiometrischen Mengen eines Carbamats der allgemeinen Formel VI, 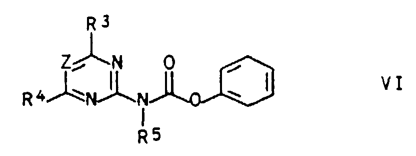 in denen R³ bis R⁵ die in Anspruch 1 genannte Bedeutung haben, in an sich bekannter Weise in einem inerten organischen Lösungsmittel umsetzt.

11. Verfahren zur Herstellung von Sulfonylharnstoffderivaten der Formel I gemäß Anspruch 1, in der der Rest R⁵ für ein Wasserstoffatom steht, dadurch gekennzeichnet, daß man ein entsprechend substituiertes Benzolsulfonamid der allgemeinen Formel V, 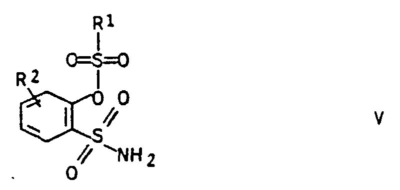 in der R¹ und R² die in Anspruch 1 genannte Bedeutung haben, mit ungefähr stöchiometrischen Mengen eines Isocyanats der allgemeinen Formel VII, 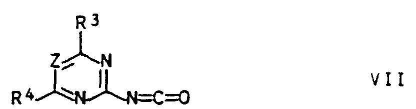 in der R³ und R⁴ die in Anspruch 1 genannte Bedeutung haben, in an sich bekannter Weise in einem inerten organischen Lösungsmittel unsetzt.

12. Herbizides Mittel, enthaltend einen Sulfonylharnstoffderivat der Formel I gemäß Anspruch 1 oder sein Salz sowie hierfür übliche Trägerstoffe.

13. Verfahren zur Bekämpfung unerwünschten Pflanzenwuches, dadurch gekennzeichnet, daß man eine herbizid wirksame Menge eines Sulfonylharnstoffderivats der Formel I gemäß Anspruch 1 oder eines seiner Salze auf die Pflanzen und/oder ihren Lebensraum einwirken läßt.

## Claims

1. A substituted sulfonylurea derivative of the formula I 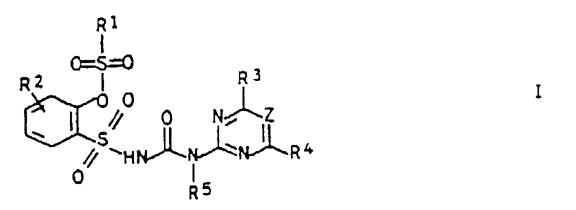 where
R¹ is C₁-C₄-alkyl which may carry up to three of the following radicals: halogen or C₁- or C₂-alkoxy; C₂- or C₃-alkenyl; propargyl; C₁-C₃-alkylamino or di-C₁-C₄-alkylamino; or phenyl which may carry up to three of the following radicals: halogen, C₁-C₄-alkyl or C₁- or C₂-alkoxy;
R² is hydrogen, halogen, methyl, methoxy or ethoxy, each of which may carry from 1 to 3 halogen atoms, or C₁- or C₂-alkylsulfonyl, nitro or cyano;
R³ is trifluoromethoxy, bromodifluoromethoxy, chlorodifluoromethoxy or fluorine;
R⁴ is halogen, methyl, ethyl, C₁- or C₂-haloalkyl, C₁- or C₂-haloalkoxy, methoxy, ethoxy, methylamino or dimethylamino;
R⁵ is hydrogen, C₁-C₃-alkyl, C₂- or C₃-alkenyl or C3- or C₄-alkynyl; and
Z is CH or N,
with the proviso that when R³ is fluorine and Z is N, R⁴ is not alkylamino, or an agriculturally useful salt thereof.

2. A substituted sulfonylurea derivative of the formula I as claimed in claim 1, where R¹ to R⁴ and Z have the meanings stated in claim 1 and R⁵ is hydrogen or methyl.

3. A substituted sulfonylurea derivative of the formula I as claimed in claim 1, where R¹ is C₁-C₄-alkyl which may be monosubstituted to trisubstituted by halogen or C₁- or C₂-alkoxy or R¹ is propargyl or C₂- or C₃-alkenyl, R⁵ is hydrogen or methyl and R² to R⁴ and Z have the meanings stated in claim 1.

4. A substituted sulfonylurea derivative of the formula I as claimed in claim 1, where R¹ is C₁-C₃-alkylamino or di-C₁-C₄-alkylamino, R⁵ is hydrogen or methyl and R² to R⁴ and Z have the meanings stated in claim 1.

5. A substituted sulfonylurea derivative of the formula I as claimed in claim 1, where R¹ is phenyl which may carry from 1 to 3 of the following radicals: halogen, C₁-C₄-alkyl, methoxy or ethoxy, R⁵ is hydrogen or methyl and R² to R⁴ and Z have the meanings stated in claim 1.

6. A substituted sulfonylurea derivative of the formula I as claimed in claim 1, where R¹ is C₁-C₄-alkyl which may carry from 1 to 3 halogen atoms or R¹ is propargyl, C₂- or C₃-alkenyl, methylamino or dimethylamino, R³ is trifluoromethoxy, chlorodifluoromethoxy or fluorine, R⁵ is hydrogen or methyl and R², R⁴ and Z have the meanings stated in claim 1.

7. A substituted sulfonylurea derivative of the formula I as claimed in claim 1, where R¹ is C₁-C₄-alkyl which may carry from 1 to 3 halogen atoms or R¹ is propargyl, C₂- or C₃-alkenyl, methylamino or dimethylamino, R³ is trifluoromethoxy, chlorodifluoromethoxy or fluorine, R⁴ is methoxy, R⁵ is hydrogen or methyl and R² and Z have the meanings stated in claim 1.

8. A process for the preparation of a sulfonylurea derivative of the formula I as claimed in claim 1, wherein a correspondingly substituted benzenesulfonyl isocyanate of the formula II 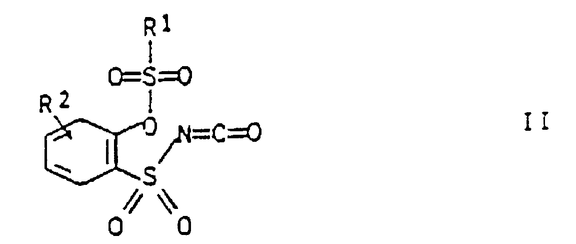 where R¹ and R² have the meanings stated in claim 1, is reacted with about the stoichiometric amount of an amine of the formula III 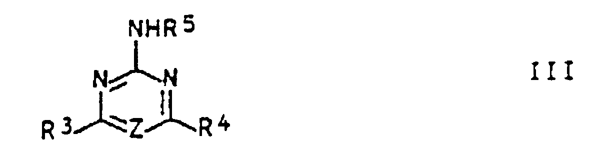 where R³ to R⁵ have the meanings stated in claim 1, in a conventional manner, in an inert organic solvent.

9. A process for the preparation of a sulfonylurea derivative of the formula I as claimed in claim 1, wherein a carbamate of the formula IV 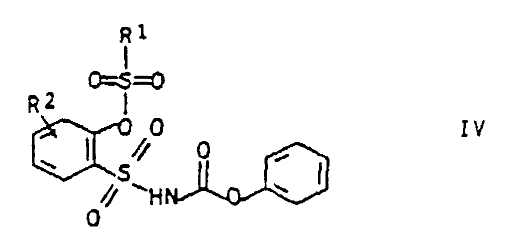 where R¹ and R² have the meanings stated in claim 1, is reacted with about the stoichiometric amount of an amine of the formula III 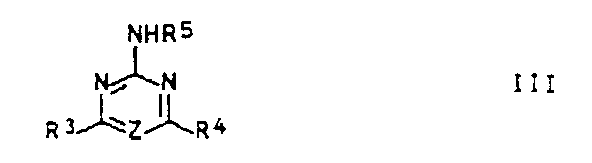 where R³ to R⁵ have the meanings stated in claim 1, in a conventional manner, in an inert organic solvent.

10. A process for the preparation of a sulfonylurea derivative of the formula I as claimed in claim 1, wherein a correspondingly substituted benzenesulfonamide of the formula V 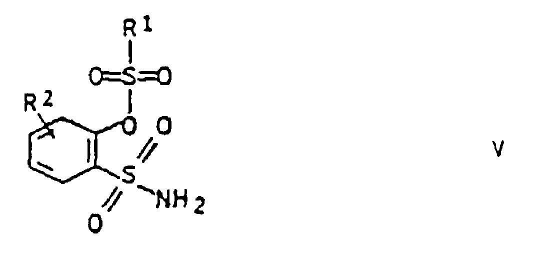 where R¹ and R² have the meanings stated in claim 1, is reacted with about the stoichiometric amount of a carbamate of the formula VI 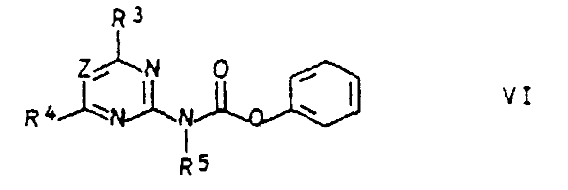 where R³ to R⁵ have the meanings stated in claim 1, in a conventional manner, in an inert organic solvent.

11. A process for the preparation of a sulfonylurea derivative of the formula I as claimed in claim 1, where R⁵ is hydrogen, wherein a correspondingly substituted benzenesulfonamide of the formula V 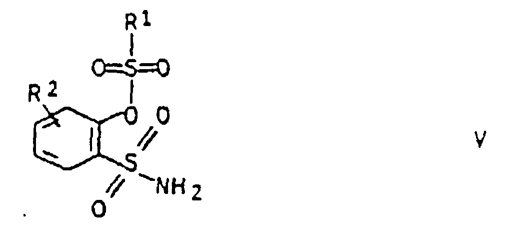 where R¹ and R² have the meanings stated in claim 1, is reacted with about the stoichiometric amount of an isocyanate of the formula VII 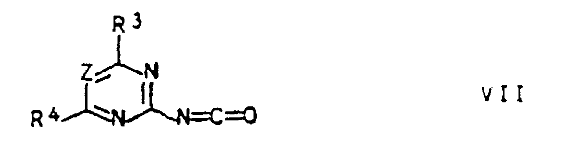 where R³ and R⁴ have the meanings stated in claim 1, in a conventional manner, in an inert organic solvent.

12. A herbicide containing a sulfonylurea derivative of the formula I as claimed in claim 1 or its salt and a carrier conventionally used for this purpose.

13. A method for controlling undesirable plant growth, wherein a herbicidal amount of a sulfonylurea derivative of the formula I as claimed in claim 1 or of one of its salts is allowed to act on the plants and/or their habitat.

## Revendications

1. Dérivés de sulfonylurée substitués de formule générale I, 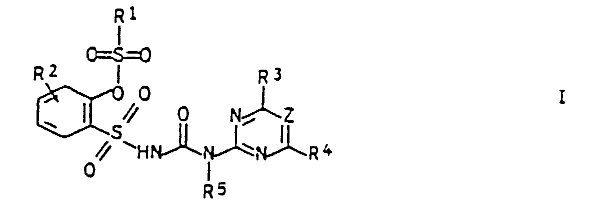 dans laquelle les symboles représentent
R¹ représente un groupe alkyle en C₁-C₄, qui peut porter jusqu'à trois des restes suivants: halogéno ou un groupe alcoxy en C₁-C₂; un groupe alcényle en C₂-C₃; le groupe propargyle; un groupe alkyl(C₁-C₃)amino ou un groupe di(alkyl en C₁-C₄)amino; un reste phényle, qui peut porter jusqu'à trois des restes suivants: halogéno, un groupe alkyle en C₁-C₄ ou un groupe alcoxy en C₁-C₂,
R² hydrogène, halogéno, le groupe méthyle, méthoxy ou éthoxy, qui peuvent chacun porter 1 à 3 atomes d'halogène, un groupe alkyl(C₁-C₂)-sulfonyle, le groupe nitro ou le groupe cyano;
R³ le groupe trifluorométhoxy, le groupe bromodifluorométhoxy, le groupe chlorodifluorométhoxy ou le fluor;
R⁴ halogéno, le groupe méthyle ou éthyle, un groupe halogénoalkyle en C₁-C₂, un groupe halogénoalcoxy en C₁-C₂, le groupe méthoxy ou éthoxy ou le groupe méthyl- ou diméthylamino;
R⁵ hydrogène, un groupe alkyle en C₁-C₃, un groupe alcényle en C₂-C₃ ou un groupe alcynyle en C₃-C₄;
Z CH ou N,
avec pour condition que, quand R³ désigne un atome de fluor et que Z représente N, R⁴ ne désigne pas un groupe alkylamino,
ainsi que leurs sels utilisables en agriculture.

2. Dérivés de sulfonylurée substitués de formule I selon la revendication 1, dans laquelle R¹ à R⁴ et Z possèdent la signification indiquée dans la revendication 1 et R⁵ représente l'hydrogène ou le groupe méthyle.

3. Dérivés de sulfonylurée substitués de formule I selon la revendication 1, dans laquelle R¹ représente un groupe alkyle en C₁-C₄, qui peut être substitué une à trois fois par un halogène ou un groupe alcoxy en C₁-C₂; le groupe propargyle ou un groupe alcényle en C₂-C₃, R⁵ désigne l'hydrogène ou le groupe méthyle, et R² à R⁴ et Z ont la signification indiquée dans la revendication 1.

4. Dérivés de sulfonylurée substitués de formule I selon la revendication 1, dans laquelle R¹ représente un groupe alkyl(C₁-C₃)amino ou un groupe di(alkyl en C₁-C₄)amino, R⁵ désigne l'hydrogène ou le groupe méthyle et R² à R⁴ et Z ont la signification indiquée dans la revendication 1.

5. Dérivés de sulfonylurée substitués de formule I selon la revendication 1, dans laquelle R¹ représente un reste phényle, qui peut porter un à trois des restes suivants: halogéno, un groupe alkyle en C₁-C₄, méthoxy ou éthoxy, R⁵ désigne l'hydrogène ou le groupe méthyle, et R² à R⁴ et Z ont la signification indiquée dans la revendication 1.

6. Dérivés de sulfonylurée substitués de formule I selon la revendication 1, dans laquelle R¹ représente un groupe alkyle en C₁-C₄, qui peut porter un à trois atomes d'halogène; le groupe propargyle; un groupe alcényle en C₂-C₃; le groupe méthylamino ou diméthylamino, R³ désigne le groupe trifluorométhoxy, le groupe chlorodifluorométhoxy ou le fluor, R⁵ représente l'hydrogène ou le groupe méthyle, et R², R⁴ et Z ont la signification indiquée dans la revendication 1.

7. Dérivés de sulfonylurée substitués de formule I selon la revendication 1, dans laquelle R¹ représente un groupe alkyle en C₁-C₄, qui peut porter un à trois atomes d'halogène; le groupe propargyle; un groupe alcényle en C₁-C₃; le groupe méthylamino ou diméthylamino, R³ désigne le groupe trifluorométhoxy, le groupe chlorodifluorométhoxy ou le fluor, R⁴ représente le groupe méthoxy, R⁵ désigne l'hydrogène ou le groupe méthyle, et R² et Z ont la signification indiquée dans la revendication 1.

8. Procédé pour la préparation de dérivés de sulfonylurée de formule I selon la revendication 1, caractérisé par le fait qu'on fait réagir un benzènesulfonylisocyanate substitué de façon appropriée, de formule générale II, 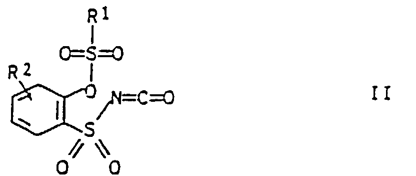 dans laquelle R¹ et R² ont la signification indiquée dans la revendication 1, avec des quantités approximativement stoechiométriques d'une amine de formule générale III, 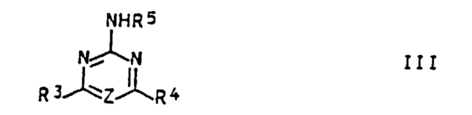 dans laquelle R³ à R⁵ ont la signification indiquée dans la revendication 1, de manière connue en soi dans un solvant organique inerte.

9. Procédé pour la préparation de dérivés de sulfonylurée de formule I selon la revendication 1, caractérisé par le fait qu'on fait réagir un carbamate de formule IV 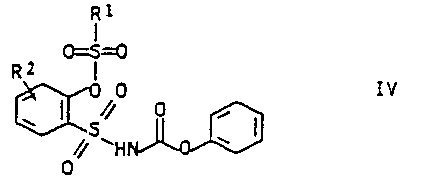 dans laquelle R¹ et R² ont la signification indiquée dans la revendication 1, avec des quantités approximativement stoechiométriques d'une amine de formule III, 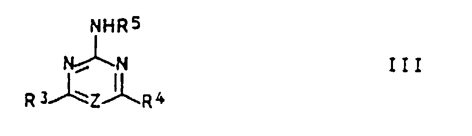 dans laquelle R³ à R⁵ ont la signification indiquée dans la revendication 1, de manière connue en soi dans un solvant organique inerte.

10. Procédé pour la préparation de dérivés de sulfonylurée de formule I selon la revendication 1, caractérisé par le fait qu'on fait réagir un benzènesulfonamide substitué de façon appropriée, de formule générale V, 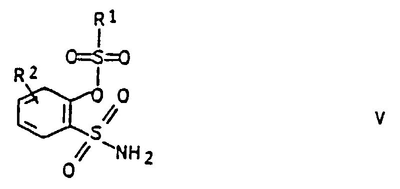 dans laquelle R¹ et R² ont la signification indiquée dans la revendication 1, avec des quantités approximativement stoechiométriques d'un carbamate de formule générale VI, 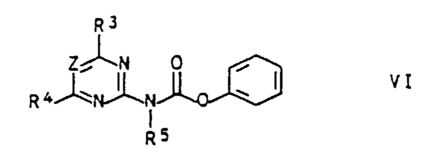 où R³ à R⁵ ont la signification indiquée dans la revendication 1, de manière connue en soi dans un solvant organique inerte.

11. Procédé pour la préparation de dérivés de sulfonylurée de formule I selon la revendication 1, dans laquelle le reste R⁵ représente un atome d'hydrogène, caractérisé par le fait qu'on fait réagir un benzènesulfonamide substitué de façon appropriée, de formule générale V, 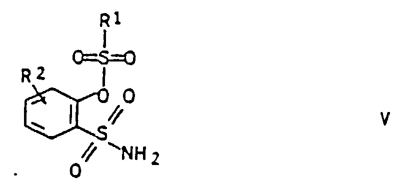 dans laquelle R¹ et R² ont la signification indiquée dans la revendication 1, avec des quantités approximativement stoechiométriques d'un isocyanate de formule générale VII, 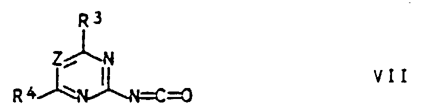 où R³ et R⁴ ont la signification indiquée dans la revendication 1, de manière connue en soi dans un solvant organique inerte.

12. Agent herbicide, contenant un dérivé de sulfonylurée de formule I selon la revendication 1 ou son sel, ainsi que des supports classiques pour cela.

13. Procédé pour lutter contre la croissance non souhaitée de plantes, caractérisé par le fait qu'on fait agir une quantité efficace du point de vue herbicide d'un dérivé de sulfonylurée de formule I selon la revendication 1 ou d'un de ses sels sur les plantes et/ou leur biotope.
